# EUROPEAN PATENT SPECIFICATION

(11) **EP 4 205 741 B1**
(45) Date of publication and mention of the grant of the patent: **10.12.2025**
(21) Application number: 21863757.7
(22) Date of filing: 29.10.2021
(51) Int. Cl.: A61K 31/426, A61K 31/427, A61K 31/4439, A61K 31/497, A61K 31/5377, A61P 1/16, A61P 35/00, A61P 19/02, A61P 31/04, A61P 25/28, A61P 25/16, A61P 9/10, A61P 3/10, A61P 3/06

(54) **CYP2E1 INHIBITORS FOR THE TREATMENT OF INFLAMMATORY-MEDIATED DISEASES**
CYP2E1-HEMMER ZUR BEHANDLUNG VON INFLAMMATORISCH VERMITTELTEN ERKRANKUNGEN
INHIBITEURS DU CYP2E1 POUR LE TRAITEMENT DES MALADIES À MÉDIATION INFLAMMATOIRE

(30) Priority: 01.09.2020 CN 202010906508
(43) Date of publication of application: 05.07.2023
(73) Proprietor: Suzhou Lingxi Biotechnology Co., Ltd., Jiangsu 215123 (CN)
(72) Inventor: QIAO, Hailing, Zhengzhou, Henan 450052 (CN); XU, Haiwei, Zhengzhou, Henan 450052 (CN); FANG, Yan, Zhengzhou, Henan 450052 (CN); GAO, Na, Zhengzhou, Henan 450052 (CN); WEN, Qiang, Zhengzhou, Henan 450052 (CN); LI, Shufeng, Zhengzhou, Henan 450052 (CN)
(74) Representative: Gille Hrabal Partnerschaftsgesellschaft mbB Patentanwälte
(86) International application number: PCT/CN2021/127710
(87) International publication number: WO 2022/048695

(56) References cited:
- CN-A- 1 276 789
- CN-A- 101 921 269
- CN-A- 105 664 181
- CN-A- 105 769 860
- SINGH ATAMJIT, MALHOTRA DANISH, SINGH KARANVIR, CHADHA RENU, BEDI PREET MOHINDER SINGH: "Thiazole derivatives in medicinal chemistry: Recent advancements in synthetic strategies, structure activity relationship and pharmacological outcomes", JOURNAL OF MOLECULAR STRUCTURE, vol. 1266, 15 October 2022 (2022-10-15), pages 133479, XP087112395, ISSN: 0022-2860, DOI: 10.1016/j.molstruc.2022.133479
- GAO NA, ZOU DAN, QIAO HAI-LING: "Concentration-Dependent Inhibitory Effect of Baicalin on the Plasma Protein Binding and Metabolism of Chlorzoxazone, a CYP2E1 Probe Substrate, in Rats In Vitro and In Vivo", PLOS ONE, vol. 8, no. 1, 2 January 2013 (2013-01-02), pages e53038, XP055907612, DOI: 10.1371/journal.pone.0053038

## Description

### TECHNICAL FIELD

The present disclosure relates to the fields of targets and drugs for the prevention and treatment of inflammation-mediated diseases (IMDs) and specifically relates to a use of a compound as a CYP2E1 inhibitor.

### BACKGROUND

It is currently recognized that the occurrence of many diseases is related to inflammation, such as tumors (liver cancer, cervical cancer, nasopharyngeal carcinoma (NPC), colorectal cancer (CRC), glioma, and lung cancer) and non-tumor diseases (Alzheimer's disease (AD), Parkinson's syndrome, stroke, arteriosclerosis, diabetes, liver fibrosis, and pulmonary fibrosis); and the above diseases related to inflammation can be collectively referred to as IMD. It is generally believed that a long-term non-controllable inflammatory microenvironment is associated with the occurrence of IMD. In 2005, on the 125th anniversary of *Science's* publication, it was written that "inflammation is a major cause for all chronic diseases."

A tumor microenvironment (TME) plays a critical role in the occurrence and development of a tumor, and in the past 10 years, the study of TME has developed rapidly. In 2010, Professor Karin M of the University of California wrote on Cell that the immunity and inflammation in a TME are closely related to the occurrence and development of a tumor. In recent years, breakthroughs have been made in the immunological research of TME, and the immunotherapeutic drugs PD-1 and PD-L1 targeting TME have been successfully used in clinical practice and have become broad-spectrum anti-tumor drugs. The American immunologist James P. Allison and the Japanese immunologist Tasuku Honjo, who had made outstanding original contributions to this field, were awarded the 2018 Nobel Prize in Physiology or Medicine.

Chronic non-controllable inflammation is an important feature of TME. For example, liver cancer usually originates from hepatitis and cirrhosis, hepatic stellate cells (HSCs) are an important constituent part of a general TME in addition to hepatoma extracellular stromal cells, and the activation of HSCs can lead to collagen deposition. Persistent liver damage caused by alcohol abuse, nonalcoholic steatohepatitis (NASH), chronic hepatitis B virus (CHBV) infection, or the like can lead to hypoxia and chronic uncontrolled inflammation, which are important features of a TME of liver cancer.

The occurrence and development of a tumor are closely related to a TME, and TME-targeted drugs have become effective means for treating liver cancer, such as immune checkpoint inhibitors (ICIs) PD-1 and PDL-1 and an angiogenesis inhibitor bevacizumab. The TME-targeted drugs generally have a broad-spectrum anti-tumor effect. For example, the angiogenesis inhibitor bevacizumab can be used to treat CRC, non-small cell lung cancer (NSCLC), renal cell carcinoma (RCC), ovarian cancer, cervical cancer, liver cancer, or the like; and the ICIs PD-1 and PDL-1 can be used to treat melanoma, lung cancer, breast cancer, liver cancer, pancreatic cancer, digestive tract tumor, gynecological tumor, urologic tumor, myeloma, lymphoma, or the like.

The successful development of drugs targeting immunity and angiogenesis in a TME provides a successful reference for tumor research. Admittedly, the traditional anti-inflammatory drugs such as COX-2 inhibitors and other non-steroidal anti-inflammatory drugs (NSAIDs) have played an important role as an antipyretic, analgesic, anti-inflammatory, and anti-rheumatic but have not exhibited satisfactory efficacy for inflammation-associated tumors, and there is currently no anti-tumor drug targeting an inflammatory TME. This may be because there are new targets of inflammation in a chronic uncontrolled inflammatory microenvironment that have not yet been discovered.

Cytochrome P450 2E1 (CYP2E1) is a protein mainly present in the endoplasmic reticulum (ER) of hepatocytes, and the applicants have found that the highest proportion of CYP2E1 in hepatic cytochrome P450 (CYP450) is about 24.8%. CYP2E1 mainly has a function of metabolism and participates in the biotransformation of drugs, procarcinogens, and environmental toxins. For example, CYP2E1 can metabolically activate more than 85 exogenous substances to produce hepatotoxic or carcinogenic substances, including nitrosamines, benzene, 1,3-butadiene, toluene, chloroform, acetone, tobacco-specific carcinogen NNK, or the like.

The metabolic activation of CYP2E1 is closely related to IMDs such as tumors. For example, the occurrence of liver cancer is related to various factors such as hepatitis virus, nitrosamine, aflatoxin, and alcohol. Nitrosamine (N-nitrosamine) is a strong carcinogen, which is metabolically activated by CYP2E1 *in vivo* to produce a carcinogen and then forms an adduct with DNA to cause liver cancer. The nitrosamine content in most traditional Chinese foods, such as cooked meat products, preserved meat, ham, and pickled vegetables exceeds a given standard; and epidemiological studies have proved that nitrosamine content in food is closely related to the occurrence of liver cancer. Animal experiments have shown that the knockout of a CYP2E1 gene in mice can significantly inhibit diethylnitrosamine (DEN)-induced liver cancer in mice. It suggests that CYP2E1 may affect the occurrence of liver cancer by affecting the metabolic activation of nitrosamine *in vivo.*

An inflammatory effect of CYP2E1 is closely related to IMDs such as tumors. CYP2E1 has a significant inflammatory effect and is involved in the occurrence and development of many IMDs. CYP2E1 is related to inflammation-associated tumors, such as liver cancer, glioma, ovarian cancer, lung cancer, NPC, bladder cancer, and gallbladder cancer, and the occurrence and development of hepatic diseases, such as liver damage, NASH, liver fibrosis, and other IMDs such as rheumatoid arthritis, sepsis, AD, hyperlipidemia, diabetes, ischemic stroke, and pulmonary fibrosis. CYP2E1 can enhance the release of TNF-α in Kupffer cells, resulting in inflammatory necrosis of hepatocytes. In NASH model mice, the activity of CYP2E1 can be inhibited by reducing the expression of TNF-α, restoring the activity of endothelial nitric oxide synthase (eNOS), or the like. The knockout of the CYP2E1 gene in mice can significantly inhibit an inflammatory response induced by chronic alcohol exposure, and the CYP2E1 inhibitor diallyl sulfide can reduce the release of IL-1β and IL-12 by inhibiting CYP2E1 to prevent and treat NASH.

The inflammatory effect of CYP2E1 is related to the promotion of oxidative stress and lipid peroxidation (LPO). CYP2E1 can promote the generation of reactive oxygen species (ROS), induce oxidative stress and LPO, and cause hepatocellular inflammation, apoptosis, and liver fibrosis. The high expression of CYP2E1 in hepatocytes can promote the generation of ROS. ROS can activate a cell-surface molecule Fas ligand (FasL) of the tumor necrosis factor (TNF) family to produce a protease-linked reaction, thereby causing cell lysis and apoptosis. Apoptotic hepatocytes can promote the aggregation of inflammatory cells and induce the production of inflammatory factors, such as TNF-α and IL-6, thereby causing liver inflammation and steatohepatitis. CYP2E1 can also affect the metabolism of arachidonic acid (AA) and promote the invasion and metastasis of liver cancer cells. Studies have shown that CYP2E1 increases AA toxicity mainly through ROS and LPO products. CYP2E1 can cause the release of Ca²⁺ in cells through ROS peroxidation to activate phospholipase A2 (PLA2), thereby promoting the generation of AA. In liver cancer cells, AA is converted into prostaglandin 2 (PGE2) under the action of cyclooxygenase 2 (COX-2), and PGE2 binds to an EP receptor coupled to G protein on a cell membrane to activate the EGFR/Met signaling pathway, thereby causing the invasion and metastasis of liver cancer cells.

In recent years, the applicants have established a large liver specimen bank including liver specimens of more than 127 healthy individuals and liver specimens of 102 liver cancer patients with liver cirrhosis to systematically investigate the physiology and pathology of CYP450. It has been found that an individual difference of CYP2E1 is about 10 times or more; the metabolic activity of CYP2E1 in a liver cancer patient is significantly increased by about 2.13 times, and a positive rate is about 44.6%; the activity of CYP2E1 is significantly negatively correlated with a postoperative survival period of a patient, and CYP2E1 positive and negative individuals have survival periods of 238 d and 612 d, respectively; and the increase in CYP2E1 activity is an independent risk factor for the occurrence and development of liver cancer. It has been proved by a rat primary hepatocellular carcinoma (PHC) model that the innate activity (before modeling) of CYP2E1 has a prominent causal relationship with the occurrence of liver cancer, that is, the higher the innate activity of CYP2E1, the more prone to liver cancer. It is suggested that CYP2E1 may be a new target for the prevention and treatment of liver cancer, and thus it is speculated that a drug targeting the new target CYP2E1 in an inflammatory TME may have a broad-spectrum prevention and treatment effect for IMDs.

In conclusion, CYP2E1 participates in the occurrence and development of many IMDs through metabolic activation and inflammation, and thus the inhibition of CYP2E1 activity is of great significance for the prevention and treatment of the IMDs. Therefore, the investigation of inhibitors for CYP2E1 has very important theoretical and practical significance.

There are currently no clinical CYP2E1-specific inhibitors. The compounds or drugs with a CYP2E1-inhibiting effect reported in the current study include 4-methylpyrazole, disulfiram, diethyldithiocarbamate (DDTC), isothiocyanic acid, orphenadrine, and chlormethiazole, and most of the compounds or drugs have poor selection specificity for the CYP2E1-inhibiting effect and great toxicity. Therefore, these compounds or drugs are mostly used in basic research. There are currently no clinical CYP2E1-specific inhibitors. Therefore, based on the role of CYP2E1 in the occurrence and development of many diseases such as hepatic diseases, there is an urgent need to conduct the screening and synthesis of CYP2E1 inhibitors

CN105769860A discloses clomethiazole, a CYP2E1 inhibitor, for use in the treatment of hepatic diseases, but fails to disclose the specific compounds of the present disclosure.

### SUMMARY

The present disclosure is defined by the appended claims.

### BRIEF DESCRIPTION OF THE DRAWINGS

FIG. 1 is a hydrogen nuclear magnetic resonance (HNMR) spectrum of the compound SMIO;
FIG. 2 is an HNMR spectrum of the compound SMI7;
FIG. 3 is an HNMR spectrum of the compound SMI16;
FIG. 4 is an HNMR spectrum of the compound SMI20;
FIG. 5 is an XRPD pattern of a crystal form A of a hydrochloride of SMIO;
FIG. 6 is a DSC-TGA pattern of a crystal form A of a hydrochloride of SMIO;
FIG. 7 is an XRPD pattern of a crystal form B of a sulfate of SMIO;
FIG. 8 is a DSC-TGA pattern of a crystal form B of a sulfate of SMIO;
FIG. 9 is a double-reciprocal plot of an inhibitory effect of the compound SMIO on human hepatic metabolism of chlorzoxazone (CZX);
FIG. 10 is a secondary plot based on the double-reciprocal plot of an inhibitory effect of the compound SMIO on metabolism of CZX by human hepatic CYP2E1;
FIG. 11 shows inhibitory effects of 21 small molecule compounds on the *in vitro* metabolic activity of CYP2E1;
FIG. 12 shows inhibitory effects of the compound SMIO on the metabolism of DEN by CYP2E1 in rats;
FIG. 13 shows inhibitory effects of the compound SMIO on DEN-induced rat liver damage models;
FIG. 14 shows inhibitory effects of the compound SMIO on mouse hepatic steatosis and hepatitis models induced by a high-fat diet, where A shows representative pictures of mouse livers in each hepatitis model group; B shows inflammation-associated histopathological manifestations; H&E refers to hematoxylin and eosin staining; Masson refers to Masson staining; Oil red refers to oil red O staining; and LCA refers to leukocyte common antigen staining;
FIG. 15 shows quantitative plotting results of relevant parameters of the inhibition of the compound SMIO on mouse hepatic steatosis and hepatitis models induced by a high-fat diet, where A shows a liver coefficient of animals in each hepatitis model group; B shows a steatosis score of animals in each group; C shows a Masson staining score of animals in each group; D shows a percentage of an oil red O staining area of animals in each group; and E shows an LCA staining score of animals in each group;
FIG. 16 shows inhibitory effects of the compound SMIO on mouse liver fibrosis models induced by a high-fat diet, where A shows representative pictures of mouse livers in each liver fibrosis model group; B shows inflammation and liver fibrosis-associated histopathological manifestations; H&E refers to hematoxylin and eosin staining; Masson refers to Masson staining; and Oil red refers to oil red O staining;
FIG. 17 shows quantitative plotting results of relevant parameters of the inhibition of the compound SMIO on mouse liver fibrosis models induced by a high-fat diet, where A shows a liver coefficient of animals in each liver fibrosis model group; B shows a steatosis score of animals in each group; C shows a Masson staining score of animals in each group; and D shows a percentage of an oil red O staining area of animals in each group;
FIG. 18 shows inhibitory effects of the compound SMIO on DEN-induced rat liver fibrosis models, where A shows representative pictures of rat livers in each liver fibrosis model group; B shows liver fibrosis-associated histopathological manifestations; H&E refers to hematoxylin and eosin staining; Masson refers to Masson staining; α-SMA refers to α-smooth muscle actin; and collagen I refers to type I collagen;
FIG. 19 shows quantitative plotting results of relevant parameters of the inhibition of the compound SMIO on DEN-induced rat liver fibrosis models, where A shows a liver coefficient of animals in each liver fibrosis model group; B shows a Ishark score of animals in each group; C shows a Masson staining score of animals in each group; D shows a α-SMA histochemical score of animals in each group; and E shows a collagen I histochemical score of animals in each group;
FIG. 20 shows inhibitory effects of CYP2E1 gene knockout on lipopolysaccharide (LPS)-induced mouse pulmonary fibrosis models, where A shows HE and Masson staining results of a mouse lung tissue in each group; and B shows a collagen-positive area percentage and a histological score of a mouse lung tissue in each group;
FIG. 21 shows inhibitory effects of the compound SMIO on LPS-induced mouse lung injury, where A shows HE staining results of a mouse lung tissue in each group; and B shows a lung injury score of mice in each group;
FIG. 22 shows inhibitory effects of the compound SMIO on LPS-induced mouse pulmonary fibrosis models, where A shows HE and Masson staining results of a mouse lung tissue in each group; and B shows a collagen-positive area percentage and a histological score of a mouse lung tissue in each group;
FIG. 23 shows a change of CYP2E1 activity and a correlation of CYP2E1 activity with a pulmonary fibrosis severity in LPS-induced mouse pulmonary fibrosis models, where A shows a change of CYP2E1 activity of mice in each group; and B shows a correlation between CYP2E1 activity and a lung index in mice;
FIG. 24 shows inhibitory effects of the compound SMIO on myeloperoxidase (MPO) levels in lung tissues in LPS-induced mouse pulmonary fibrosis models, where A shows MPO staining results of a mouse lung tissue in each group; and B shows an MPO-positive staining area percentage in a mouse lung tissue in each group;
FIG. 25 shows inhibitory effects of the compound SMIO on inflammatory factors in lung tissues of LPS-induced mouse pulmonary fibrosis models, where A shows a relative expression level of TNF-α in a mouse lung tissue in each group; and B shows a relative expression level of IL-1β in a mouse lung tissue in each group;

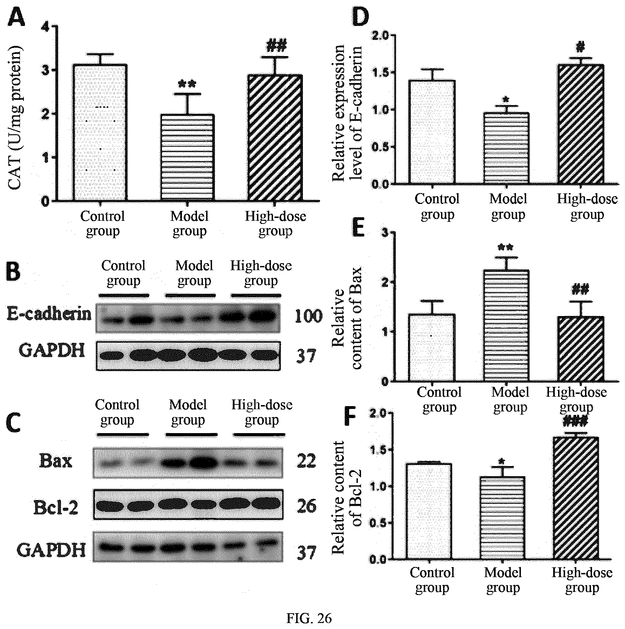

FIG. 26 shows inhibitory effects of the compound SMIO on the oxidative stress and the expression of epithelial cell marker E-cadherin and apoptosis-associated proteins in lung tissues of LPS-induced mouse pulmonary fibrosis models, where A shows an oxidative stress index CAT level in a mouse lung tissue in each group; B and C show Western Blot (WB) results of the expression of epithelial cell marker E-cadherin and apoptosis-associated proteins in a mouse lung tissue in each group; and D, E, and F show quantification results of the expression of epithelial cell marker E-cadherin and apoptosis-associated proteins in a mouse lung tissue in each group;
FIG. 27 shows inhibitory effects of the compound SMIO on the expression of TGF-β1 and α-SMA in lung tissues of LPS-induced mouse pulmonary fibrosis models, where A and C show TGF-β1 and α-SMA staining results of a mouse lung tissue in each group; and B and D show TGF-B1 and α-SMA-positive staining area percentages in each group;
FIG. 28 shows a high expression level of CYP2E1 in a paracancerous tissue of lung cancer;
FIG. 29 shows inhibitory effects of CYP2E1 gene knockout on mouse lung cancer models constructed through *in situ* implantation of lung cancer Lewis cells into lungs of mice, where A is a general picture illustrating a lung tumor in each mouse; and B shows a weight of a lung tumor in each mouse;
FIG. 30 shows inhibitory effects of the compound SMIO on mouse lung cancer models constructed through *in situ* implantation of lung cancer Lewis cells into lungs of mice, where A shows a representative picture of a tumor-bearing lung tissue of mice in each group; B is a general picture illustrating a lung tumor in each mouse; and C shows a weight of a lung tumor in each mouse;
FIG. 31 shows inhibitory effects of the compound SMIO on lung metastasis models constructed through tail vein injection of CRC cells CT26, where A is a general picture illustrating a tumor-bearing lung tissue of each mouse; and B shows a weight of a lung of each mouse;
FIG. 32 shows inhibitory effects of the compound SMIO on lung metastasis models constructed through tail vein injection of melanoma cells B16-F10, where A is a general picture illustrating a tumor-bearing lung tissue of each mouse; and B shows a weight of a lung of each mouse;
FIG. 33 shows a change of CYP2E1 activity and a correlation between CYP2E1 activity and tumor severity in mouse lung cancer models constructed through *in situ* implantation of lung cancer Lewis cells into lungs of mice, where the left panel shows a change of CYP2E1 activity in mice of each group; and the right panel shows a correlation between CYP2E1 activity and lung tumor weight in mice;
FIG. 34 shows the inhibition of the compound SMIO on an inflammatory microenvironment in a paracancerous lung tissue of a tumor model constructed through *in situ* transplantation of Lewis cells into lungs, where A shows WB results of the expression of inflammatory factors and related signaling pathway proteins in a paracancerous lung tissue of mice in each group; and B shows quantification results of the expression of inflammatory factors and related signaling pathway proteins in a paracancerous lung tissue of mice in each group;
FIG. 35 shows effects of the compound SMIO on apoptosis and autophagy-associated proteins in a cancer tissue of a tumor model constructed through *in situ* transplantation of Lewis cells into lungs, where A shows WB results of apoptosis and expression of autophagy-associated proteins in lung cancer tissues of mice in each group; and B shows quantification results of apoptosis and expression of autophagy-associated proteins in lung cancer tissues of mice in each group;
FIG. 36 shows that the compound SMIO has no direct inhibitory effect on lung cancer cells, where A is for Lewis lung cancer cells; and B is for A549 lung cancer cells;
FIG. 37 shows that the compound SMIO inhibits the proliferation of A549 lung cancer cells by inhibiting the M2 polarization of macrophages;
FIG. 38 shows the increased CYP2E1 contents in paracancerous liver tissues of liver cancer patients;
FIG. 39 shows inhibitory effects of CYP2E1 gene knockout on rat liver cancer models constructed through *in situ* implantation of Walker256 cells into livers, where A is a representative picture illustrating tumor-bearing livers of rats in each group; B shows liver tumors of rats in each group; and C shows a total tumor weight and a maximum tumor diameter in the liver of each rat;
FIG. 40 shows inhibitory effects of the compound SMIO on mouse liver cancer models constructed through *in situ* implantation of liver cancer cells H22 into livers, where A is a representative picture illustrating tumor-bearing livers of mice in each group; B shows liver tumors of mice in each group; and C shows a total tumor weight and a maximum tumor diameter in the liver of each mouse;
FIG. 41 shows the inhibition of the compound SMIO on an inflammatory microenvironment in a paracancerous liver tissue of a mouse tumor model constructed through *in situ* transplantation of H22 cells into a liver, where A shows WB results of the expression of inflammatory factors and related signaling pathway proteins in a paracancerous liver tissue of mice in each group; and B shows quantification results of the expression of inflammatory factors and related signaling pathway proteins in a paracancerous liver tissue of mice in each group;
FIG. 42 shows effects of the compound SMIO on apoptosis and related signaling pathway proteins in a paracancerous liver tissue and a cancer tissue of a mouse tumor model constructed through *in situ* transplantation of H22 cells into a liver, where A shows WB results of apoptosis and expression of related signaling pathway proteins in liver cancer tissues of mice in each group; and B shows quantification results of apoptosis and expression of related signaling pathway proteins in liver cancer tissues of mice in each group;
FIG. 43 shows that the compound SMIO has no direct inhibitory effect on liver cancer cells, where A is for HepG2 liver cancer cells; and B is for H22 liver cancer cells;
FIG. 44 shows that the compound SMIO plays an anti-liver cancer role by influencing macrophages, where A, B, and C show the influence of SMIO on apoptosis, migration, and proliferation of HepG2 liver cancer cells co-cultivated with macrophages; and D and E show quantification results of the influence of SMIO on apoptosis and migration of HepG2 liver cancer cells co-cultivated with macrophages;
FIG. 45 shows a high expression level of CYP2E1 in a paratumoral tissue of a glioma patient, where A shows immunohistochemical results; and B shows qPCR results;
FIG. 46 shows an inhibitory effect of CYP2E1 gene knockout on a mouse glioma model constructed through *in situ* implantation of glioma GL261 cells into the brain;
FIG. 47 shows an inhibitory effect of the compound SMIO on a mouse glioma model constructed through *in situ* implantation of glioma GL261 cells into the brain, where A is a general picture illustrating brains of mice in each group; B shows representative pictures of HE staining of mouse brain tissues in each group; C shows a tumor volume of mice in each group; and D shows an *in vitro* inhibitory effect of SMIO on GL261 cells;
FIG. 48 shows a change of CYP2E1 activity and a correlation between CYP2E1 activity and tumor severity in a mouse model constructed through *in situ* transplantation of glioma, where the left panel shows a change of CYP2E1 activity in mice of each group; and the right panel shows a correlation between CYP2E1 activity and lung tumor weight in mice;
FIG. 49 shows that the compound SMIO has no direct effect on GL261 (left) or U251 (right) glioma cells;
FIG. 50 shows that the compound SMIO inhibits GL261 or U251 glioma by inhibiting primary microglial cells or source microglial cells HMC-3, where A and C show the inhibition on proliferation of tumor cells; and B and D show the promotion on apoptosis of tumor cells;
FIG. 51 shows that the compound SMIO inhibits GL261 glioma by inhibiting primary astrocytes, where A shows the inhibition on proliferation of tumor cells; and B shows the promotion on apoptosis of tumor cells;
FIG. 52 shows the inhibition of the compound SMIO on M2 polarization of primary microglial cells;
FIG. 53 shows the inhibition of the compound SMIO on cholesterol metabolism of astrocytes;
FIG. 54 shows an inhibitory effect of the compound SMIO on a mouse ovarian cancer model constructed through *in situ* implantation of ovary cancer ID-8 cells into the ovary, where A shows a total tumor weight of mice in each group; B shows an ascites weight of mice in each group; and C shows an *in vitro* inhibitory effect of SMIO on ID-8 cells;
FIG. 55 shows an inhibitory effect of the compound SMIO on a mouse model constructed through abdominal transplantation of ovarian cancer ID-8 cells, where the left panel shows a total tumor weight of mice in each group; and the right panel shows an ascites weight of mice in each group;
FIG. 56 shows an inhibitory effect of CYP2E1 gene knockout on a collagen-induced rat rheumatoid arthritis model, where A shows general representative pictures of feet in each group; and B shows the hindfoot score, hindfoot thickness, and hindfoot volume of rats in each group;
FIG. 57 shows an inhibitory effect of the compound SMIO on a collagen-induced rat rheumatoid arthritis model, where A shows the foot swelling coefficient of rats in each group; and B shows the total score, foot thickness, and foot volume of rats in each group;
FIG. 58 shows a dose-response relationship of the compound SMIO for resisting rheumatoid arthritis, where A shows a foot swelling recovery rate of rats in SMIO groups of different doses; and B shows a correlation between a SMIO dose and a rat foot swelling recovery rate on day 2, day 8, and day 20;
FIG. 59 shows general pictures of feet of complete Freund's adjuvant (CFA)-induced rat rheumatoid arthritis models under the inhibition of the compound SMIO;
FIG. 60 shows quantification results of relevant parameters of the inhibition of the compound SMIO on CFA-induced rat rheumatoid arthritis models, where A shows foot swelling rates of rats in each group at different time points; B shows a rat foot swelling rate at 24 h; C shows a rat foot swelling rate at 36 h; and D shows a rat foot swelling rate at 48 h;
FIG. 61 shows a dose-response relationship of the inhibition of the compound SMIO on CFA-induced rat rheumatoid arthritis models, where A shows dose-response relationships for a rat foot swelling rate at different time points; B shows a dose-response relationship at 24 h; C shows a dose-response relationship at 36 h; and D shows a dose-response relationship at 48 h;
FIG. 62 shows a change of CYP2E1 activity and a correlation of CYP2E1 activity with a foot swelling severity in rheumatoid arthritis rat models, where A shows a change of CYP2E1 activity in rheumatoid arthritis rat models; and B shows a correlation between CYP2E1 activity and foot swelling severity in rats;
FIG. 63 shows the inhibition of the compound SMIO on a body temperature change in LPS-induced rat sepsis models, where A shows body temperatures of rats in each group at different time points; B shows a body temperature of rats in each group at 4 h; C shows a body temperature of rats in each group at 5 h; and D shows a body temperature of rats in each group at 6 h;
FIG. 64 shows the inhibition of the compound SMIO on a body temperature change in LPS-induced mouse sepsis models, where A shows body temperatures of mice in each group at different time points; B shows a body temperature of mice in each group at 6 h; C shows a body temperature of mice in each group at 12 h; and D shows a body temperature of mice in each group at 24 h;
FIG. 65 shows an improvement effect of the compound SMIO on renal and cardiac function impairment in LPS-induced mouse sepsis models, where A shows a serum urea nitrogen level of mice in each group at 24 h; B shows a serum creatinine level of mice in each group at 24 h; C shows a creatine kinase (CK) level of mice in each group at 24 h; and D shows a serum lactate dehydrogenase (LDH) level of mice in each group at 24 h;
FIG. 66 shows the improvement of the compound SMIO on cognitive dysfunction in streptozotocin (STZ)-induced rat AD models, where A shows incubation periods of rats in each group at different training time points; B shows a time spent in the platform quadrant of rats in each group; and C shows a number of platform crossings of rats in each group;
FIG. 67 shows an inhibitory effect of the compound SMIO on focal cerebral ischemia-reperfusion injury (CIRI) in rats;
FIG. 68 shows the inhibition of the compound SMIO on cerebral infarction and cerebral edema caused by focal CIRI in rats, where A shows a cerebral infarction rate in rats; and B shows a cerebral edema rate in rats;
FIG. 69 shows that the compound SMIO can reduce a blood lipid level in ApoE^{-/-} mouse hyperlipidemia models induced by a high-fat diet;
FIG. 70 shows an inhibitory effect of the compound SMIO on the formation of aortic atherosclerotic plaques in ApoE^{-/-} mice induced by a high-fat diet, where A shows a total plaque area of animals in each group; and B shows a percentage of an oil red O area in a total plaque area of animals in each group;
FIG. 71 shows that the compound SMIO can reduce a blood glucose level in diabetes rats induced by a high-fat diet and STZ, where A shows a fasting blood glucose (FBG) level of rats in each group; B shows blood glucose levels of rats in each group at different time points during a glucose tolerance test; and C shows an area under the curve (AUC) for rats in each group during a glucose tolerance test;
FIG. 72 shows a high expression level of CYP2E1 in a paracancerous tissue of bladder cancer; and
FIG. 73 shows a high expression level of CYP2E1 in a paratumoral tissue of gallbladder cancer.

### DETAILED DESCRIPTION OF THE EMBODIMENTS

The present disclosure will be described in detail below with reference to examples, but the present disclosure is not limited to these examples.

The synthesis of the CYP2E1 inhibitors in Examples 2, 3, 4, and 5 of the present disclosure adopts a high-resolution mass spectrometer of American Waters, with a model of Q-Tof micro.

The synthesis of the CYP2E1 inhibitors in Examples 2, 3, 4, and 5 of the present disclosure adopts a nuclear magnetic resonance (NMR) instrument of German Bruker, with a model of DPX-400.

In Examples 8, 9, and 10 of the present disclosure, the *in vivo* and *in vitro* CYP2E1 inhibitory effects are determined by a high performance liquid chromatograph of Agilent Technologies Inc., with a model of Agilent 1260.

In Example 17 of the present disclosure, a mouse glioma model is prepared by a brain stereotaxic instrument of Shanghai Puxin Instrument Technology Co., Ltd., with a model of ZR-09.

### Example 1 Preparation of a human liver microsome

Differential centrifugation was adopted. A liver specimen was taken out, thawed, weighed, and mixed with a 50 mM Tris-HCl buffer (pH = 7.0) (including 150 mM KCl and 2 mM EDTA) in a ratio of 1:4 (W/V), and a resulting mixture was ground with a glass homogenizer to obtain a liver homogenate; the liver homogenate was centrifuged at 4°C and 9,000 × g for 20 min, and a resulting supernatant was centrifuged at 4°C and 100,000 × g for 60 min; a resulting precipitate was resuspended in 4 mL of 0.15 M Tris-HCl (pH = 7.6), and a resulting suspension was centrifuged at 100,000 g and 4°C for 60 min; a resulting precipitate was added to a 0.25 M sucrose suspension in a ratio of 1:2 (W/V) to finally obtain 2 mL of a microsome suspension per g of the liver tissue; and the microsome suspension was dispensed, stored in liquid nitrogen overnight, and then transferred to -80°C the next day for long-term storage. All of the above operations were conducted in an ice bath. A protein content (mg/mL) in a microsome was determined by the Bradford method.

Liver microsomes in both liver damage patients and healthy individuals were prepared by this method.

### Example 2 Synthesis of SMIO

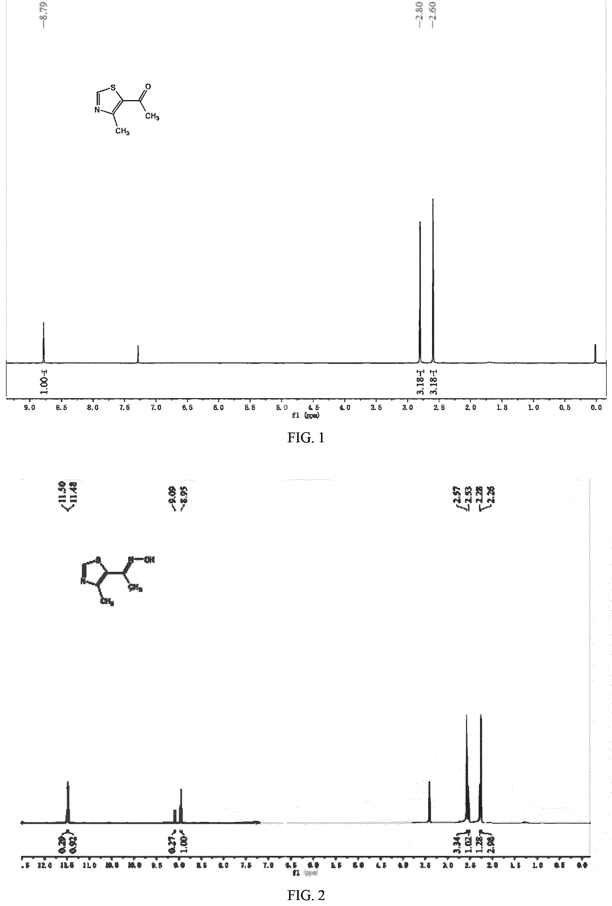

Ethyl 4-methylthiazole-5-carboxylate (1 mol) and NaOH (1.6 mol) were mixed in a mixed solution of ethanol and water, and a reaction was conducted overnight at room temperature; when it was detected by thin layer chromatography (TLC) (pure ethyl acetate) that the reaction was completed, the ethanol was completely evaporated under reduced pressure, a pH was adjusted with concentrated sulfuric acid to 2 to 3, and a resulting system was subjected to suction filtration to obtain a solid; the solid was washed and dried; 4-methylthiazole-5-carboxylic acid (1 mol) and DDC (1 mol) were added to anhydrous THF, and a resulting mixture was stirred at room temperature and subjected to activation for 2 h to 3 h; dimethylhydroxylamine hydrochloride (1.2 mol) was added, TEA (1.5 mol) was added dropwise, and a resulting mixture was stirred overnight at room temperature; when it was detected by TLC (PE : EA = 3:1) that the reaction was completed, THF was completely evaporated under reduced pressure, and extraction was conducted three times with ethyl acetate; a resulting organic phase was washed two times with a saturated sodium bicarbonate aqueous solution, dried with anhydrous magnesium sulfate, subjected to suction filtration, and subjected to evaporation under reduced pressure; a product (1 mol) was dissolved in anhydrous THF and then pre-cooled in a cold trap under nitrogen protection, and a Grignard reagent CH₃MgCl (1.5 mol) was added dropwise at - 10°C to 15°C; when it was detected by TLC (PE : EA = 3:1) that the reaction was completed, a resulting reaction system was quenched with saturated NH₄Cl, and extraction was conducted with ethyl acetate; a resulting organic phase was dried with anhydrous magnesium sulfate, subjected to suction filtration, and subjected to vacuum evaporation; and a crude product was subjected to vacuum distillation to obtain a pure product. NMR data for the product were as follows (as shown in FIG. 1):
¹H NMR (400 MHz, CDCl₃) δ 8.79 (s, 1H), 2.80 (s, 3H), 2.60 (s, 3H).

### Example 3 Synthesis of compound SMI7

Hydroxylamine hydrochloride (3 mmol) was added to a round-bottom flask, then 3 mL of ethanol was added, and a resulting mixture was stirred at 25°C for 10 min; then 3 mL of a 1 M NaOH solution was added, then SMIO (3 mmol) was added, and a reaction was conducted in an 80°C oil batch under reflux; when it was detected by TLC that the reaction was completed, a resulting reaction system was neutralized with 10% dilute hydrochloric acid, and extraction was conducted with water and ethyl acetate; resulting organic phases were combined, dried with anhydrous magnesium sulfate, and subjected to suction filtration to remove the magnesium sulfate; and a filtrate was subjected to vacuum concentration and then to silica gel column chromatography with petroleum ether and ethyl acetate in a ratio of 1:2 as an eluent to obtain a compound SMI7. NMR data of the product were as follows (as shown in FIG. 2):
¹H NMR (400 MHz, DMSO) δ 11.50 (s, 2/3H), 11.48 (s, 1/3H), 9.09 (s, 1/3H), 8.95 (s, 2/3H), 2.57 (s, 2/3H), 2.53 (s, 1/3H), 2.28 (s, 1/3H), 2.26 (s, 2/3H).

### Example 4 Synthesis of compound SMI16

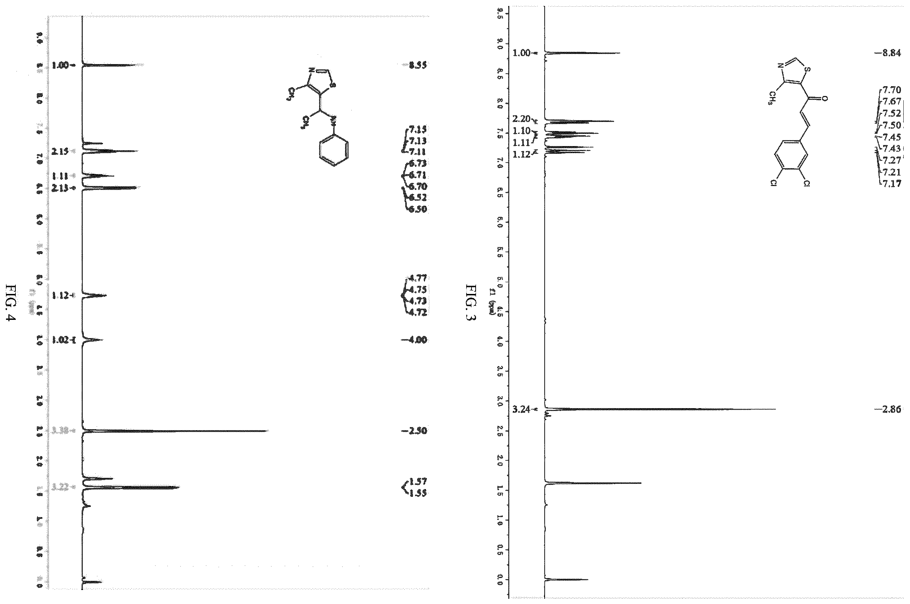

3,4-Dichlorobenzaldehyde (1 mmol) was added to a round-bottom flask, 2 mL of absolute ethanol was added, and a resulting mixture was stirred at 50°C for dissolution; 30 µL of a 3 M KOH solution was added, then SMIO (1 mmol) was added, and a resulting mixture was stirred at 50°C; when it was detected by TLC that the reaction was completed, a resulting reaction system was neutralized with 10% dilute hydrochloric acid, and extraction was conducted with water and ethyl acetate; resulting organic phases were combined, dried with anhydrous magnesium sulfate, and subjected to suction filtration to remove the magnesium sulfate; and a filtrate was subjected to vacuum concentration and then to silica gel column chromatography with petroleum ether and ethyl acetate in a ratio of 2:1 as an eluent to obtain a compound SMI16. NMR data of the product were as follows (as shown in FIG. 3):
¹H NMR (400 MHz, CDCl₃) δ 8.84 (s, 1H), 7.68 (d, J = 11.2 Hz, 2H), 7.51 (d, *J =* 8.3 Hz, 1H), 7.44 (d, *J* = 8.2 Hz, 1H), 7.19 (d, *J* = 15.5 Hz, 1H), 2.86 (s, 3H).

### Example 5 Synthesis of compound SMI20

Phenylamine (2 mmol) and SMIO (2 mmol) were added to a round-bottom flask, 3 mL of absolute ethanol was added, and a resulting mixture was stirred at 25°C for 10 min; BH₃CNNa (2 mmol) and acetic acid (1 mmol) were added, and a resulting mixture was stirred at 25°C; when it was detected by TLC that the reaction was completed, a resulting reaction system was neutralized with 10% dilute hydrochloric acid, and extraction was conducted with water and ethyl acetate; resulting organic phases were combined, dried with anhydrous magnesium sulfate, and subjected to suction filtration to remove the magnesium sulfate; and a filtrate was subjected to vacuum concentration and then to silica gel column chromatography with petroleum ether and acetone in a ratio of 4:1 as an eluent to obtain a compound SMI20. NMR data of the product were as follows (as shown in FIG. 4):
¹H NMR (400 MHz, CDCl₃) δ 8.55 (s, 1H), 7.13 (t, *J =* 7.7 Hz, 2H), 6.71 (t, *J =* 7.2 Hz, 1H), 6.51 (d, *J =* 7.9 Hz, 2H), 4.74 (q, *J =* 6.2 Hz, 1H), 4.00 (s, 1H), 2.50 (s, 3H), 1.56 (d, *J* = 6.6 Hz, 3H).

### Example 6 Synthesis of a hydrochloride of SMIO

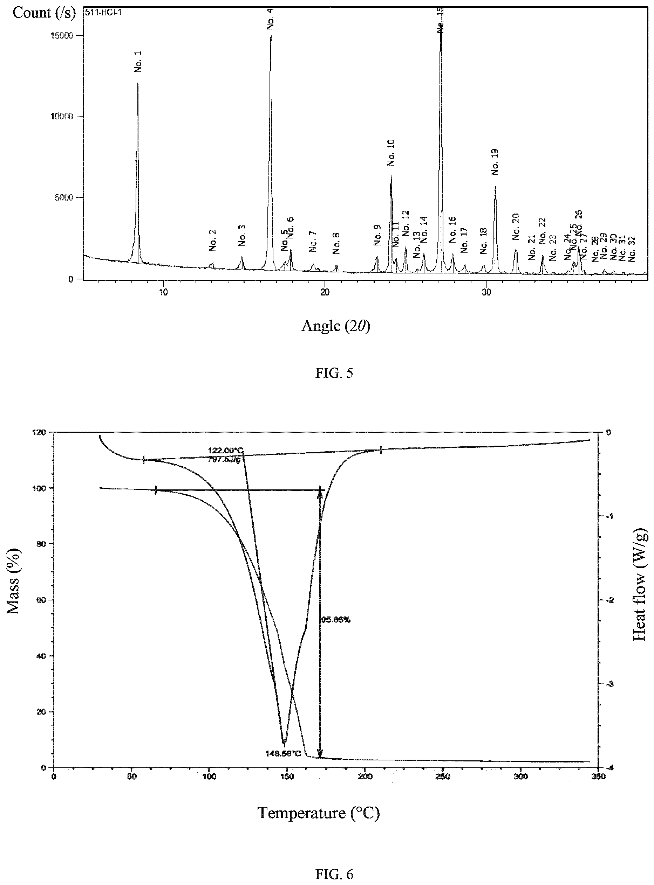

SMIO (1 g, 7.08 mmol) was added to a round-bottom flask, then 5 mL of ethanol was added for dissolution, and 11 mL of a 1 mol/L hydrochloric acid-ethanol solution was slowly added dropwise under stirring to allow a reaction for 1 h at room temperature; a resulting reaction system was subjected to vacuum concentration and cooled for crystallization, and a resulting mixture was filtered; and a filter cake was washed with 0.5 mL of cold absolute ethanol and then dried to obtain 1.06 g of a white solid, with a yield of 85%, a purity of 99.8% (HPLC), and a melting range of 160°C to 162°C. An XRPD pattern of a crystal form A of the hydrochloride of SMIO was shown in FIG. 5; and a DSC-TGA pattern of the crystal form A was shown in FIG. 6.

### Example 7 Synthesis of a sulfate of SMIO

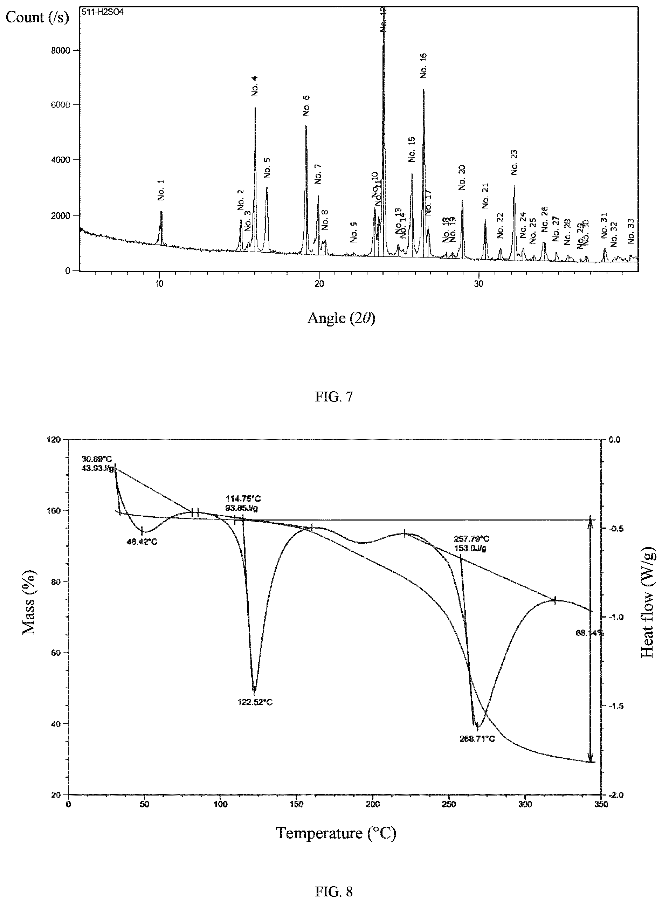

SMIO (1 g, 7.08 mmol) was added to a round-bottom flask, then 5 mL of ethanol was added for dissolution, and 8 mL of a 1 mol/L sulfuric acid-ethanol solution was slowly added dropwise under stirring to allow a reaction for 1 h at room temperature; and the solvent was completely evaporated, and then recrystallization was conducted with a small amount of methanol to obtain 0.85 g of a yellow solid, with a yield of 70% and a purity of 99.5% (HPLC). An XRPD pattern of a crystal form B of the sulfate of SMIO was shown in FIG. 7; and a DSC-TGA pattern of the crystal form B was shown in FIG. 8.

### Example 8 Determination of an inhibitory effect on metabolic activity of CYP2E1

CZX was used as a probe substrate to detect an inhibitory effect of an inhibitor to be tested on the metabolic activity of a mixed liver microsome CYP2E1 of a healthy individual (IC₅₀: half inhibition concentration; Kᵢ: inhibition constant; the smaller the IC₅₀ and the smaller the Kᵢ, the higher the inhibition intensity of the inhibitor), thereby determining an inhibitory effect of the inhibitor to be tested on the human liver microsome CYP2E1.

### Determination of IC₅₀ for a CYP2E1 inhibitory effect

An incubation system in a total volume of 100 µL was adopted, including a substrate, an inhibitor of different concentrations, a liver microsomal protein, and phosphate-buffered saline (PBS). The incubation system was pre-incubated in a 37°C water bath for 5 min, reduced coenzyme was added to allow a reaction for 30 min, and then the reaction was terminated in an ice bath. In a specific experiment, a concentration of an inhibitor can be selected as required.

In this example, the incubation system includes 62.5 µM CZX, 100 mM (pH = 7.4) PBS, 0.3 mg/mL liver microsomal protein, and 1 mM NADPH.

In other preferred examples, the incubation system may include 7.8 µM to 1,000 µM CZX as a substrate. A concentration of the liver microsomal protein may be 0.1 mg/mL to 0.5 mg/mL. 50 mM to 100 mM PBS or 50 mM to 100 mM Tris-HCL buffer can be used as required.

An NADPH regeneration system can also be used. Preferably, the incubation system includes 62.5 µM CZX, 100 mM (pH = 7.4) PBS, 0.3 mg/mL liver microsomal protein, and 1 mM NADPH.

In this example, the reaction is terminated with 1 mL of ethyl acetate. In other preferred examples, the reaction may also be terminated with 1 mL of methyl tert-butyl ether (MTBE), 1 mL of diethyl ether, or 100 µL of methanol.

### Determination of Kᵢ for a CYP2E1 inhibitory effect

When it was determined that an inhibitor had a prominent inhibitory effect on CYP2E1, different concentrations of a substrate and different concentrations of the inhibitor were selected to conduct an *in vitro* metabolic incubation inhibition test, and an inhibition constant Kᵢ of the inhibitor for the metabolism of CZX by CYP2E1 was calculated.

An incubation system in a total volume of 100 µL was adopted, including a substrate, an inhibitor of different concentrations, a liver microsomal protein, and PBS. The incubation system was pre-incubated in a 37°C water bath for 5 min, reduced coenzyme was added to allow a reaction for a specified time, and then the reaction was terminated in an ice bath.

In this example, the incubation system includes 15.6 µM, 31.25 µM, 62.5 µM, 125 µM, or 250 µM CZX; an inhibitor of a specified concentration determined based on the determined IC₅₀ according to a ratio of 1/4 to 4; 100 mM (pH=7.4) PBS; 0.3 mg/mL liver microsomal protein; and 1 mM NADPH.

In other preferred examples, the incubation system may include 7.8 µM to 1,000 µM CZX as a substrate, and more preferably, the incubation system may include 15.6 µM to 250 µM (15.6 µM, 31.25 µM, 62.5 µM, 125 µM, and 250 µM) CZX. A concentration of the liver microsomal protein may be 0.1 mg/mL to 0.5 mg/mL. 50 mM to 100 mM PBS or 50 mM to 100 mM Tris-HCL buffer can be used as required.

1 mM NADPH or an NADPH regeneration system can also be used. The NADPH regeneration system includes 1.3 mM NADP⁺, 3.3 mM glucose-6-phosphate, 0.4 U/mL glucose dehydrogenase (GDH), and 3.3 mM magnesium chloride.

In this example, the reaction is terminated with 1 mL of ethyl acetate. In other preferred examples, the reaction may also be terminated with 1 mL of MTBE, 1 mL of diethyl ether, or 100 µL of methanol.

### Determination of selectivity of a CYP2E1 inhibitor

With a mixed liver microsome of a healthy individual as a research object, probe drugs CYP1A2, CYP2A6, CYP2B6, CYP2C8, CYP2C9, CYP2C19, CYP2D6, CYP2E1, and CYP3A4 were selected to determine an *in vitro* inhibitory effect of an inhibitor to be tested on the metabolism of the probe drugs CYP1A2, CYP2A6, CYP2B6, CYP2C8, CYP2C9, CYP2C19, CYP2D6, CYP2E1, and CYP3A4 for the mixed liver microsome of the healthy human, and the selectivity of the inhibitor to be tested for a CYP2E1 inhibitory effect was evaluated.

The CYP1A2 probe is 6.25 µM to 800 µM phenacetin or 27.5 µM to 12,520 µM caffeine;
the CYP2A6 probe is 0.156 µM to 20 µM coumarin or 12.5 µM to 2,000 µM nicotine;
the CYP2B6 probe is 7.8 µM to 500 µM bupropion or 0.25 mM to 30 mM cyclophosphamide;
the CYP2C8 probe is 2.5 µM to 80 µM paclitaxel or 0.125 µM to 128 µM amodiaquine;
the CYP2C9 probe is 31.25 µM to 2,000 µM tolbutamide or 0.1 µM to 200 µM diclofenac;
the CYP2C19 probe is 3.9 µM to 500 µM omeprazole or 1.95 µM to 1,000 µM mephenytoin;
the CYP2D6 probe is 0.625 µM to 96 µM dextromethorphan or 0.0195 µM to 80 µM propafenone; and
the CYP3A4 probe is 0.39 µM to 50 µM midazolam or 1.98 µM to 1,000 µM testosterone.

An incubation system in a total volume of 100 µL was adopted, including a substrate, an inhibitor of different concentrations, a liver microsomal protein, and PBS. The incubation system was pre-incubated in a 37°C water bath for 5 min, reduced coenzyme was added to allow a reaction for a specified time, and then the reaction was terminated in an ice bath.

In this example, the incubation system includes at least one selected from the group consisting of 62.5 µM phenacetin, 2.5 µM coumarin, 62.5 µM bupropion, 10 µM paclitaxel, 250 µM tolbutamide, 62.5 µM omeprazole, 20 µM dextromethorphan, 62.5 µM CZX, and 1.56 µM midazolam; 100 mM (pH = 7.4) PBS; 0.3 mg/mL liver microsomal protein; and 1 mM NADPH.

In other preferred examples, the incubation system may include 7.8 µM to 1,000 µM CZX as a substrate. A concentration of the liver microsomal protein may be 0.1 mg/mL to 0.5 mg/mL. The buffer may be one selected from the group consisting of 50 mM PBS, 100 mM PBS, 50 mM Tris-HCL buffer, and 100 mM Tris-HCL buffer. 1 mM NADPH or an NADPH regeneration system can also be used.

In this example, the reaction is terminated with 1 mL of ethyl acetate. In other preferred examples, the reaction may also be terminated with 1 mL of MTBE, 1 mL of diethyl ether, or 100 µL of methanol.

### Example 9 In vitro screening test results of CYP2E1 inhibitors

SMIO (IC₅₀ for 2E1 inhibition: 1.64 µM; IC₅₀ for 2A6 inhibition: 76.20 µM; and no significant inhibitory effect for other CYP enzymes):

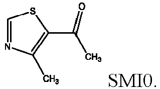

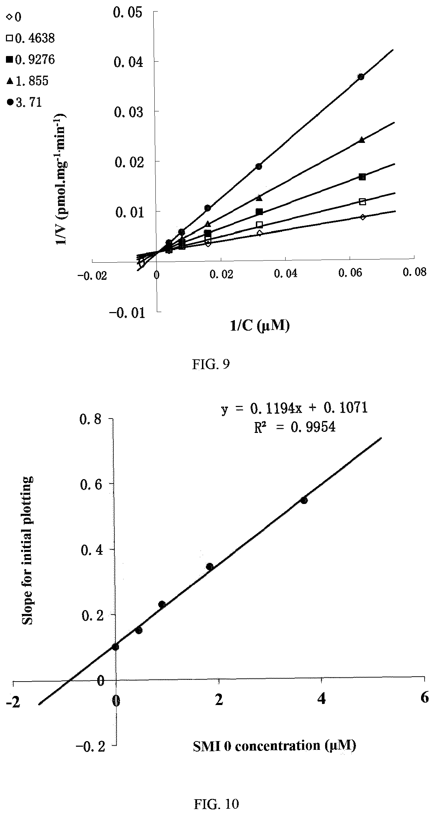

As shown in FIG. 9, a 100 µL incubation system is adopted for all reactions, with CZX concentrations of 15.6 µM, 31.2 µM, 62.5 µM, 125 µM, and 250 µM and SMIO series concentrations of 0 µM, 0.4638 µM, 0.9276 µM, 1.855 µM, and 3.710 µM. All experimental data are derived from a mean of three independent experiments. FIG. 9 is a double-reciprocal plot of an inhibitory effect of the compound SMIO on human hepatic metabolism of CZX. It indicates that the compound SMIO is a hybrid inhibitor for CYP2E1.

FIG. 10 is a secondary plot based on the double-reciprocal plot of an inhibitory effect of the compound SMIO on metabolism of CZX by human hepatic CYP2E1. The series concentrations of SMIO (0 µM, 0.4638 µM, 0.9276 µM, 1.855 µM, and 3.710 µM) were subjected to linear regression with slopes of different inhibition curves, and an absolute value of an intersection point between a straight line and a horizontal axis was Kᵢ, which was 0.8970 µM.

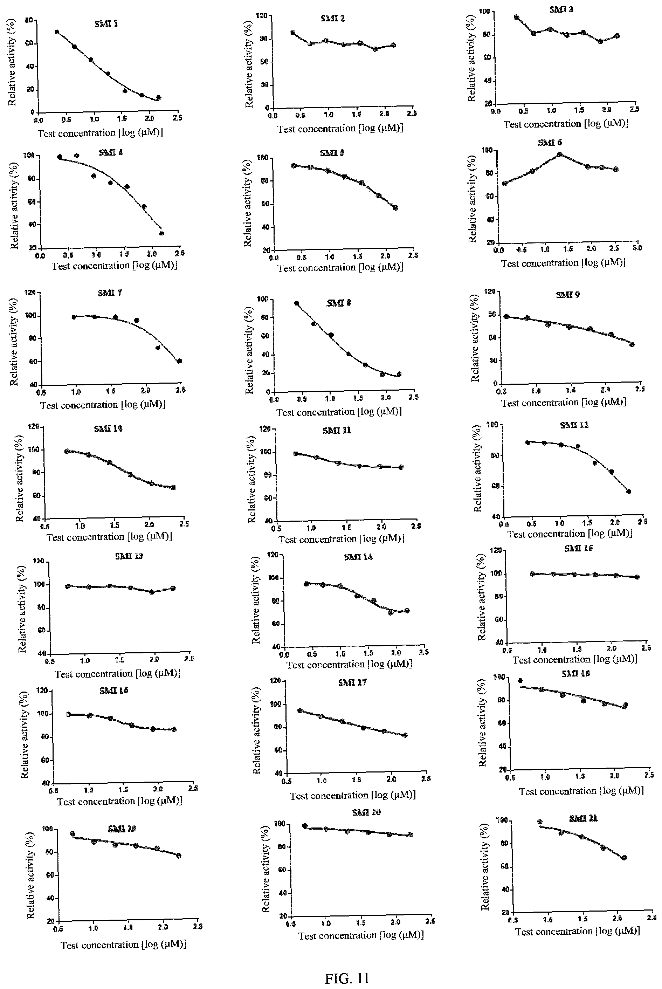

With reference to the specific research method in Example 8, inhibitory effects of 21 small molecule compounds (Table 1) on CYP2E1 were tested *in vitro.* Results showed that SMI1 and SMI8 each had a significant inhibitory effect on CYP2E1, and IC₅₀ values of SMI1 and SMI8 for CYP2E1 inhibition were 7.99 µM and 17.03 µM, respectively; SMI10 had a slight inhibitory effect on CYP2E1, with IC₅₀ of 114.5 µM, and compared with SMI0, SMI1, SMI8, and SMI10, other small molecules had a weak inhibitory effect on CYP2E1 (as shown in FIG. 11).

In addition, selectivity studies showed that IC₅₀ of the inhibition of SMI1 for CYP2A6 was 55.63 µM and IC₅₀ of the inhibition of SMI1 for CYP2C9 was 1.86 µM. Structures of the 21 small molecules were shown in Table 1.

**Table 1**

| No. | Structural formula |
|---|---|
| SMI1 | 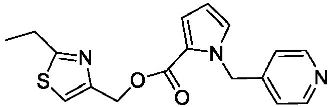 |
| SMI2 | 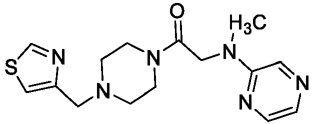 |
| SMI4 | 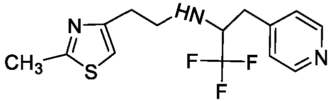 |
| SMI5 | 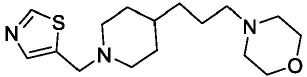 |
| SMI6 | 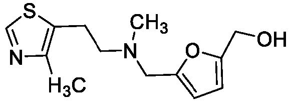 |
| SMI7 | 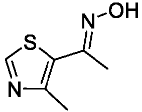 |
| SMI8 | 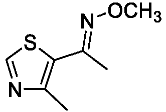 |
| SMI9 | 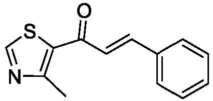 |
| SMI10 | 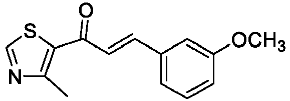 |
| SMI11 | 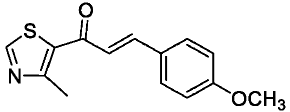 |
| SMI12 | 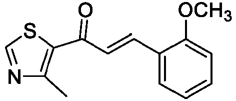 |
| SMI13 | 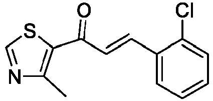 |
| SMI14 | 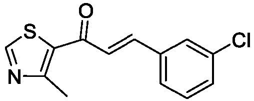 |
| SMI15 | 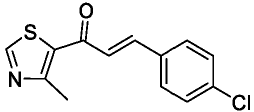 |
| SMI16 | 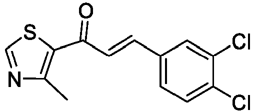 |
| SMI17 | 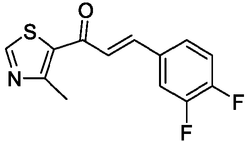 |
| SMI18 | 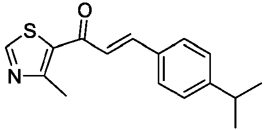 |
| SMI19 | 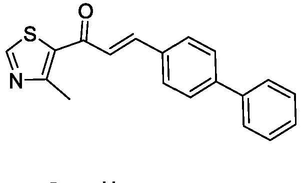 |
| SMI20 | 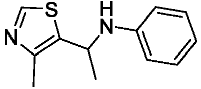 |
| SMI21 | 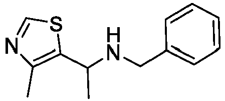 |

### Example 10 In vivo inhibition test results of the compound SMIO for CYP2E1

Experimental method: An inhibitory effect of the compound SMIO on CYP2E1 in rats was investigated by a self-controlled crossover experiment design. In a first round of experiments, DEN alone was intraperitoneally injected at 50 mg/kg (30 SD rats in total) in the first week; after a one-week interval, a second round of experiments were conducted as follows: each rat was first intragastrically administered with the compound SMIO at a low, medium, or high dose (6 mg/kg, 30 mg/kg, or 150 mg/kg), and 5 min later, each rat was intraperitoneally injected with DEN at 50 mg/kg (10 rats were set for each of low, medium, and high doses); blood was collected at 2 min, 7 min, 15 min, and 30 min and 1 h, 2 h, 4 h, 6 h, 9 h, 12 h, 24 h, 36 h, 48 h, and 60 h; a DEN concentration in plasma was determined at different blood collection time points, and a toxicokinetic parameter of DEN was calculated; and plasma DEN was detected by high performance liquid chromatography (HPLC) as follows: 100 µL of plasma was added to 10 µL of perchloric acid, a resulting mixture was vortexed for 3 min and then centrifuged at 12,000 rpm for 10 min, and 10 µL of a resulting supernatant was collected and injected into an instrument, where the HPLC was conducted with methanol : water = 50:50 as a mobile phase and a detection wavelength of 240 nm.

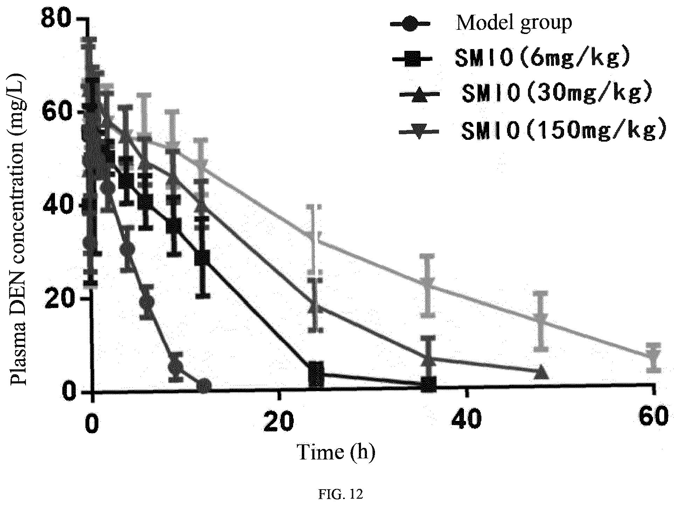

Experimental results: The toxicokinetic parameter of DEN was used to represent CYP2E1 activity, and the influence of the compound SMIO at low, medium, and high doses on CYP2E1 activity in rats was analyzed. The results showed that, compared with the model group in which DEN was administered alone, SMIO at low, medium, and high doses could reduce a clearance (CL) of DEN by (63.98 ± 7.78)%, (79.63 ± 7.29)%, and (85.42 ± 3.74%)% with inhibition rates of 62.43%, 80.42%, and 86.77%, respectively (as shown in FIG. 12 and Table 2, **P* < 0.05 and ****P* < 0.001 vs model group); similarly, SMIO at low, medium, and high doses could extend a half-life t_{1/2} of DEN by (133.60 ± 116.80)%, (619.97 ± 363.57)%, and (868.70 ± 241.26)%, increase AUC₀₋ₜ by (196.82 ± 73.63)%, (407.70 ± 184.30)%, and (529.67 ± 153.72)%, and increase AUC_{0-∞}. by (189.99 ± 67.55)%, (455.90 ± 219.26)%, and (626.56 ± 187.15)%, respectively; and the compound SMIO at a medium dose exhibited a significantly better inhibitory effect than the compound SMIO at a low dose, and the compound SMIO at a high dose exhibited a significantly better inhibitory effect than the compound SMIO at low and medium doses (^{#}*P* < 0.05, *^{##}P* < 0.01, *^{###}P <* 0.001 vs low-dose SMIO group; ^{$}*P* < 0.05, ^{$$$}*P* < 0.001 vs medium-dose SMIO group), indicating that the compound SMIO at different doses could significantly inhibit the metabolic activity of CYP2E1 for DEN in rats, and there was a prominent dose-dependent relationship.

It can be seen from the above results that SMIO has a significant inhibitory effect on CYP2E1 activity in rats.

**Table 2 Toxicokinetic parameters for the inhibition of the compound SMIO on in vivo metabolism of DEN by CYP2E1**

| Rat | Cₘₐₓ | Tₘₐₓ | t_{1/2} | V_{d} | CL | AUC₀₋ₜ | AUC_{0-∞} |
|---|---|---|---|---|---|---|---|
| | mg/L | h | h | L/kg | L/h/kg | mg/L*h | mg/L*h |
| Model group | | | | | | | |
| Mean | 55.99 | 0.33 | 2.11 | 0.556 | 0.189 | 260.55 | 270.59 |
| SD | 5.17 | 0.19 | 0.92 | 0.192 | 0.031 | 38.45 | 43.26 |

| SMIO (6mg/kg) | | | | | | | |
|---|---|---|---|---|---|---|---|
| Mean | 60.97 | 0.21 | 4.50*** | 0.455 | 0.071*** | 701.75*** | 724.58*** |
| SD | 7.43 | 0.16 | 1.29 | 0.156 | 0.012 | 105.91 | 112.50 |

| SMIO (30 mg/kg) | | | | | | | |
|---|---|---|---|---|---|---|---|
| Mean | 68.10**^{#} | 0.38 | 11.93***^{###} | 0.580^{#} | 0.037***^{###} | 1304.93***^{##} | 1476.49***^{##} |
| SD | 6.84 | 0.62 | 5.18 | 0.062 | 0.011 | 483.24 | 603.32 |

| SMI0 (150 mg/kg) | | | | | | | |
|---|---|---|---|---|---|---|---|
| Mean | 70.70* | 0.70 | 19.69***^{###$$$} | 0689*^{##$} | 0.025***^{###$} | 1749.59*** ^{###$} | 2066.53***^{###$} |
| SD | 13.15 | 0.82 | 4.94 | 0.110 | 0.006 | 352.96 | 474.90 |

Notes: In Table 2, mean represents an average value, and SD represents a standard deviation.

Cₘₐₓ represents a peak concentration of a drug, which is the highest blood drug concentration after administration; and this parameter is an important index to reflect an absorption rate and an absorption degree of a drug in the body.

Tₘₐₓ represents a time to peak (TTP), which is a time required to reach a peak concentration after administration; and this parameter reflects a speed at which a drug enters the body, and the higher the absorption rate, the shorter the peak time.

V_{d} represents an apparent volume of distribution, which is a ratio constant of a drug amount in the body to a blood drug concentration when a drug reaches a dynamic equilibrium in the body and has a unit generally of L; and this parameter reflects a distribution range of a drug in the body, and the larger the value, the wider the distribution. The apparent volume of distribution is numerically obtained through a ratio of a clearance to a terminal elimination rate.

CL represents a clearance, which is an apparent volume of distribution of a drug cleared from the body per unit time and has a unit generally of L/h; and this parameter is an important parameter to reflect a treatment characteristic of the body for a drug and is closely related to physiological factors. A clearance is obtained based on a ratio of a dose to AUC_{(0-∞)}.

AUC represents an area under the concentration-time curve, which is an area surrounded by a blood drug concentration curve and a time axis; and this parameter is an important index to evaluate an absorption degree of a drug, and reflects an exposure characteristic of a drug in the body. Since a blood drug concentration can only be observed until a specified time point t in pharmacokinetic studies, AUC has two representations: AUC₍₀₋ₜ₎ and AUC_{(0-∞)}, where the former is obtained according to a trapezoidal area method and the latter is calculated according to the following equation: AUC_{(0-∞)} = AUC₍₀₋ₜ₎ + terminal point concentration/terminal elimination rate.

### Example 11 Inhibition of the compound SMIO on DEN-induced rat liver damage

Experimental method: An SD rat liver damage model was constructed through intermittent intraperitoneal injection of DEN. A model group was injected intraperitoneally with DEN at 50 mg/kg twice a week in the first 4 weeks and then once a week in the 5th to 8th weeks. SMIO intervention groups, which were injected with DEN at the amount and the way the same as that in the model group, included SMIO + DEN low-dose and high-dose groups and an SMIO high-dose continuous administration group, where the SMIO + DEN low-dose and high-dose groups were intragastrically administered with the compound SMIO at 30 mg/kg and 150 mg/kg 5 min before DEN administration each time, respectively; and the SMIO high-dose continuous administration group was intragastrically administered with the compound SMIO at 150 mg/kg every day from the 1st week to the 8th week. At the end of molding, blood was collected from the orbit, and plasma liver function indexes of rats were determined by an automatic biochemical analyzer (model group, n =10 rats; SMIO compound low-dose group, n = 21 rats; (150 mg/kg)^{a}, SMIO compound + DEN high-dose group, n = 28 rats; and (150 mg/kg)^{b}, SMIO compound high-dose continuous administration group, n = 24 rats].

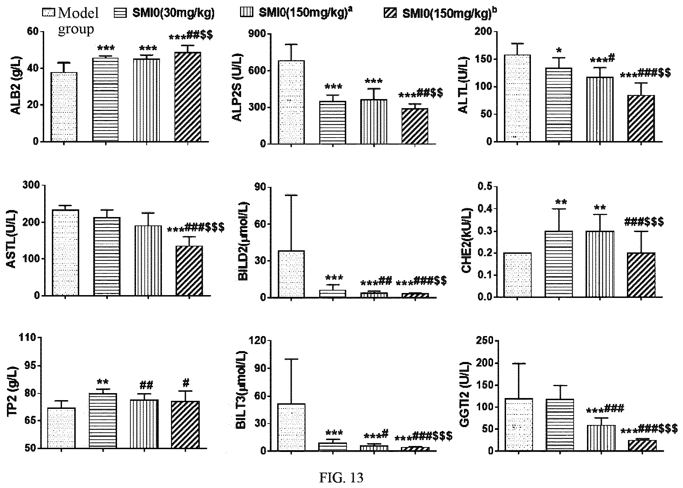

Experimental results: Compared with the model group, in the SMIO intervention group, the albumin (ALB2) and cholinesterase (CHE2) levels were significantly increased, and the alkaline phosphatase (ALP) (ALP2S), alanine aminotransferase (ALTL), direct bilirubin (BILD2), total bilirubin (BILT3), and glutamyl transpeptidase (GGTI2) levels were significantly reduced; and the TP2 level was significantly increased in the SMIO low-dose group, and the GGTI2 level was significantly reduced in the SMIO + DEN high-dose group and the SMIO high-dose continuous administration group (as shown in A to I of FIG. 13, **P* < 0.05, ***P* < 0.01, and ***P < 0.001 vs model group; and 150 mg/kg^{a} was for the SMIO + DEN high-dose group, and 150 mg/kg^{b} was for the SMIO high-dose continuous administration group); compared with the SMIO low-dose group, in the SMIO + DEN high-dose group, the ALTL, BILD2, BILT3, and GGTI2 levels were significantly reduced, and the TP2 level was significantly increased; and in the SMIO high-dose continuous administration group, the ALB2 level was significantly increased, and the ALP2S, ALTL, ASTL, BILD2, CHE2, BILT3, and GGTI2 levels were significantly reduced (^{##}*P* < 0.01, *^{###}P* < 0.001 vs SMIO low-dose group); and compared with the SMIO + DEN high-dose group, in the SMIO high-dose continuous administration group, the ALB2 level was significantly increased, and the ALP2S, ALTL, ASTL, BILD2, CHE2, BILT3, and GGTI2 levels were significantly reduced (^{$}*P* < 0.05 and ^{$$$}*P* < 0.001 vs SMIO + DEN high-dose group), indicating that the SMIO administration can significantly improve the DEN-induced rat liver damage.

It can be seen from the above results that SMIO has a significant prevention and treatment effect on the DEN-induced rat liver damage, indicating that the compound SMIO can be used for the prevention and treatment of clinical liver damage.

### Example 12 Inhibition of SMIO on hepatic steatosis and hepatitis in mice caused by a high-fat diet

Experimental method: Healthy male C57/6J mice were fed with a high-fructose, high-fat, and high-cholesterol feed to prepare mouse hepatic steatosis and hepatitis models, and a control group was fed with a low-fat and low-sugar control feed. An SMIO intervention group was divided into a low-dose intervention group and a high-dose intervention group, which were intragastrically administered with the compound SMIO at 30 mg/kg and 150 mg/kg every day from the beginning of modeling to the 22nd week of modeling. At the end of the 22-week experiment, blood was collected from the orbit, a body weight was measured, and then the mice each were sacrificed. The weight and appearance of a liver (color, texture, and presence or absence of nodules of the liver) were recorded. Some liver specimens were subjected to HE staining and then observed, and a steatosis score of mice in each group was recorded; and Masson staining was conducted to evaluate a liver fibrosis status of mice, and an oil red O staining area percentage and an LCA staining inflammation score were determined (model group, n = 8 mice; SMIO compound low-dose group, n = 10 rats; and SMIO compound high-dose group, n = 10 rats).

The pathological results of HE staining of a liver tissue were subjected to quantitative scoring based on the following criteria to determine a steatosis severity:

| | |
|---|---|
| (1) steatosis area less than 5% | 0 point |
| (2) steatosis area: 5% to 20% | 1 point |
| (3) steatosis area: 20% to 35% | 2 points |
| (4) steatosis area: 35% to 50% | 3 points |
| (5) steatosis area: 50% to 65% | 4 points |
| (6) steatosis area: 65% to 80% | 5 points |
| (7) steatosis area larger than 80% | 6 points. |

The pathological results of Masson staining of a liver tissue were subjected to quantitative scoring based on the following criteria to determine a fibrosis severity:

| | |
|---|---|
| (1) no fibrosis | 0 point |
| (2) in area 3, there is weak perisinusoidal fibrogenesis | 1 point |
| (3) in area 3, there is medium-intensity perisinusoidal fibrogenesis | 2 points |
| (4) there is obvious fibrogenesis in or around the portal vein | 3 points |
| (5) there is obvious fibrogenesis around the sinus and in or around the portal vein | 4 points |
| (6) fiber bridging formation | 5 points |
| (7) cirrhosis formation | 6 points |

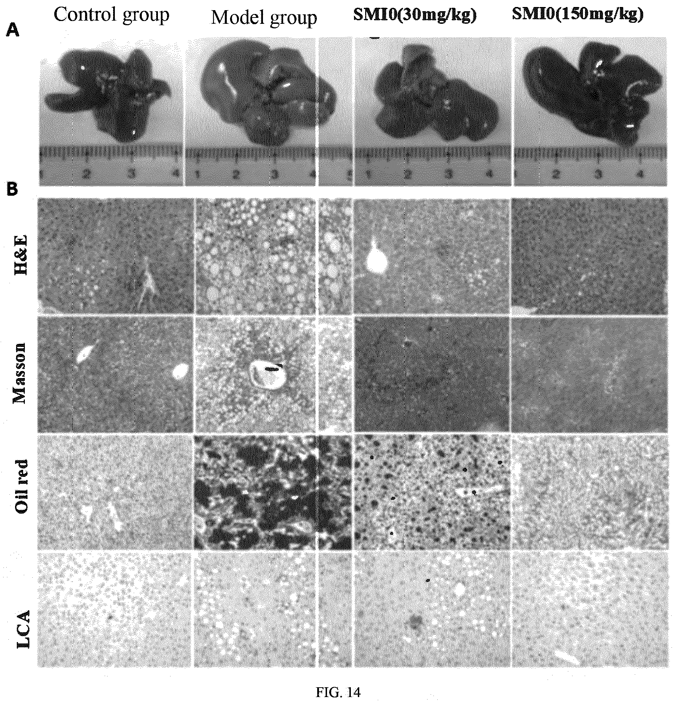

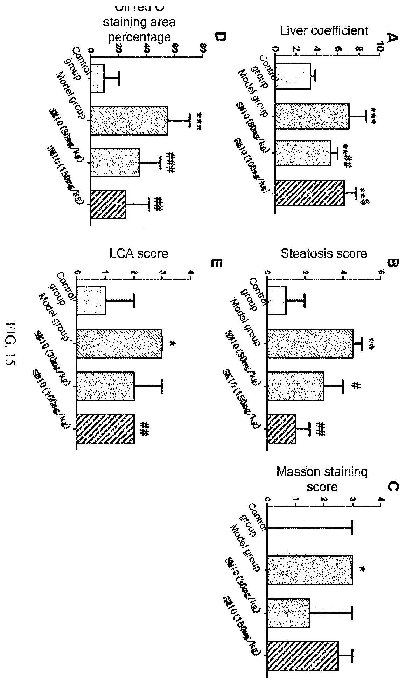

Experimental results: Compared with the control group, in the model group, the liver coefficient, steatosis degree (steatosis score and oil red O staining area percentage), and liver inflammation level (number of LCA-positive cell staining foci) were significantly increased to varying degrees (as shown in A to B of FIG. 14 and A to E of FIG. 15, **P* < 0.05, ***P* < 0.01, and ***P < 0.001 vs the control group; and scales in FIG. 14 each were 100 µm); and compared with the model group, in the SMIO low-dose intervention group, the liver coefficient and steatosis degree (steatosis score and oil red O staining area percentage) were significantly reduced (^{#}*P* < 0.05, *^{##}P* < 0.01, and *^{###}P* < 0.001 vs the model group), and in the SMIO high-dose intervention group, the steatosis degree (steatosis score and oil red O staining area percentage) and liver inflammation level (number of LCA-positive cell staining foci) were significantly reduced, indicating that the compound SMIO can significantly reduce the hepatic steatosis and inflammation induced by a high-fat diet.

It can be seen from the above results that SMIO has a significant prevention and treatment effect on the hepatic steatosis and hepatitis induced by a high-fat diet, indicating that the compound SMIO can be used for the prevention and treatment of clinical fatty liver and hepatitis.

### Example 13 Inhibition of SMIO on the occurrence and development of liver fibrosis

### (1) Inhibition of SMIO on liver fibrosis in mice induced by a high-fat diet

Experimental method: Male C57/6J mice were fed with a high-fructose, high-fat, and high-cholesterol feed to prepare mouse hepatic steatosis and hepatitis models, and a control group was fed with a low-fat and low-sugar control feed. An SMIO intervention group was divided into a low-dose intervention group and a high-dose intervention group, which were intragastrically administered with the compound SMIO at 30 mg/kg and 150 mg/kg every day from the beginning of modeling to the 26nd week of modeling. At the end of the 26-week experiment, blood was collected from the orbit, a body weight was measured, and then the mice each were sacrificed. The weight and appearance of a liver (color, texture, and presence or absence of nodules of the liver) were recorded. Some liver specimens were subjected to HE staining and then observed, and a steatosis score of mice in each group was recorded; and Masson staining was conducted to evaluate a liver fibrosis status of mice, and an oil red O staining area percentage was determined (model group, n = 10 mice; SMIO compound low-dose group, n = 10 rats; and SMIO compound high-dose group, n = 10 rats).

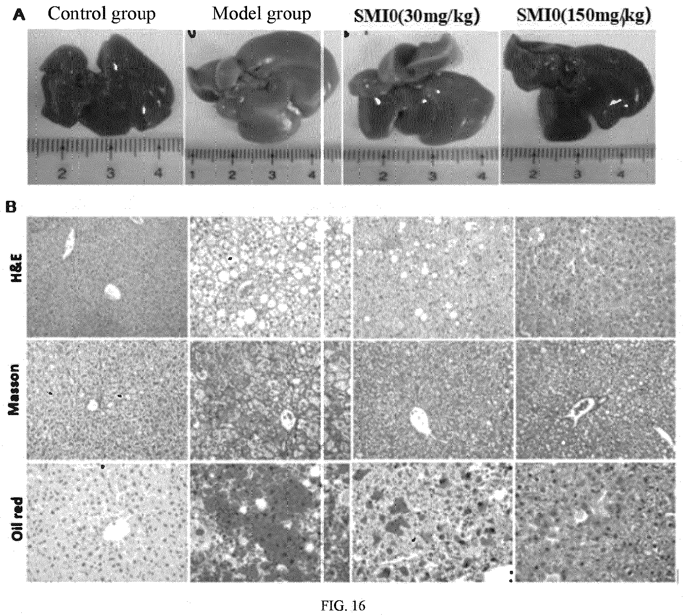

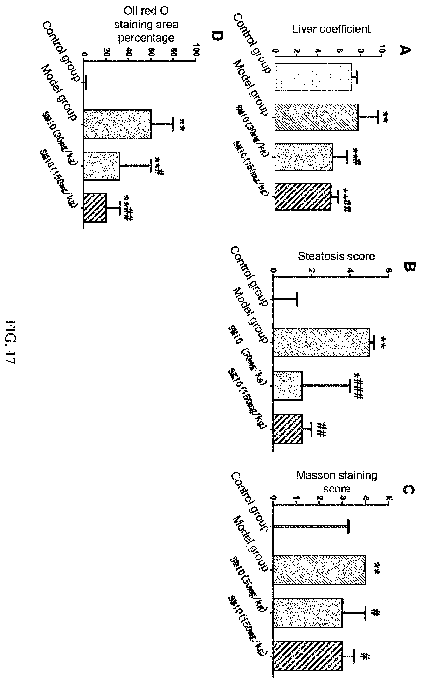

Experimental results: Compared with the control group, in the model group, the liver coefficient, index reflecting a steatosis degree (steatosis score and oil red O staining area percentage), and Masson staining score reflecting a fibrosis degree were significantly increased to varying degrees (as shown in A to B of FIG. 16 and A to D of FIG. 17, **P* < 0.05 and ***P* < 0.01 vs the control group; and in B of FIG. 16, HE and Masson staining scales each were 100 µm, and an oil red staining scale was 50 µm); and compared with the model group, in the SMIO low-dose and high-dose intervention groups, the steatosis degree (steatosis score and oil red O staining area percentage) and fibrosis degree (Masson staining score) (^{#}*P* < 0.05, *^{##}P* < 0.01, and *^{###}P* < 0.001 vs the model group), indicating that the compound SMIO can significantly reduce a liver fibrosis level in mice induced by a high-fat diet.

It can be seen from the above results that SMIO has a significant prevention and treatment effect on the liver fibrosis in mice induced by a high-fat diet, indicating that the compound SMIO can be used for the prevention and treatment of clinical liver fibrosis.

### (2) Inhibition of SMIO on DEN-induced liver fibrosis in rats

Experimental method: An SD rat liver damage model was constructed through intermittent intraperitoneal injection of DEN. A model group was injected intraperitoneally with DEN at 50 mg/kg twice a week in the first 4 weeks and then once a week in the 5th to 12th weeks. SMIO intervention groups, which were injected with DEN at the amount and the way the same as that in the model group, included SMIO + DEN low-dose and high-dose groups and an SMIO high-dose continuous administration group, where the SMIO + DEN low-dose and high-dose groups were intragastrically administered with the compound SMIO at 30 mg/kg and 150 mg/kg 5 min before DEN administration each time, respectively; and the SMIO high-dose continuous administration group was intragastrically administered with the compound SMIO at 150 mg/kg every day from the 1st week to the 12th week. At the end of molding, blood was collected from the orbit, and plasma liver function indexes of rats were determined by an automatic biochemical analyzer (model group, n = 10 rats; SMIO compound low-dose group, n = 21 rats; SMIO compound + DEN high-dose group, n = 28 rats; and SMIO compound high-dose continuous administration group, n = 24 rats).

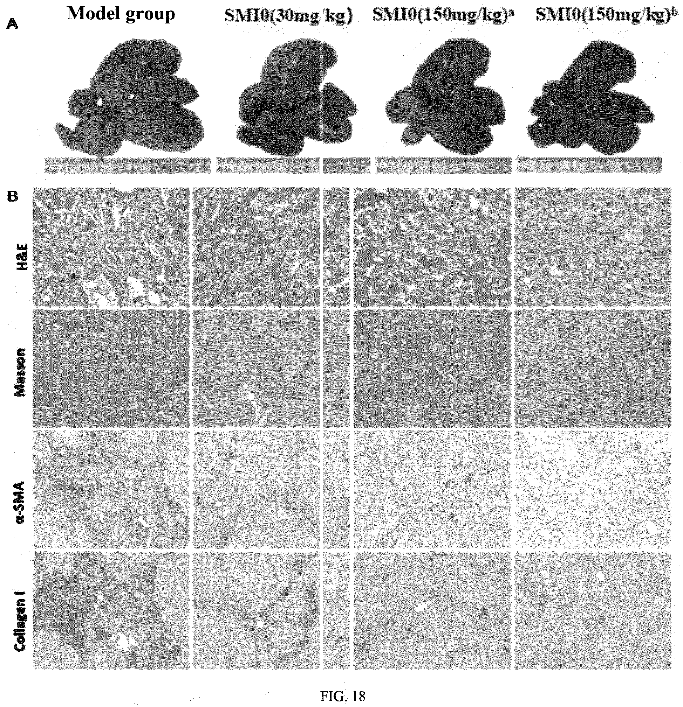

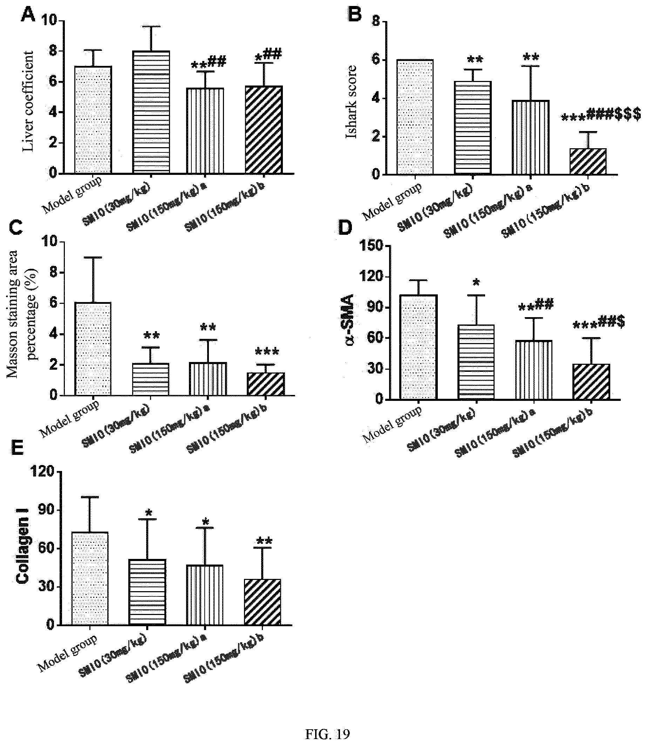

Experimental results: Compared with the model group, in the SMIO intervention group, the liver coefficient, liver fibrosis Ishark score, liver fibrosis Masson staining area percentage, α-SMA, and Collagen I were reduced to varying degrees (as shown in A to B of FIG. 18 and A to E of FIG. 19, **P* < 0.05, ***P* < 0.01, and ***P < 0.001 vs the model group; and scales in B of FIG. 18 each were 100 µm); and the SMIO high-dose (150 mg/kg^{b}) continuous administration group exhibited a significantly better effect than the SMIO + DEN high-dose group (150 mg/kg^{a}) and the SMIO low-dose group (^{##}*P* < 0.01 and *^{###}P* < 0.001 vs the SMIO low-dose group; and ^{&}*P* < 0.05 and *^{&&&}P* < 0.001 vs the SMIO + DEN high-dose group).

It can be seen from the above results that SMIO has a significant prevention and treatment effect on the DEN-induced rat liver fibrosis, indicating that the compound SMIO can be used for the prevention and treatment of clinical liver fibrosis.

### Example 14 Inhibition of SMIO on the occurrence and development of pulmonary fibrosis in mice

### (1) Inhibition of CYP2E1 gene knockout on pulmonary fibrosis in mice

Experimental method: A C57BL/6N mouse pulmonary fibrosis model was established through intratracheal instillation with 8 mg/kg LPS. C57BL/6N mice were divided into a control group, a model group, and a CYP2E1 gene-knockout group, and the modeling took 28 days (control group, n = 8; model group, n = 8; and gene knockout group, n=8). At the end of the experiment, a body weight of mice in each group was recorded, blood was collected from the orbit, the mice were sacrificed, and lung tissues were collected and weighed. The left lungs were fixed in 4% formalin and subjected to HE and Masson staining, and the lung tissues were subjected to fibrosis grading and fibrosis and histological scoring to reflect a severity of pulmonary fibrosis.

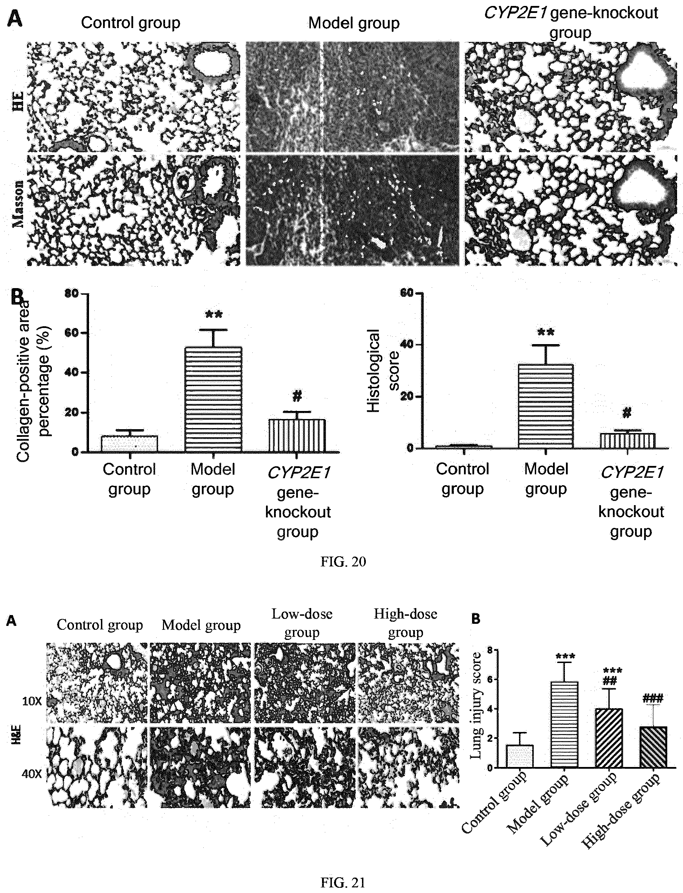

Experimental results: Compared with the control group, in the model group, the lung index of mice was significantly increased, the alveolar septum was pathologically thickened, the alveolar chamber shrank or even disappeared, the lung parenchyma underwent obvious inflammatory cell infiltration, and the fibrosis score and histological score were significantly increased; and compared with the model group, in the CYP2E1 gene-knockout group, the lung index of mice was significantly reduced, there was no obvious pathological inflammatory cell infiltration, and the fibrosis score and histological score were significantly reduced (as shown in A to B of FIG. 20), indicating that the CYP2E1 gene knockout can significantly inhibit the occurrence of LPS-induced pulmonary fibrosis.

### (2) Intervention of SMIO in lung injury in mice

Experimental method: A C57BL/6N mouse pulmonary fibrosis model was established through intratracheal instillation with 8 mg/kg LPS. An SMIO intervention group was divided into a low-dose group and a high-dose group, which were intragastrically administered with the compound SMIO respectively at 30 mg/kg and 90 mg/kg every day from three days before modeling until 6 h before the end of the modeling (control group, n = 8; model group, n = 8; and SMIO compound high-dose group, n = 8). At the end of the experiment, a body weight of mice in each group was recorded, blood was collected from the orbit, the mice were sacrificed, and lung tissues were collected and weighed. The left lungs were fixed in 4% formalin and subjected to HE staining.

Experimental results: Compared with the control group, in the model group, the lung index of mice was significantly increased (P < 0.001); and compared with the model group, in both the SMIO low-dose and high-dose groups, the lung index of mice was significantly reduced (P < 0.01), indicating that SMIO can significantly reduce the increase of lung index in mice with LPS-induced acute lung injury (ALI). Similarly, compared with the control group, in the model group, the lung tissue structure of mice was obviously damaged, which was mainly manifested as alveolar edema, alveolar wall thickening, alveolar chamber shrinkage, and infiltration of a large number of inflammatory cells, and the lung injury score was significantly increased; and compared with the model group, in the SMIO administration group, there was alleviated pulmonary edema, narrowed alveolar septum, reduced inflammatory cell infiltration, and normalized lung tissue results, and the lung injury score was significant reduced (as shown in A to B of FIG. 21), indicating that SMIO can mitigate the LPS-induced ALI in mice.

### (3) Intervention of SMIO in pulmonary fibrosis in mice

Experimental method: A C57BL/6N mouse pulmonary fibrosis model was established through intratracheal instillation with 8 mg/kg LPS. An SMIO intervention group was divided into a low-dose group and a high-dose group, which were intragastrically administered with the compound SMIO respectively at 30 mg/kg and 90 mg/kg every day from three days before modeling to the end of the 28-d modeling (control group, n = 8; model group, n = 8; and SMIO compound high-dose group, n = 8). At the end of the experiment, a body weight of mice in each group was recorded, blood was collected from the orbit, the mice were sacrificed, and lung tissues were collected and weighed. The left lungs were fixed in 4% formalin and subjected to HE and Masson staining, and the lung tissues were subjected to fibrosis grading and fibrosis and histological scoring to reflect a severity of pulmonary fibrosis.

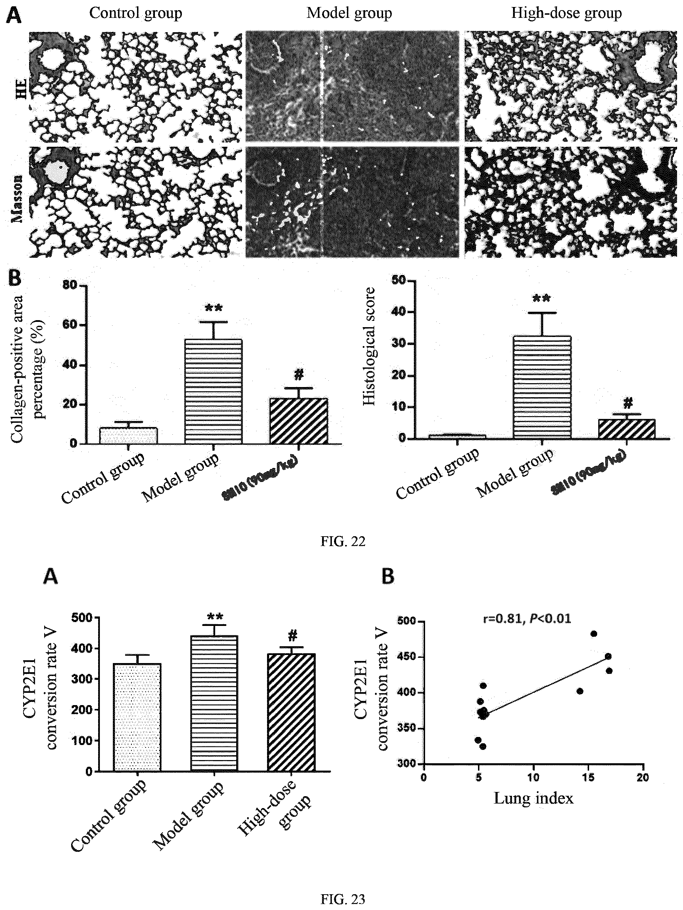

Experimental results: Compared with the control group, in the model group, the lung index of mice was significantly increased, the alveolar septum was pathologically thickened, the alveolar chamber shrank or even disappeared, and the lung parenchyma underwent obvious inflammatory cell infiltration; and compared with the model group, in the SMIO intervention group, the lung index of mice was significantly reduced, and there was no obvious pathological inflammatory cell infiltration (as shown in A to B of FIG. 22), indicating that the administration of SMIO can significantly inhibit the occurrence of LPS-induced pulmonary fibrosis.

It can be seen from the above results that the CYP2E1 inhibitor SMIO has a significant prevention and treatment effect on the occurrence and development of pulmonary fibrosis induced by intratracheal instillation of LPS, indicating that SMIO can be used for the prevention and treatment of clinical pulmonary fibrosis.

### (4) Correlation between CYP2E1 activity and pulmonary fibrosis severity in mice after SMIO inhibition

Experimental method: A calcium precipitation method was used to prepare a mouse liver microsome, and a Braford method was used to determine a protein concentration in the microsome. An incubation system was prepared with 2 × PBS, a CZX solution, and the liver microsome at a final concentration of 0.5 mg/mL, and then pre-incubated at 37°C for 5 min; NADPH was added to initiate a reaction, and a resulting mixture was incubated at 37°C for 30 min and then placed on ice to terminate the reaction; and ethyl acetate was added to extract 6-hydroxychlorzoxazone, a resulting mixture was vortexed and centrifuged, and a resulting upper organic phase was collected and blow-dried with nitrogen. A peak area of a CZX metabolite 6-hydroxychlorzoxazone was detected by HPLC under the following conditions: methanol : water = 56:44, and detection wavelength: 287 nm. A concentration ΔC of the metabolite 6-hydroxychlorzoxazone was calculated through substitution into a standard curve; and a reaction rate of conversion of CZX into 6-hydroxychlorzoxazone was calculated according to V(pmol/min/mg) = (ΔC*1000)/(B*T) to evaluate an enzymatic activity of CYP2E1, where B represents a protein concentration of the microsome (mg/mL) and T represents an incubation time (min). A correlation between a CYP2E1 activity and a pulmonary fibrosis severity in mice was analyzed.

Experimental results: Compared with the control group, a reaction rate V of CZX metabolism in mice of the model group was significantly increased (*P* < 0.01); and compared with the model group, a reaction rate V of CZX metabolism in the SMIO high-dose group was significantly reduced (*P* < 0.05), indicating that SMIO can effectively inhibit the increase in enzymatic activity of CYP2E1 in the LPS-induced mouse pulmonary fibrosis model. A correlation between the reaction rate V of CZX and the lung index of pulmonary fibrosis was analyzed, and a correlation coefficient r of the two was 0.81, *P* < 0.01 (as shown in A to B of FIG. 23), indicating that the enzymatic activity of CYP2E1 has prominent correlation with the pulmonary fibrosis severity, and an antifibrosis effect of SMIO may be related to the inhibition on enzymatic activity of CYP2E1.

### (5) Inhibition of SMIO on an inflammatory microenvironment of pulmonary fibrosis in mice

### A. Inhibition of SMIO on an inflammatory response in lung injury mice

Experimental method: A neutrophil level in a mouse lung tissue was evaluated through immunohistochemistry (IHC) staining, and an immunostaining-positive area percentage was quantitatively evaluated by Image-Pro Plus software. RNA of a mouse lung tissue was extracted by a kit method, and expression levels of TNF-α and IL-1β in lung tissues of mice were determined by RT-PCR.

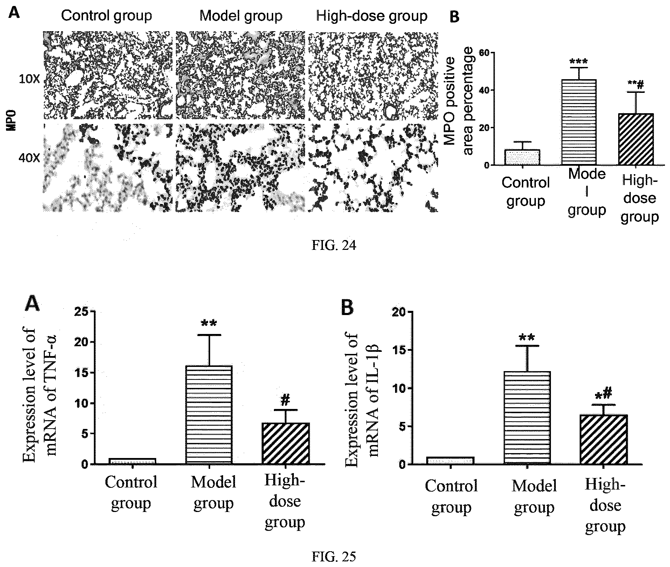

Experimental results: Compared with the control group, in the model group, the MPO immunostaining-positive area percentage in the lung tissue was significantly increased (*P* < 0.001) (as shown in A to B of FIG. 24), and expression levels of TNF-α and IL-1β in the lung tissue were significantly increased (*P* < 0.01) (as shown in A to B of FIG. 25); and compared with the model group, in the SMIO high-dose group, the MPO immunostaining-positive area percentage was significantly reduced (*P* < 0.05), and expression levels of TNF-α and IL-1β in the lung tissue were significantly reduced (*P* < 0.05), indicating that SMIO can alleviate the neutrophil infiltration and inhibit the release of proinflammatory cytokines in the lung tissue of mice with LPS-induced ALI.

### B. Inhibition of SMIO on an inflammatory microenvironment in pulmonary fibrosis mice

Experimental method: An ammonium molybdate method and a microplate method were used to treat a mouse lung tissue homogenate, and changes of oxidative stress indexes CAT and GSH in the lung tissue of mice in each group were detected. Expression levels of TGF-β1 and α-SMA in the mouse lung tissue were evaluated through immunohistochemical staining, and a positive area percentage was counted by Image-Pro Plus software. Expression levels of the epithelial cell marker E-cadherin, the apoptosis-associated protein Bax, and the anti-apoptotic protein Bcl-2 were detected by WB.

Experimental results: Compared with the control group, in the model group, the oxidative stress index CAT (*P <* 0.01) and epithelial cell marker E-cadherin (*P* < 0.05) levels were significantly reduced, the immunostaining-positive area percentages of α-SMA and TGF-β1 were significantly increased (*P* < 0.01), the expression level of the apoptotic factor Bax was significantly increased (*P* < 0.01), and the expression level of the anti-apoptotic factor Bcl-2 was significantly reduced (*P* < 0.05); and compared with the model group, in the SMIO intervention group, the oxidative stress index CAT (*P* < 0.01) and epithelial cell marker E-cadherin (*P* < 0.05) levels were significantly increased, the immunostaining-positive area percentages of α-SMA and TGF-β1 were significantly reduced (*P* < 0.01), the Bax level was significantly reduced (*P* < 0.01), and the Bcl-2 level was significantly increased (*P* < 0.001) (as shown in FIG. 26 to FIG. 27), indicating that the alleviation of LPS-induced pulmonary fibrosis in mice by SMIO may be related to the improvement of oxidation resistance, the anti-apoptotic effect, and the reduction in fibrosis-inducing factor TGF-β1 level and epithelial mesenchymal cell transformation in the lung tissue of pulmonary fibrosis mice. **staining**

### Example 15 Inhibition of SMIO on the lung cancer proliferation in mice

### (1) CYP2E1 changes in clinical lung cancer patients

Experimental method: The changes of CYP2E1 expression in paracancerous tissues of 30 clinical lung cancer patients were investigated with lung tissues of 30 healthy individuals as a control, and the changes of CYP2E1 contents in the paracancerous tissues of the lung cancer patients were determined.

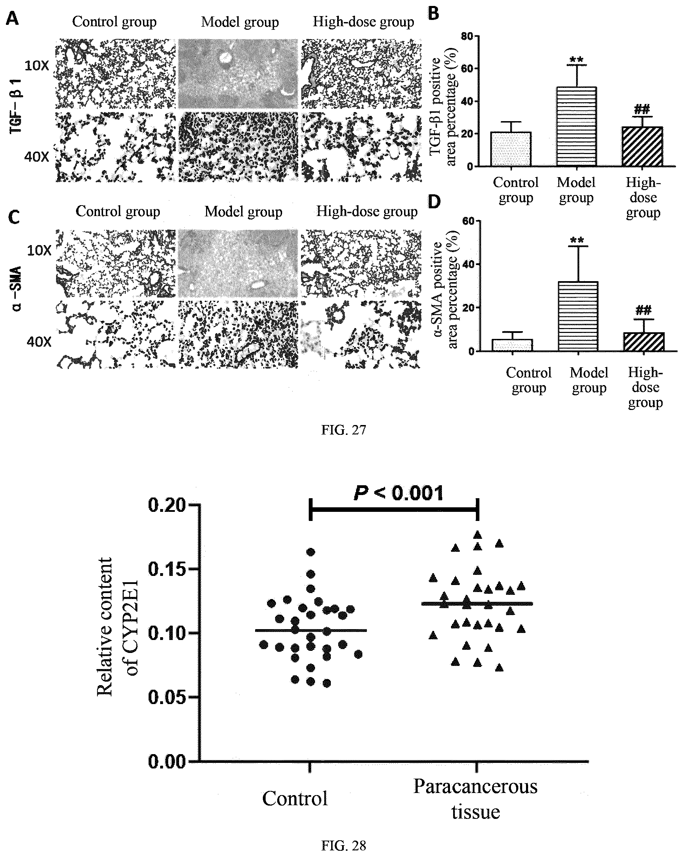

Experimental results: Immunohistochemical results showed that the expression of CYP2E1 in the paratumoral tissues of the lung cancer patients was significantly higher than that in the healthy lung tissue (as shown in FIG. 28, ****P* < 0.001 vs the healthy lung tissue group).

### (2) Changes of in situ transplanted tumors in lungs of CYP2E1 gene-knockout mice

Experimental method: A female C57 mouse lung cancer model with an *in situ* transplanted tumor was prepared with a lung cancer cell line Lewis at a cell amount of 2 * 10⁴. C57BL/6N mice were divided into a control group, a model group, and a CYP2E1 gene-knockout group, and the modeling took 18 days (control group, n = 8; model group, n = 8; and gene knockout group, n=8). At the end of the experiment, a body weight of mice in each group was recorded, blood was collected from the orbit, the mice were sacrificed, and lung tissues and tumor tissues were collected and weighed. Some lung specimens were subjected to HE staining, and then the lung lesion and tumorigenesis of mice in each group were observed.

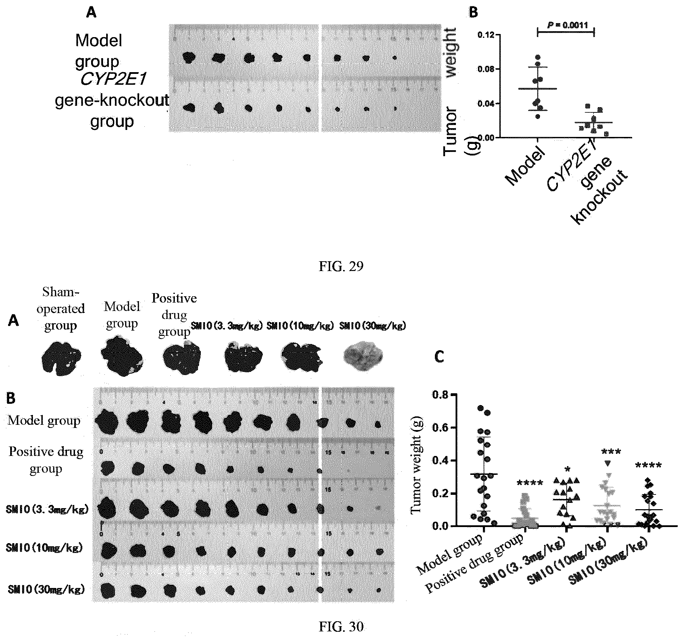

Experimental results: Compared with the model group, a weight of lung cancer in CYP2E1 gene-knockout mice was significantly reduced (as shown in A to B of FIG. 29), indicating that the CYP2E1 knockout can significantly inhibit the occurrence and development of lung cancer in the Lewis lung cancer model with the *in situ* transplanted tumor.

### (3) Intervention of SMIO in an in situ transplanted Lewis cell tumor in lungs of mice

Experimental method: A female C57 mouse lung cancer model with an *in situ* transplanted tumor was prepared with a lung cancer cell line Lewis at a cell amount of 2 * 10⁴. C57BL/6N mice were divided into a control group, a model group, a positive drug group, and an SMIO intervention group, and the modeling took 18 days (control group, n = 20; model group, n = 20; positive drug group (20 mg/kg), n = 15; SMIO (3.3 mg/kg), n = 15; SMIO (10 mg/kg), n = 20; and SMIO (30 mg/kg), n = 20). At the end of the experiment, a body weight of mice in each group was recorded, blood was collected from the orbit, the mice were sacrificed, and lung tissues and tumor tissues were collected and weighed. The weight and appearance of the lung (color, texture, or the like of the lung) were recorded. All tumors of an animal were cumulatively arranged together to indicate the tumorigenesis of the animal, and a cumulative weight of lung tumors in each animal was determined. Some lung specimens were subjected to HE staining, and then the lung lesion and tumorigenesis of mice in each group were observed.

Experimental results: Compared with the model group, the proliferation of lung tumors in mice of the SMIO intervention group was significantly inhibited, that is, the SMIO intervention could significantly inhibit the growth of lung tumors in mice, with a tumor proliferation inhibition rate as high as 68.3% (as shown in A to C of FIG. 30, **P <* 0.05 vs the model group, where A of FIG. 30 shows a representative picture of a tumor-bearing lung tissue of each mouse in each group; B of FIG. 30 shows a lung tumor tissue of each mouse; and C of FIG. 30 shows a tumor weight in each group), indicating that the SMIO administration can significantly inhibit the occurrence and development of lung cancer in the mouse Lewis lung cancer model with the *in situ* transplanted tumor.

### (4) Intervention of SMIO in a mouse CT26 cell lung metastasis

Experimental method: A female Balb/C mouse lung metastasis model was constructed through tail vein injection of a CRC cell CT26, with a cell amount of 3 * 10⁵. Mice were divided into a control group, a model group, and an SMIO intervention group, and the modeling took 14 days (control group, n = 5; model group, n = 5; and SMIO (30 mg/kg), n = 5). At the end of the experiment, a body weight of mice in each group was recorded, blood was collected from the orbit, the mice were sacrificed, and lung tissues were collected and weighed. The lung tissues were fixed in a *Brucella* staining solution for 24 h, and a number of lung nodules of mice in each group was recorded.

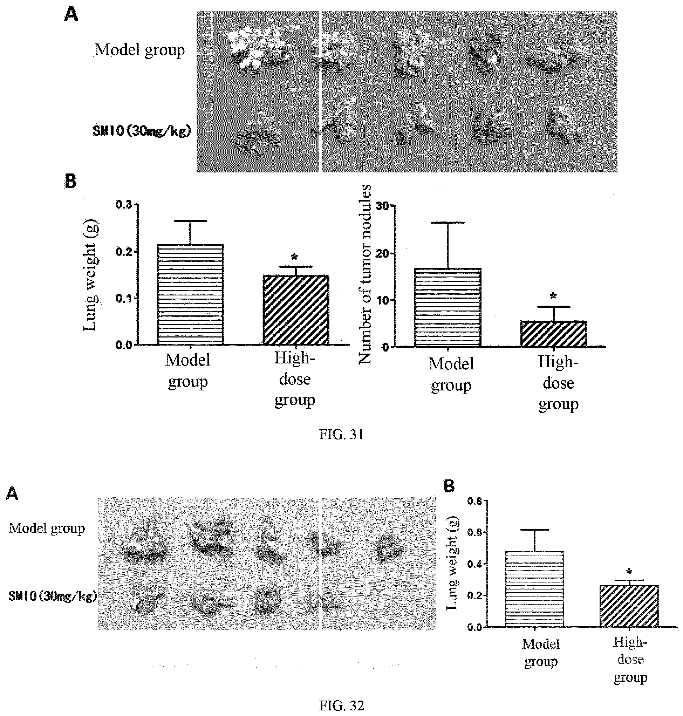

Experimental results: Compared with the model group, in the SMIO intervention group, the lung weight of mice was significantly reduced, the number of nodules was significantly reduced, and the tumor proliferation inhibition rate was as high as 66.7% (as shown in A to B of FIG. 31, **P* < 0.05 vs the model group, where A of FIG. 31 shows a representative picture of a tumor-bearing lung tissue of mice in each group and B of FIG. 31 shows the lung weight and the number of lung nodules of each mouse), indicating that the SMIO administration can significantly inhibit the occurrence and development of the lung metastasis in the lung metastasis model constructed through tail veil injection of the cell CT26.

### (5) Intervention of SMIO in a mouse B16-F10 cell lung metastasis

Experimental method: A female C57BL/6 mouse lung metastasis model was constructed through tail vein injection of a melanoma cell B16-F10, with a cell amount of 8 * 10⁵. Mice were divided into a control group, a model group, and an SMIO intervention group, and the modeling took 14 days (control group, n = 5; model group, n = 5; and SMIO (30 mg/kg), n = 4). At the end of the experiment, a body weight of mice in each group was recorded, blood was collected from the orbit, the mice were sacrificed, and lung tissues were collected and weighed. The lung tissues were fixed in a *Brucella* staining solution for 24 h, and a number of lung nodules of mice in each group was recorded.

Experimental results: Compared with the model group, in the SMIO intervention group, the lung weight of mice was significantly reduced, the number of nodules was significantly reduced, and the tumor proliferation inhibition rate was as high as 58.3% (as shown in A to B of FIG. 32, **P* < 0.05 vs the model group, where A of FIG. 32 shows a representative picture of a tumor-bearing lung tissue of mice in each group and B of FIG. 32 shows the lung weight of each mouse), indicating that the SMIO administration can significantly inhibit the occurrence and development of the lung metastasis in the mouse lung metastasis model constructed through tail veil injection of the cell B16-F10.

It can be seen from the above results that the CYP2E1 inhibitor SMIO has a significant prevention and treatment effect on the occurrence and development of the Lewis cell lung *in situ* transplanted tumor and the lung metastases constructed through tail veil injection of CT26 and B16-F 10, indicating that the compound SMIO can be used for the prevention and treatment of lung cancer.

### (6) Correlation between CYP2E1 activity and tumor severity in a lung cancer in situ model after SMIO inhibition

Experimental method: A calcium precipitation method was used to prepare a mouse liver microsome, and a Braford method was used to determine a protein concentration in the microsome. An incubation system was prepared with 2 × PBS, a CZX solution, and the liver microsome at a final concentration of 0.5 mg/mL, and then pre-incubated at 37°C for 5 min; NADPH was added to initiate a reaction, and a resulting mixture was incubated at 37°C for 30 min and then placed on ice to terminate the reaction; and ethyl acetate was added to extract 6-hydroxychlorzoxazone, a resulting mixture was vortexed and centrifuged, and a resulting upper organic phase was collected and blow-dried with nitrogen. A peak area of a CZX metabolite 6-hydroxychlorzoxazone was detected by HPLC under the following conditions: methanol : water = 56:44, and detection wavelength: 287 nm. A concentration ΔC of the metabolite 6-hydroxychlorzoxazone was calculated through substitution into a standard curve; and a reaction rate of conversion of CZX into 6-hydroxychlorzoxazone was calculated according to V(pmol/min/mg) = (ΔC*1000)/(B*T) to evaluate an enzymatic activity of CYP2E1, where B represents a protein concentration of the microsome (mg/mL) and T represents an incubation time (min). A correlation between CYP2E1 activity and lung tumor weight in the Lewis lung cancer *in situ* model was analyzed.

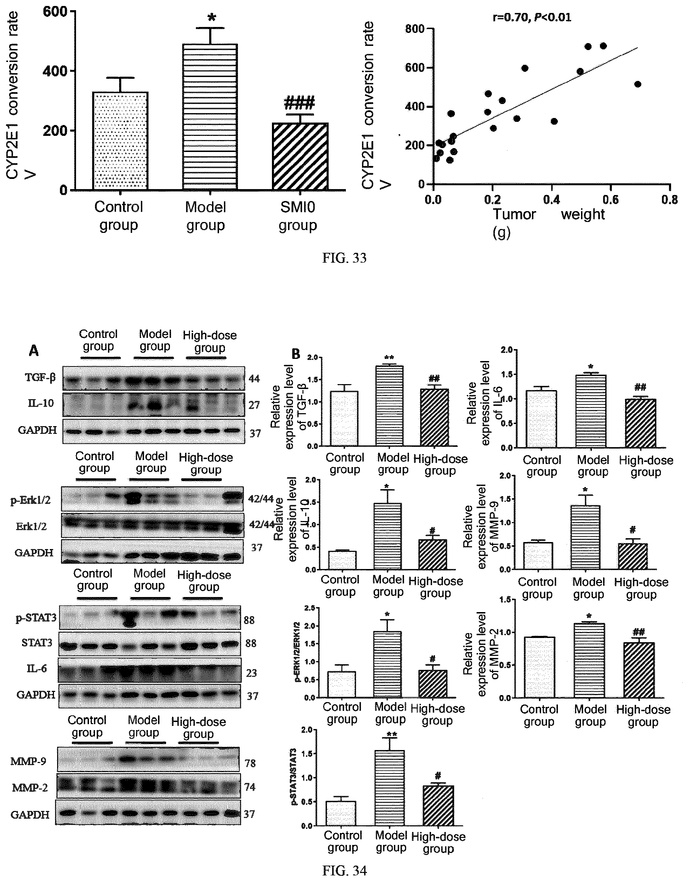

Experimental results: Compared with the sham-operated group, the CYP2E1 activity in the model group was significantly increased (*P*<0.05); and compared with the model group, the CYP2E1 activity in the SMIO intervention group was significantly reduced (*P<* 0.05), indicating that SMIO can effectively inhibit the increase in enzymatic activity of CYP2E1 in the liver tissue of the mouse Lewis lung cancer *in situ* model, and the CYP2E1 activity of mice is significantly positively correlated with the tumor weight of the mouse Lewis lung cancer *in situ* model (r = 0.70, *P <* 0.01) (as shown in FIG. 33), indicating that the enzymatic activity of CYP2E1 is positively correlated with the severity of Lewis lung cancer *in situ,* and the anti-lung cancer effect of SMIO may be related to the inhibition on enzymatic activity of CYP2E1.

### (7) Inhibition of SMIO on an inflammatory microenvironment of a mouse lung in situ transplanted tumor

Experimental method: A WB method was used to detect the expression of proinflammatory cytokines TGF-β, IL-10, and IL-4, inflammation-associated signaling pathway proteins IL-6/p-STAT3 and p-ERK1/2/ERK1/2, and epithelial-mesenchymal transition (EMT)-associated proteins MMP-2 and MMP-9 in the mouse paracancerous lung tissue of each group and detect the expression of anti-apoptosis-associated proteins caspase3 and Bcl-2 and an autophagy-associated protein p53 in the tumor tissue.

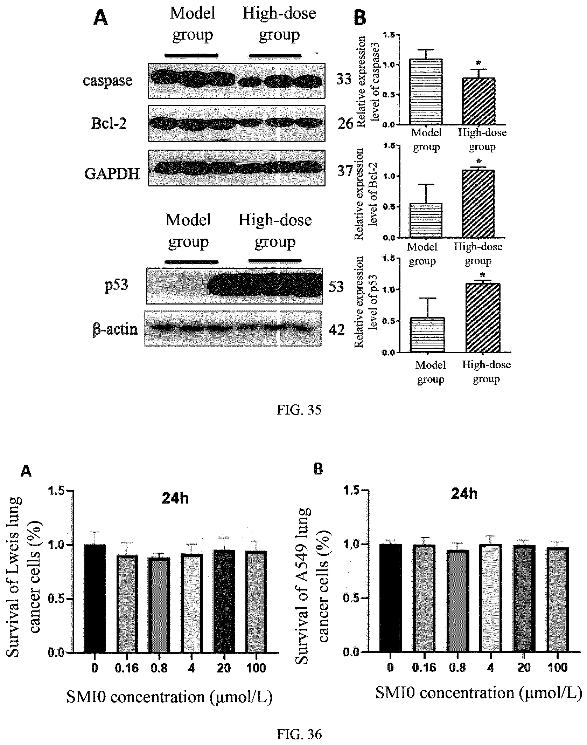

Experimental results: Compared with the sham-operated group, the expression of proinflammatory cytokines (TGF-β and IL-10), inflammation-associated signaling pathway proteins (IL-6/p-STAT3 and p-ERK1/2/ERK1/2), and matrix metalloproteinase (MMP) (MMP-2 and MMP-9) was significantly increased in the paratumoral tissue of the model group (*P* < 0.05); further, compared with the model group, the expression of the above proteins was significantly reduced in the SMIO intervention group (*P* < 0.05) (as shown in A to B of FIG. 34); and similarly, compared with the model group, in the SMIO (30 mg/kg) intervention group, the expression of anti-apoptotic proteins caspase3 and Bcl-2 in the tumor tissue was significantly reduced (*P* < 0.05), and the expression of the autophagy-associated protein p53 was significantly increased (*P*< 0.01) (as shown in A to B of FIG. 35), indicating that SMIO can significantly inhibit the activation of inflammation and related signaling pathways and the expression of MMPs in the paratumoral lung tissue of the Lewis lung *in situ* transplanted tumor model and enhance the p53-mediated autophagy in the tumor tissue.

### (8) Inhibition of SMIO on M2 polarization of macrophages in a paratumoral microenvironment

A. SMIO exhibited no direct inhibitory effect on lung cancer cells.

Experimental method: Lewis lung cancer cells and A549 lung cancer cells in a logarithmic growth phase each were selected and inoculated into a 96-well plate at a concentration of 1 * 10⁵ cells/mL, then 100 µL of a basic medium including SMIO at different concentrations (0 µmol/L, 0.16 µmol/L, 0.8 µmol/L, 4 µmol/L, 20 µmol/L, and 100 µmol/L) was added to each well, and 24 h later, 10 µL of a CCK8 reagent was added to each well; and the cells were further cultivated for 2 h, and an absorbance OD value of each well was determined at 450 nm by a microplate reader. 3 replicates were set for each well, and an average was taken. The proliferation activity was calculated based on the OD value.

Experimental results: SMIO at a test concentration (up to 100 µmol/L) exhibited no significant inhibitory effect on the proliferation of Lewis lung cancer cells and A549 lung cancer cells (as shown in A to B of FIG. 36).

B. SMIO inhibited the proliferation of lung cancer cells by inhibiting the M2 polarization of macrophages.

Experimental method: PMA was used at 100 µmol/L to induce the transformation of human monocytic leukemia cells THP-1 into M0 macrophages, and then IL-4 and IL-13 were used at 20 ng/mL to induce the transformation of M0 macrophages into M2 macrophages. A supernatant was collected to establish a co-cultivation system with an A549 lung cancer cell, thereby simulating a M2-type macrophage microenvironment of lung cancer. In the SMIO intervention group, SMIO (50 µmol/L) was added for intervention while interleukin was added for induction. Culture supernatants of the M0 macrophages, M2 macrophages, and SMIO intervention group were collected to prepare conditional media. Lewis lung cancer cells in a logarithmic growth phase were selected and inoculated into a 96-well plate at a concentration of 1 * 10⁵ cells/mL, then 100 µL of each of different conditional media was added to each well, and 24 h later, 10 µL of a CCK8 reagent was added to each well; and the cells were further cultivated for 2 h, and an absorbance OD value of each well was determined at 450 nm by a microplate reader. 3 replicates were set for each well, and an average was taken. The proliferation activity was calculated based on the OD value.

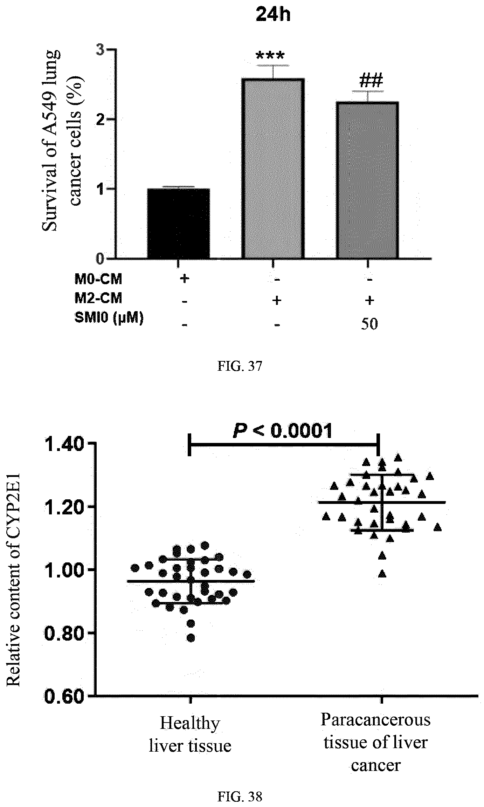

Experimental results: Compared with the group in which A549 lung cancer cells were cultivated alone, the proliferation activity of A549 cells in the M2 macrophage co-cultivation group was significantly enhanced (P *<* 0.01); and compared with the M2 macrophage co-cultivation group, the proliferation activity of A549 cells in the SMIO intervention group (50 µmol/L) was significantly weakened (*P* < 0.05) (as shown in FIG. 37), indicating that the inhibition of the CYP2E1 inhibitor SMIO on the Lewis lung *in situ* transplanted tumor may be related to the inhibition on the paratumoral inflammatory microenvironment of lung cancer, especially to the M2 polarization of macrophages in the paratumoral microenvironment.

### Example 16 Inhibition of SMIO on the occurrence and development of liver cancer in mice

### (1) CYP2E1 changes in clinical liver cancer patients

Experimental method: The changes of CYP2E1 expression in paracancerous tissues of 35 clinical liver cancer patients were investigated with liver tissues of 35 healthy individuals as a control, and the changes of CYP2E1 contents in the paracancerous tissues of the liver cancer patients were determined.

Experimental results: Immunohistochemical results showed that the expression of CYP2E1 in the paratumoral tissues of the liver cancer patients was significantly higher than that in the healthy liver tissue (as shown in FIG. 38, ****P* < 0.0001 vs the healthy liver tissue group).

### (2) Inhibition of CYP2E1 gene knockout on a rat liver in situ transplanted tumor

Experimental method: An SD rat liver *in situ* transplanted tumor model was constructed through *in situ* implantation of a breast sarcoma cell line Walker256 into the liver, with a cell concentration of 4 * 10⁶. SD rats were divided into a model group and a CYP2E1 gene-knockout group, and the modeling took 21 days (model group, n = 9 rats; and gene-knockout group, n = 8 rats). At the end of the experiment, blood was collected from the orbit, a body weight was measured, and then the rats each were sacrificed. The weight and appearance of a liver (color and texture of the liver) were recorded. Some liver specimens were subjected to HE staining, and then the liver lesion and tumorigenesis of rats in each group were observed.

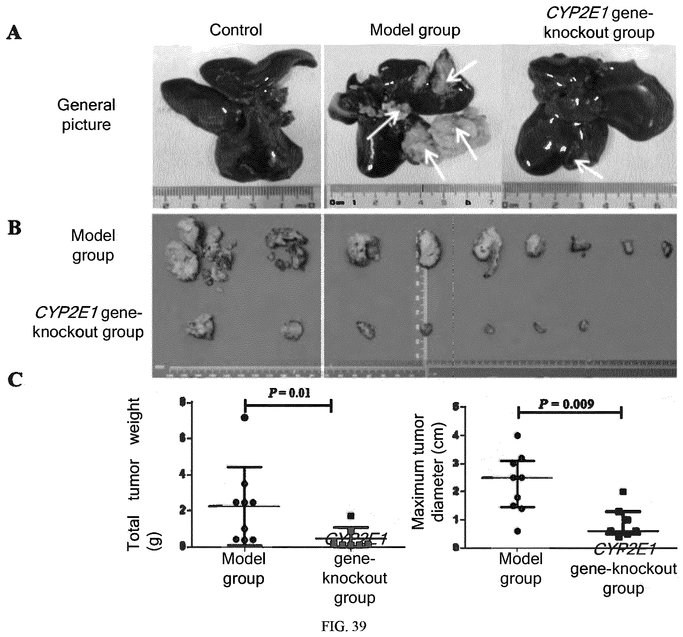

Experimental results: Compared with the model group, the liver tumor growth was significantly inhibited in rats of the CYP2E1 gene-knockout group, with a tumor proliferation inhibition rate as high as 82.0% (as shown in FIG. 39), indicating that the CYP2E1 gene knockout can significantly inhibit the occurrence of a tumor in the rat liver transplanted tumor model constructed through *in situ* implantation of the Walker256 cell.

### (3) Intervention of SMIO in a mouse liver in situ transplanted tumor

Experimental method: A male BALB/c mouse liver transplanted tumor model was constructed through *in situ* implantation of a liver cancer cell line H22 into the liver, with a cell concentration of 1.5 * 10⁵. An SMIO intervention group was divided into a low-dose group, a medium-dose group, and a high-dose group, which were intragastrically administered with the compound SMIO respectively at 3.3 mg/kg, 10 mg/kg, and 30 mg/kg every day from two days before modeling to the end of the 24-day modeling (model group, n = 19 mice; SMIO compound low-dose group, n = 19 mice; SMIO compound medium-dose group, n = 18 mice; and SMIO compound high-dose group, n = 29 mice). At the end of the experiment, blood was collected from the orbit, a body weight was measured, and then the mice each were sacrificed. The weight and appearance of a liver (color and texture of the liver) were recorded. Some liver specimens were subjected to HE staining, and then the liver lesion and tumorigenesis of mice in each group were observed.

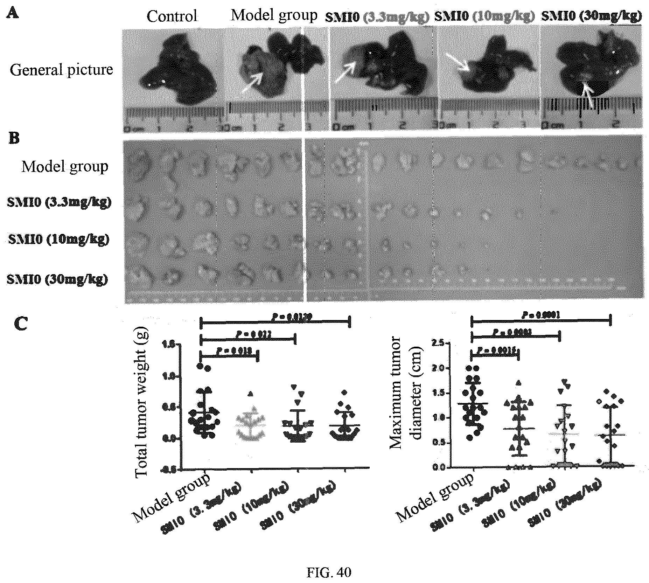

Experimental results: Compared with the model group, the liver H22 tumor incidences in mice of the low-dose, medium-dose, and high-dose SMIO intervention groups were significantly reduced from 100% in the model group respectively to 78.9%, 72.2%, and 68.4% (as shown in A to C of FIG. 40, **P* < 0.05 and ***P* < 0.01 vs the model group). FIG. 40 shows pictures of the liver tumor tissues of mice. If multiple tumors are formed on the liver of a mouse, all of the tumors are cumulatively arranged together to indicate the tumorigenesis of the mouse. A cumulative weight of liver tumors in each animal was measured. It was found that there was tumorigenesis in the liver of each mouse in the model group, and compared with the model group, the tumor proliferation was significantly inhibited in the SMIO intervention group, that is, the SMIO intervention significantly inhibited the growth of liver tumors in mice, with a tumor proliferation inhibition rate as high as 71.8%, indicating that the SMIO administration can significantly inhibit the occurrence and development of a tumor in the liver cancer transplanted tumor model constructed through *in situ* implantation of the H22 cell.

### (4) Inhibition of SMIO on an inflammatory microenvironment of a mouse liver in situ transplanted tumor

Experimental method: The levels of oxidative stress indexes MDA and T-AOC in the mouse paracancerous liver tissue were determined by a kit method; the expression of the chemokine CXCL-12 in the mouse paracancerous liver tissue was detected by RT-PCR; the expression of proinflammatory cytokines (TNF-α, IL-6, and IL-1β) and inflammation-associated signaling pathway proteins (ARRB1, Src, ITGAV, p-STAT3/STAT3, p-ERK/ERK, and p-P38MAPK/P38MAPK) in the mouse paracancerous liver tissue was detected by a WB method; and the expression of apoptosis-associated proteins and signaling pathways casPase3, cleaved casPase3, p-ERK/ERK, and p-p38MAPK/p38MAPK in the tumor tissue was detected by a WB method.

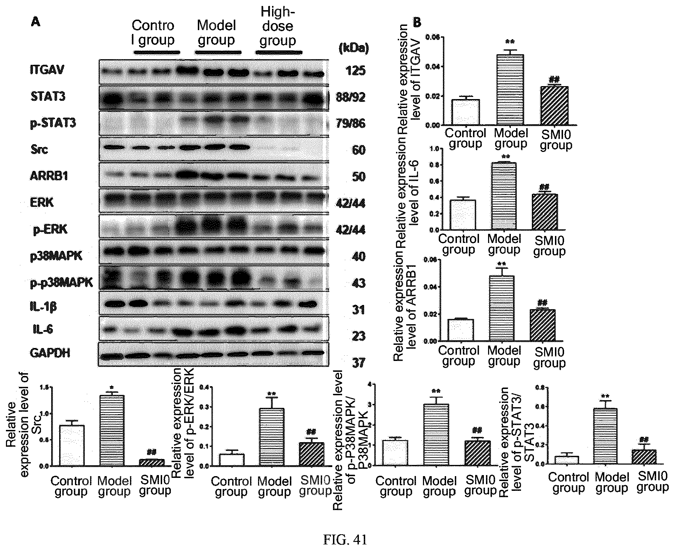

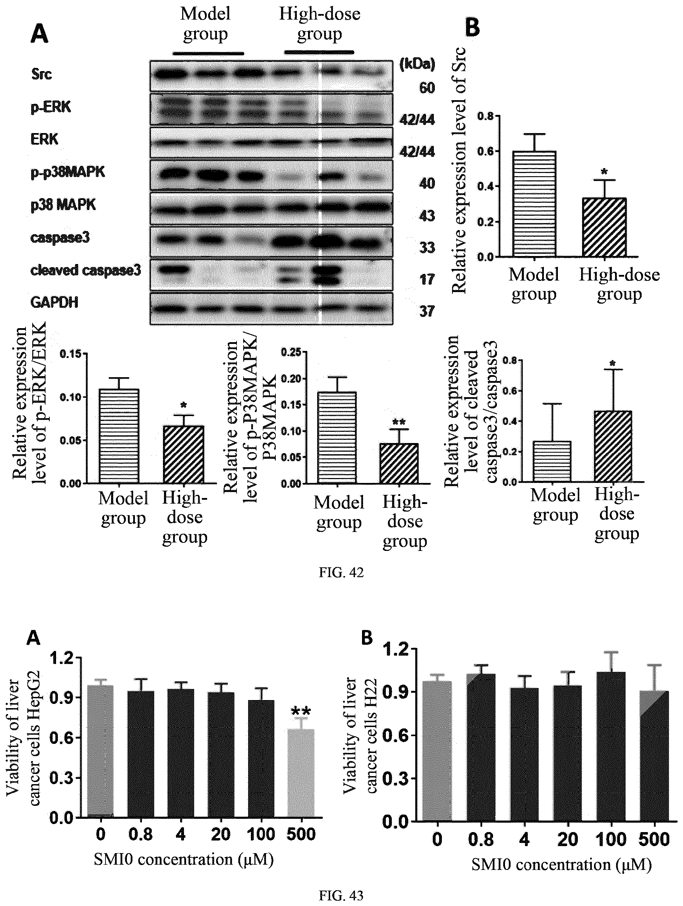

Experimental results: Compared with the sham-operated group, in the model group, the expression of an oxidative stress index MDA, a chemokine CXCL-12, proinflammatory cytokines (TNF-α, IL-6, and IL-1β), and inflammation-associated signaling pathway proteins (ARRB1, Src, ITGAV, p-STAT3/STAT3, p-ERK/ERK, and p-p38MAPK/p38MAPK) in the paratumoral liver tissue of mice was significantly up-regulated (*P* < 0.05 or *P* < 0.01), and the expression of a total oxidation resistance index T-AOC was significantly decreased (*P* < 0.01); further, compared with the model group, in the SMIO intervention group, the expression of the total oxidation resistance index T-AOC was significantly increased (*P* < 0.01), and the expression of other proteins was significantly reduced (*P* < 0.05 or *P* < 0.01) (as shown in A to B of FIG. 41); and similarly, compared with the model group, in the SMIO (30 mg/kg) intervention group, the expression of apoptosis-associated proteins cleaved caspase3/caspase3 in the tumor tissue was significantly up-regulated (*P* < 0.01), and the expression of p-ERK/ERK and p-p38MAPK/p38MAPK was significantly down-regulated (*P* < 0.05 or *P* < 0.01) (as shown in A to B of FIG. 42), indicating that SMIO can significantly inhibit the activation of oxidative stress and inflammation-associated signaling pathways in the paracancerous liver tissue of the mouse H22 liver *in situ* transplanted tumor and promote the apoptosis of the tumor.

### (5) SMIO played an anti-liver cancer role by affecting macrophages.

A. SMIO exhibited no direct inhibitory effect on liver cancer cells.

Experimental method: HepG2 liver cancer cells and H22 liver cancer cells in a logarithmic growth phase each were selected and inoculated into a 96-well plate at a concentration of 1 * 10⁵ cells/mL, and then 100 µL of a basic medium including SMIO at different concentrations (0 µmol/L, 0.16 µmol/L, 0.8 µmol/L, 4 µmol/L, 20 µmol/L, and 100 µmol/L) was added to each well; and 24 h later, the proliferation activity of HepG2 was determined by a CCK8 method, a migration ability of HepG2 was determined by wound-healing assay, and an apoptosis ability of HepG2 was determined by flow cytometry (FCM).

Experimental results: SMIO at a test concentration (up to 100 µmol/L) exhibited no significant inhibitory effect on the proliferation, migration, and apoptosis of HepG2 and H22 liver cancer cells (as shown in A to B of FIG. 43), indicating that the CYP2E1 inhibitor SMIO has no direct inhibitory effect on liver cancer cells.

B. SMIO played an anti-liver cancer role by affecting macrophages.

Experimental method: A supernatant of a murine macrophage RAW264.7 was collected to establish a co-cultivation system with the HepG2 liver cancer cell, thereby simulating a macrophage inflammatory microenvironment of liver cancer. In the SMIO intervention groups, SMIO was added at different concentrations (0 µmol/L, 0.16 µmol/L, 0.8 µmol/L, 4 µmol/L, 20 µmol/L, and 100 µmol/L) while interleukin was added for induction. Culture supernatants of the SMIO intervention groups were collected to prepare conditional media. HepG2 liver cancer cells in a logarithmic growth phase were adopted. The proliferation activity of HepG2 was determined by a CCK8 method, a migration ability of HepG2 was determined by wound-healing assay, and an apoptosis ability of HepG2 was determined by FCM.

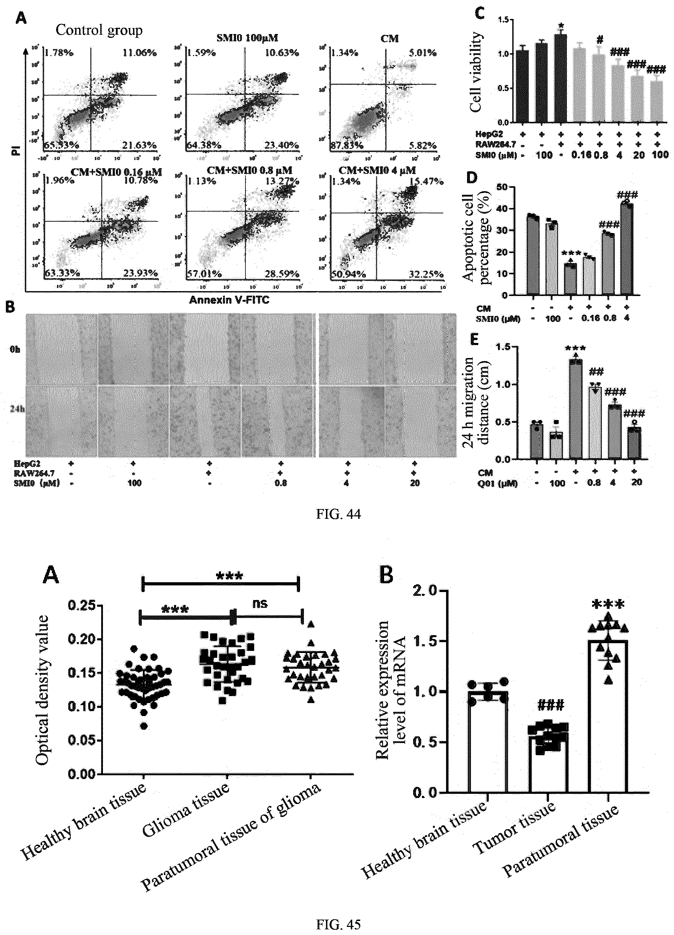

Experimental results: Compared with the group in which HepG2 liver cancer cells were cultivated alone, in the RAW264.7 macrophage co-cultivation group, the proliferation activity (*P* < 0.05) and migration ability (*P* < 0.001) of the HepG2 cells were significantly enhanced, and the apoptosis ability of the HepG2 cells was significantly reduced (*P*< 0.001); and compared with the macrophage co-cultivation group, in the SMIO intervention groups (especially 4 µmol/L), the proliferation activity (*P* < 0.001) and migration ability (*P* < 0.001) of the HepG2 liver cancer cells were significantly weakened, and the apoptosis ability of the HepG2 liver cancer cells was significantly enhanced (*P* < 0.001) (as shown in FIG. 44), indicating that the inhibition of the CYP2E1 inhibitor SMIO on liver cancer may be achieved by affecting macrophages in a paratumoral microenvironment to inhibit the proliferation and migration of HepG2 liver cancer cells and promote the apoptosis of HepG2 liver cancer cells.

It can be seen from the above results that SMIO has a significant prevention and treatment effect on the occurrence and development of a tumor in a mouse liver cancer model constructed through *in situ* implantation of H22 cells, indicating that the compound SMIO can be used for the prevention and treatment of clinical liver cancer.

### Example 17 Inhibition of SMIO on the glioma proliferation in mice

### (1) CYP2E1 changes in clinical glioma patients

Experimental method: The changes of CYP2E1 expression in paracancerous tissues of 32 clinical glioma patients were investigated with brain tissues of 46 healthy individuals as a control, and the changes of CYP2E1 protein contents in the paracancerous tissues of the glioma patients were determined. CYP2E1 mRNA levels in paratumoral brain tissues of 12 glioma patients were investigated with brain tissues of 6 healthy individuals as a control, and the CYP2E1 mRNA level changes in the paratumoral brain tissues were determined.

Experimental results: Immunohistochemical results showed that the expression of CYP2E1 in the paratumoral tissues of the glioma patients was significantly higher than that in the healthy brain tissues (as shown in A of FIG. 45, ****P* < 0.001 vs the healthy brain tissue group); and qPCR results showed that the expression of CYP2E1 mRNA in the paratumoral tissues of the glioma patients was significantly higher than that in the healthy brain tissues (as shown in B of FIG. 45, ****P* < 0.001 vs the healthy brain tissue group).

### (2) Inhibition of CYP2E1 knockout on a mouse brain in situ transplanted tumor

Experimental method: A female C57/BL6 mouse brain glioma *in situ* model was constructed with a mouse brain glioma cell line GL261. Molding method: The skull was perforated at a position 0.15 mm posterior to and 2 mm at the right of the anterior fontanelle, 5 µL of a GL261 single-cell suspension (1 × 10⁶ cells in total) was drawn with a microsyringe, and then a needle of the microsyringe was slowly vertically inserted along a needle hole with an insertion depth of 4 mm and a retracting depth of 1 mm; and this step was repeated 10 times, and tumor-bearing cells were then slowly injected at a speed of 1 µL/min. A model group and a *CYP2E1* gene-knockout group were adopted for the experiment, and the modeling took about 21 days (model group, n = 10 mice; and CYP2E1 gene-knockout group, n = 6 mice). At the end of the experiment, a body weight was measured, blood was collected from the orbit, then the mice each were sacrificed, and brain tissues were collected. Brain tissue specimens were subjected to HE staining, and then the tumorigenesis and tumor size of mice in each group were observed.

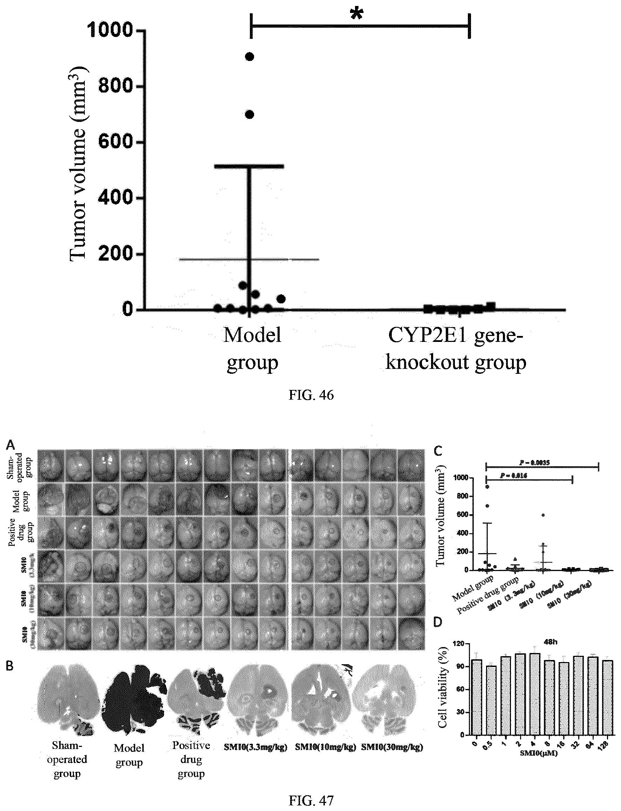

Experimental results: Compared with the model group, the *CYP2E1* gene knockout significantly inhibited the glioma growth in mice (as shown in FIG. 46, **P* < 0.05 vs the wild-type (WT) model group), indicating that the CYP2E1 gene knockout can significantly inhibit the occurrence of a tumor in the GL261 cell brain *in situ* transplanted mouse glioma model.

### (3) Intervention of SMIO in a mouse brain in situ transplanted tumor

Experimental method: A female C57/BL6 mouse glioma model was constructed through *in situ* implantation of a glioma cell line GL261, with a cell concentration of 1 * 10⁶. Experimental groups: sham-operated group (n = 9); model group (n = 10); temozolomide group (50 mg/kg, ig, n = 11); SMIO low-dose group (3.3 mg/kg, ig, n = 13); SMIO medium-dose group (10 mg/kg, ig, n = 13); and SMIO high-dose group (30 mg/kg, ig, n = 13). The SMIO intervention group was intragastrically administered with the compound SMIO every day starting from three days before modeling, and the positive drug group was administered on day 3 to day 7 and day 10 to day 14 after surgery. The modeling took about 21 days. At the end of the experiment, blood was collected from the orbit, a body weight was measured, then the mice each were sacrificed, and brain tissues were collected. Brain tissue specimens were subjected to HE staining, and then the brain tissue lesion and tumorigenesis of mice in each group were observed.

Experimental results: Compared with the model group, the SMIO intervention significantly inhibited the glioma growth in mice, where the SMIO high-dose group (30 mg/kg) had the optimal inhibitory effect, with a tumor proliferation inhibition rate as high as 97.6% (as shown in A to C of FIG. 47, **P* < 0.05 vs the model group and ***P* < 0.01 vs the model group), indicating that SMIO can significantly inhibit the occurrence and development of a tumor in the GL261 cell brain *in situ* transplanted mouse glioma model.

In addition, an *in vitro* inhibition experiment (D of FIG. 47) showed that SMIO in a concentration range of 0 µM to 128 µM continuously acted on GL261 cells for 48 h, and exhibited no significant inhibitory effect on GL261.

It can be seen from the above results that the CYP2E1 gene knockout can significantly inhibit the occurrence and development of glioma in mice, and the CYP2E1 inhibitor SMIO has a significant prevention and treatment effect on the occurrence and development of glioma in GL261 cell brain *in situ* implanted mice, indicating that the compound SMIO can be used for the prevention and treatment of clinical glioma.

### (4) Correlation between CYP2E1 activity and tumor severity in a glioma model after SMIO inhibition

Experimental method: A calcium precipitation method was used to prepare a mouse liver microsome, and a Braford method was used to determine a protein concentration in the microsome. An incubation system was prepared with 2 × PBS, a CZX solution, and the liver microsome at a final concentration of 0.5 mg/mL, and then pre-incubated at 37°C for 5 min; NADPH was added to initiate a reaction, and a resulting mixture was incubated at 37°C for 30 min and then placed on ice to terminate the reaction; and ethyl acetate was added to extract 6-hydroxychlorzoxazone, a resulting mixture was vortexed and centrifuged, and a resulting upper organic phase was collected and blow-dried with nitrogen. A peak area of a CZX metabolite 6-hydroxychlorzoxazone was detected by HPLC under the following conditions: methanol : water = 56:44, and detection wavelength: 287 nm. A concentration ΔC of the metabolite 6-hydroxychlorzoxazone was calculated through substitution into a standard curve; and a reaction rate of conversion of CZX into 6-hydroxychlorzoxazone was calculated according to V(pmol/min/mg) = (ΔC*1000)/(B*T) to evaluate an enzymatic activity of CYP2E1, where B represents a protein concentration of the microsome (mg/mL) and T represents an incubation time (min). A correlation between CYP2E1 activity and tumor size in the mouse glioma model was analyzed.

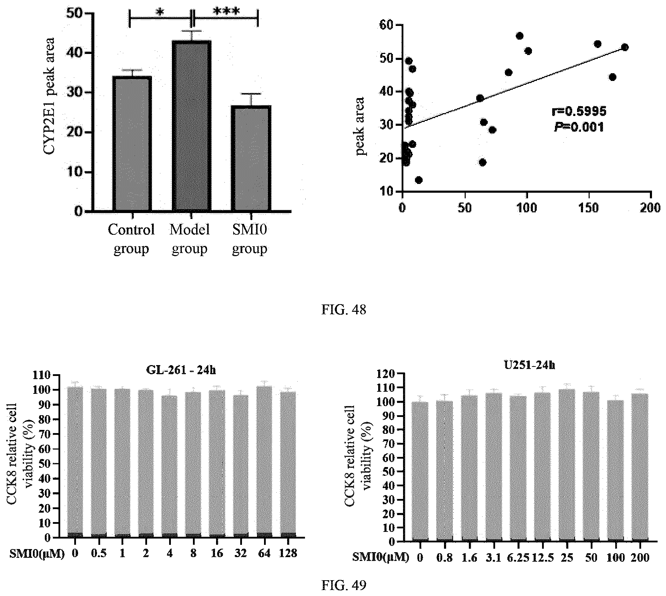

Experimental results: Compared with the sham-operated group, the CYP2E1 activity in the model group was significantly increased (*P* < 0.05); and compared with the model group, the CYP2E1 activity in the SMIO intervention group was significantly reduced (P < 0.001), indicating that SMIO can effectively inhibit the increase in enzymatic activity of CYP2E1 in the liver tissue of the *in situ* transplanted mouse glioma model, and the CYP2E1 activity of mice is significantly positively correlated with the tumor weight of the *in situ* transplanted mouse glioma model (r = 0.5995, *P* < 0.001) (as shown in FIG. 48). It can be known that the enzymatic activity of CYP2E1 is related to glioma, and an anti-glioma effect of SMIO may be related to the inhibition on enzymatic activity of CYP2E1.

### (5) SMIO inhibited the growth of GL261 glioma through a paratumoral microenvironment.

A. SMIO exhibited no direct inhibitory effect on glioma cells.

Experimental method: GL261 murine glioma cells and U251 human glioma cells in a logarithmic growth phase each were selected and inoculated into a 96-well plate at a concentration of 5 * 10⁵ cells/mL, then 100 µL of a cell suspension was added to each well, and 12 h later, a medium was discarded; 200 µL of a basic medium including SMIO at different concentrations (0 µmol/L, 0.16 µmol/L, 0.8 µmol/L, 4 µmol/L, 20 µmol/L, and 100 µmol/L) was added to each well, and 24 h later, 10 µL of a CCK8 reagent was added to each well; and the cells were further cultivated for 2 h, and an absorbance OD value of each well was determined at 450 nm by a microplate reader. 3 replicates were set for each well, and an average was taken. The proliferation activity was calculated based on the OD value.

Experimental results: SMIO at a test concentration (up to 128 µmol/L or 200 µmol/L) exhibited no significant inhibitory effect on the proliferation of GL261 and U251 glioma cells (as shown in FIG. 49).

B. SMIO resisted glioma through paratumoral microglial cells and astrocytes.

Experimental method: 10 1-2 d newborn C57BL/6 WT mice and 6 *CYP2E1*^{*-*/*-*} mice were taken, primary microglial cells and astrocytes were collected and inoculated into 6-well plates, and 24 h later, culture supernatants were taken and co-cultivated with glioma cells. Experimental groups: control group; M2 model group; high-concentration, medium-concentration, and low-concentration SMIO (50 µmol/L, 12.5 µmol/L, and 3.1 µmol/L) groups; and *CYP2E1* gene-knockout group. 24 h later, supernatants were discarded; and IL-4 and IL-13 each were added at 20 ng/mL to induce the transformation of microglial cells into the M2 type in the M2 model group and the high-concentration, medium-concentration, and low-concentration SMIO groups, and different concentrations of SMIO (50 µmol/L, 12.5 µmol/L, and 3.1 µmol/L) were also added in the intervention groups. The proliferation of glioma cells was determined by a CCK8 method, and an apoptosis proportion was determined by an Annexin V-FITC-PI apoptosis staining kit.

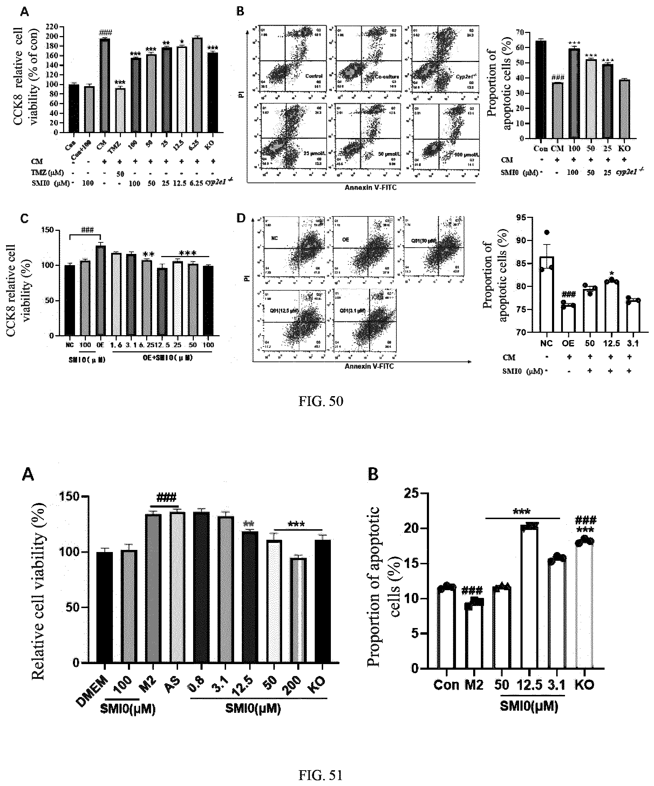

Experimental results: Compared with the group in which GL261/U251 glioma cells were cultivated alone, in the M2 microglial cell co-cultivation group, the proliferation activity of GL261/U251 cells was significantly enhanced, and the apoptosis was significantly inhibited; and compared with the M2 microglial cell co-cultivation group, in the SMIO intervention group (50 µmol/L), the proliferation of GL261/U251 cells was significantly inhibited (P < 0.001), and the apoptosis of GL261 cells was promoted (as shown in FIG. 50), indicating that the inhibition of the CYP2E1 inhibitor SMIO on GL261 and U251 *in situ* transplanted tumors may be related to microglial cells.

Compared with the group in which GL261 glioma cells were cultivated alone, in the M2 astrocyte co-cultivation group, the proliferation activity of GL261 cells was significantly enhanced (P < 0.01), and the apoptosis was significantly inhibited (P < 0.01); and compared with the M2 astrocyte co-cultivation group, in the SMIO intervention group (50 µmol/L), the proliferation activity of GL261 cells was significantly weakened (*P* < 0.05) (as shown in FIG. 51, *P<* 0.01), indicating that the inhibition of the CYP2E1 inhibitor SMIO on GL261 *in situ* transplanted tumors may be related to astrocytes.

C. The inhibition of SMIO on glioma growth was related to the inhibition on M2 polarization of microglial cells and the cholesterol metabolism of astrocytes.

Experimental method: Astrocytes in the co-cultivation group were collected, RNA was extracted and reverse-transcribed into cDNA, and the changes of inflammatory factor and cholesterol metabolism-associated genes were detected by qPCR.

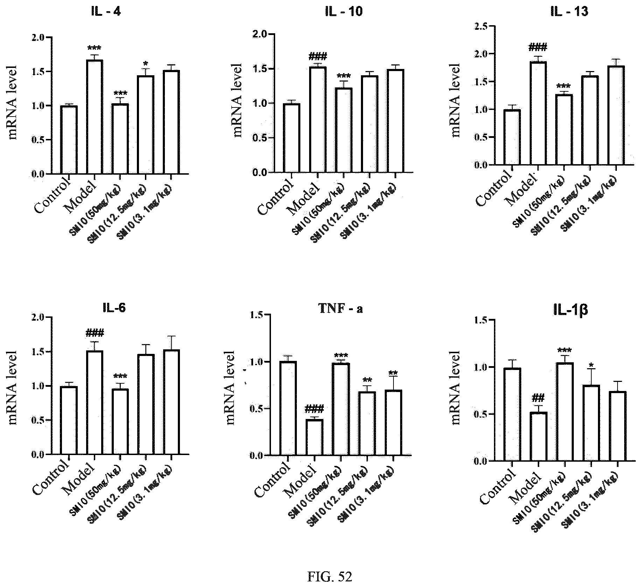

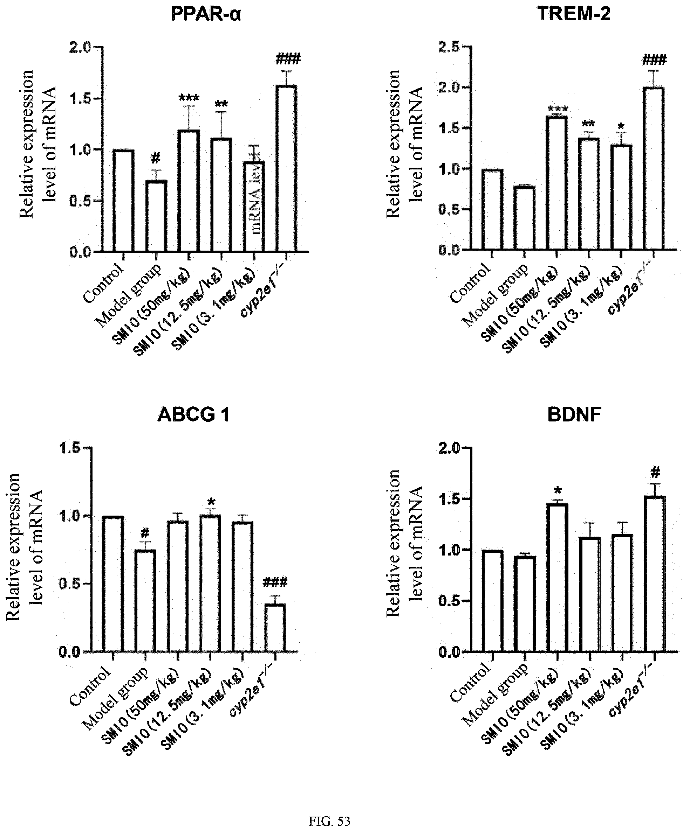

Experimental results: Compared with the group in which GL261 glioma cells were cultivated alone, in M2 microglial cells, the levels of IL-4, IL-10, and IL-13 were significantly increased, and the level of the lipid metabolism-associated gene PPAR-α was significantly reduced; and compared with the M2 microglial cell co-cultivation group, in the SMIO intervention group (50 µmol/L), the levels of IL-4, IL-10, and IL-13 were significantly reduced, and the level of the lipid metabolism-associated gene PPAR-α was significantly increased (as shown in FIG. 52 and FIG. 53), indicating that the inhibition of the CYP2E1 inhibitor SMIO on GL261 *in situ* transplanted tumors may be related to the inhibition of M2 polarization of microglial cells and the cholesterol metabolism of astrocytes.

### Example 18 Inhibition of SMIO on the occurrence and development of ovarian cancer in mice

### (1) Ovarian cancer in situ transplanted tumor

Experimental method: A female C57/6 mouse ovarian cancer *in situ* transplanted tumor model was constructed with a murine ovarian cancer ID-8 cell line at a cell concentration of 1 × 10⁶. A model group and an SMIO intervention group were set for the experiment. The intervention group was intragastrically administered with the SMIO compound at 30 mg/kg every day from three days before modeling to the end of the 60-day modeling (model group, n = 15; and SMIO compound (30 mg/kg) group, n = 12). At the end of the experiment, blood was collected from the orbit, a body weight was measured, then the mice each were sacrificed, and ovary tissues and tumor tissues were collected. Ovary tissue specimens were subjected to HE staining, and then the ovary tumor tissue and tumorigenesis of mice in each group were observed.

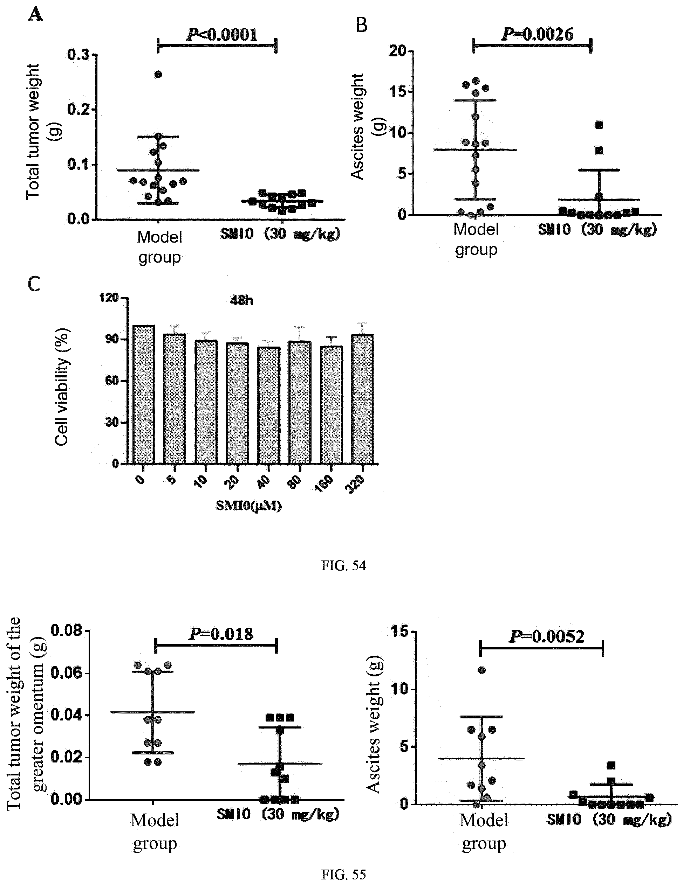

Experimental results: Compared with the model group, the SMIO intervention significantly inhibited the growth of ovarian cancer in mice, with a tumor proliferation inhibition rate as high as 63.14% and an ascites weight inhibition rate as high as 76.40% (as shown in A of FIG. 54), indicating that the SMIO administration can significantly inhibit the occurrence and development of a tumor in the ID-8 ovarian cancer *in situ* transplanted tumor model.

In addition, an *in vitro* inhibition experiment (B of FIG. 54) showed that SMIO in a concentration range of 0 µM to 320 µM continuously acted on ID-8 cells for 48 h, and exhibited no significant inhibitory effect on ID-8.

### (2) Ovarian cancer abdominal transplanted tumor

Experimental method: A female C57/6 mouse ovarian cancer abdominal transplanted tumor model was constructed with an ovarian cancer murine ID8 cell line at a cell concentration of 2.5 × 10⁶. A model group and an SMIO intervention group were set for the experiment. The intervention group was intragastrically administered with the SMIO compound at 30 mg/kg every day from three days before modeling to the end of the 30-day modeling (model group, n = 10 mice; and SMIO compound (30 mg/kg) group, n = 11 mice). At the end of the experiment, blood was collected from the orbit, a body weight was measured, then the mice each were sacrificed, and ovary tissues were collected. Ovary tissue specimens were subjected to HE staining, and then the ovary tissue lesion and tumorigenesis of mice in each group were observed.

Experimental results: Compared with the model group, in the SMIO compound (30 mg/kg) intervention group, the body weight of mice was significantly reduced and the ascites volume was significantly reduced, that is, the administration of the SMIO compound significantly inhibited the ascites production and tumor progression of the ovarian cancer abdominal transplanted tumor in mice, where an inhibition rate for tumor proliferation of the greater omentum was as high as 58.70%, and an inhibition rate for the ascites weight was 83.78% (as shown in FIG. 55), indicating that the SMIO administration can significantly inhibit the occurrence and development of a tumor in the ID-8 ovarian cancer abdominal transplanted tumor model.

It can be seen from the above results that the CYP2E1 inhibitor SMIO has a significant prevention and treatment effect on the occurrence and development of the ID-8 ovarian cancer *in situ* transplanted tumor and abdominal transplanted tumor, indicating that the compound SMIO can be used for the prevention and treatment of ovarian cancer.

### Example 19 Inhibition of SMIO on rheumatoid arthritis in rats

### (1) Inhibition of CYP2E1 gene knockout on collagen-induced rheumatoid arthritis in rats

Experimental method: A collagen emulsion was prepared with collagen and incomplete freund's adjuvant (IFA) in a volume ratio of 1:1 and then used to construct an SD rat rheumatoid arthritis model. A model group and a CYP2E1 gene-knockout group were set. Each rat was injected subcutaneously with 0.25 mL of the collagen emulsion through the tail for a first immunization, and one week later, a second immunization was conducted in the same way as the first immunization. Within one week after the second immunization, if a rat arthritis score was greater than or equal to 4 (0 point: no redness and swelling; 1 point: slight swelling of a little toe joint; 2 points: swelling of a toe joint and a sole; 3 points: swelling of a toenail below an ankle; 4 points: swelling of the whole foot including an ankle; and 16 points: total points for four feet), it was determined that the modeling was successful. After the modeling was successful, a thickness of each of the left and right hindfeet and the left and right forefeet of rats was measured every day by a vernier caliper, and a volume of each of the left and right hindfeet of rats was measured by a rat foot swelling measuring instrument (which was measured every 12 h in the first 3 days). A foot swelling rate of each rat was calculated according to a change of a hindfoot volume of the rat relative to a hindfoot volume of the rat before the modeling at each time point, and a difference in foot swelling rate between groups of rats was analyzed. After the successful modeling, blood was collected from the orbit on day 7; rats were sacrificed on day 20, blood was collected, and serum was isolated; and tissues such as ankles, toe joints, spleens, and livers were collected. The tissues were subjected to a decalcification treatment and then to HE staining, and the joint lesion, synovial hyperplasia, and connective tissue proliferation were observed under a microscope.

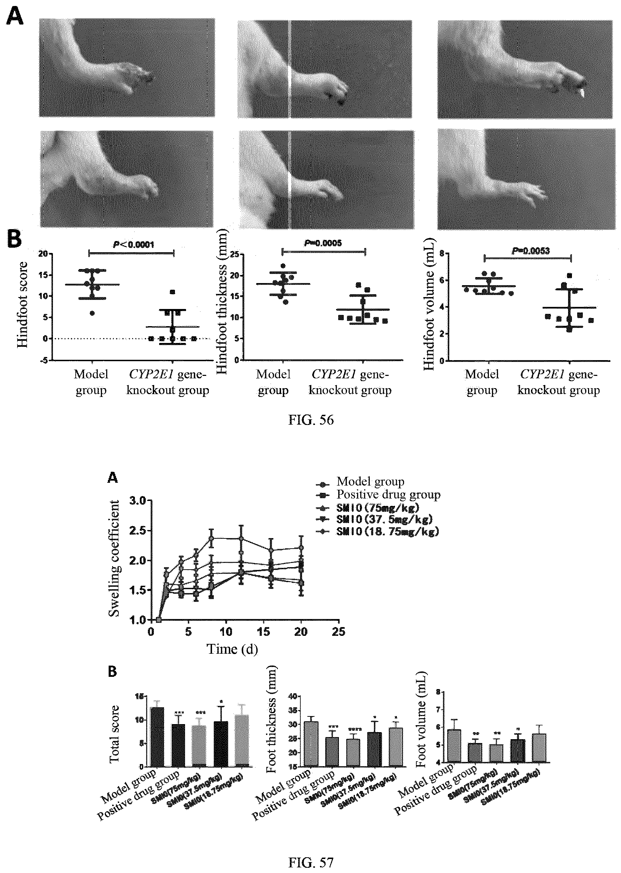

Experimental results: Compared with the model group, in the *CYP2E1* gene-knockout group, a success rate of rat modeling was 40%, which was significantly lower than that of the model group (90%); and a joint swelling degree of rats was also significantly lower than that of the model group (*P* < 0.05) (as shown in FIG. 56), indicating that the *CYP2E1* gene knockout can significantly reduce the incidence of collagen-induced rheumatoid arthritis and improve the swelling degree in rats.

### (2) Inhibition of SMIO on collagen-induced rheumatoid arthritis in rats

Experimental method: A collagen emulsion was used to construct an SD rat rheumatoid arthritis model. 50 rats successfully modeled were randomly divided into five groups: model group (normal saline (NS), 0.5 mL/kg, i.g.); positive control group (celecoxib, 5 mg/kg, i.g.); SMIO low-dose group (18.75 mg/kg, i.g.); SMIO medium-dose group (37.5 mg/kg, i.g.); and SMIO high-dose group (75 mg/kg, i.g.). The rats each were administered once every day starting from the first day after successful modeling. A thickness of each of the left and right hindfeet and the left and right forefeet of rats was measured every day by a vernier caliper, and a volume of each of the left and right hindfeet of rats was measured by a rat foot swelling measuring instrument (which was measured every 12 h in the first 3 days). A foot swelling rate of each rat was calculated according to a change of a hindfoot volume of the rat relative to a hindfoot volume of the rat before the modeling at each time point, and a difference in foot swelling rate between groups of rats was analyzed. After the successful modeling, blood was collected from the orbit on day 7; rats were sacrificed on day 20, blood was collected, and serum was isolated; and tissues such as ankles, toe joints, spleens, and livers were collected. The tissues were subjected to a decalcification treatment and then to HE staining, and the joint lesion, synovial hyperplasia, and connective tissue proliferation were observed under a microscope.

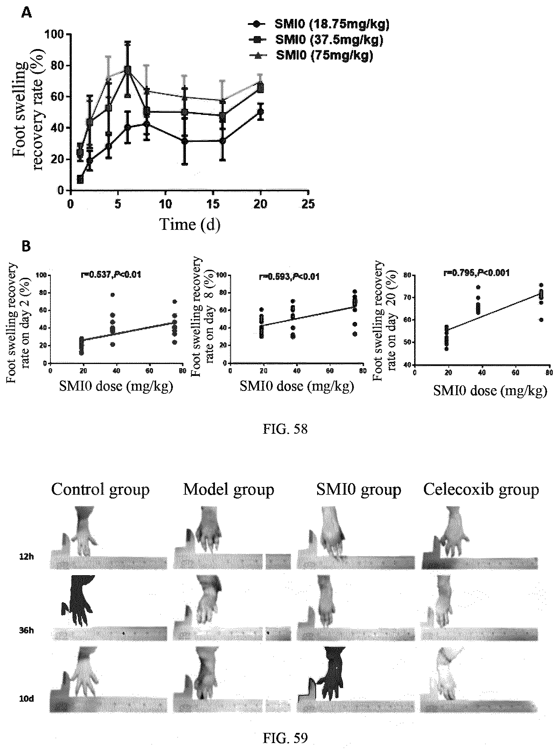

Experimental results: Compared with the model group, the foot swelling degrees in the SMIO (75 mg/kg, 37.5 mg/kg, and 18.75 mg/kg) groups were significantly reduced on day 21, where the SMIO (75 mg/kg) group had the optimal foot swelling improvement effect, which was similar to the swelling change in the 5 mg/kg celecoxib group (as shown in FIG. 57); and the analysis of a correlation between an SMIO dose and a foot swelling alleviation rate showed that the anti-rheumatoid arthritis effect of SMIO exhibited a significant dose-response relationship, where the SMIO dose was moderately correlated with the foot swelling alleviation rate (*P* < 0.01) from day 2 to day 8, and the SMIO dose was strongly correlated with the foot swelling alleviation rate on day 8 to day 20 *(P* < 0.001) (as shown in FIG. 58), indicating that SMIO can significantly alleviate the foot swelling in the collagen-induced rheumatoid arthritis rat model, which is dose-dependent.

### (3) Inhibition of SMIO on CFA-induced rheumatoid arthritis in rats

Experimental method: An SD rat rheumatoid arthritis model was constructed with CFA (0.1 mL/rat). The SMIO intervention group was divided into an ultra-low-dose group, a low-dose group, a medium-dose group, and a high-dose group, which were intragastrically administered with the compound SMIO respectively at 1 mg/kg, 6 mg/kg, 30 mg/kg, and 150 mg/kg every day starting from two days before modeling; and the positive control group was intragastrically administered with celecoxib at 5 mg/kg. 10 animals were provided for each group. 1 d before administration of CFA to the rats, the above grouping and doses were adopted, and then the rats were administered once every day until the modeling was completed on day 10. Each rat was administered with CFA at a sole of a right hindfoot. A volume of each of the right hindfeet of rats was measured every day by a rat foot swelling measuring instrument (which was measured every 12 h in the first 3 days). A foot swelling rate of each rat was calculated according to a change of a right hindfoot volume of the rat relative to a hindfoot volume of the rat before the CFA administration at each time point, and a difference in foot swelling rate between groups of rats was analyzed.

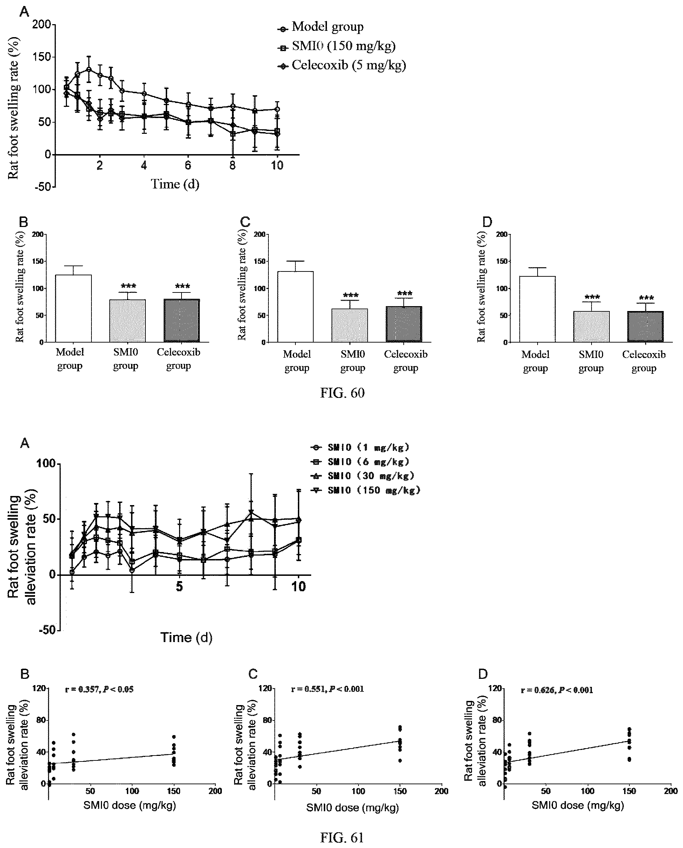

Experimental results: Compared with the control group, the feet of rats in the model group were significantly swollen (as shown in FIG. 59); compared with the model group, the rat foot swelling caused by CFA was significantly alleviated in the SMIO intervention group (as shown in FIG. 59 and FIG. 60, B of FIG. 60 shows the 24 h rat foot swelling rate, C of FIG. 60 shows the 36 h rat foot swelling rate, and D of FIG. 60 shows the 48 h rat foot swelling rate) (****P* < 0.001 vs the model group); the compound SMIO at 1 mg/kg, 6 mg/kg, 30 mg/kg, and 150 mg/kg exhibited rat foot swelling alleviation rates respectively of 20.8%, 34.2%, 44.1%, and 52.5% at 36 h (a time point when the maximum foot swelling degree was reached in the model group); and there was a prominent dose-response relationship between the SMIO dose and the rat foot swelling alleviation rate (*P* < 0.05) (as shown in A to D of FIG. 61, B of FIG. 61 shows the 24 h dose-response relationship, C of FIG. 61 shows the 36 h dose-response relationship, and D of FIG. 61 shows the 48 h dose-response relationship), indicating that SMIO can significantly alleviate the foot swelling in the CFA-induced rheumatoid arthritis rat model.

### (4) Correlation between CYP2E1 activity and rheumatoid arthritis severity in rats after SMIO inhibition

Experimental method: A calcium precipitation method was used to prepare a liver microsome of a collagen-induced rheumatoid arthritis rat model, and a Braford method was used to determine a protein concentration in the microsome. An incubation system was prepared with 2 × PBS, a CZX solution, and the liver microsome at a final concentration of 0.5 mg/mL, and then pre-incubated at 37°C for 5 min; NADPH was added to initiate a reaction, and a resulting mixture was incubated at 37°C for 30 min and then placed on ice to terminate the reaction; and ethyl acetate was added to extract 6-hydroxychlorzoxazone, a resulting mixture was vortexed and centrifuged, and a resulting upper organic phase was collected and blow-dried with nitrogen. A peak area of a CZX metabolite 6-hydroxychlorzoxazone was detected by HPLC under the following conditions: methanol : water = 56:44, and detection wavelength: 287 nm. A concentration ΔC of the metabolite 6-hydroxychlorzoxazone was calculated through substitution into a standard curve; and a reaction rate of conversion of CZX into 6-hydroxychlorzoxazone was calculated according to V(pmol/min/mg) = (ΔC*1000)/(B*T) to evaluate an enzymatic activity of CYP2E1, where B represents a protein concentration of the microsome (mg/mL) and T represents an incubation time (min). The correlation between the CYP2E1 activity and the rheumatoid arthritis severity in rats was analyzed.

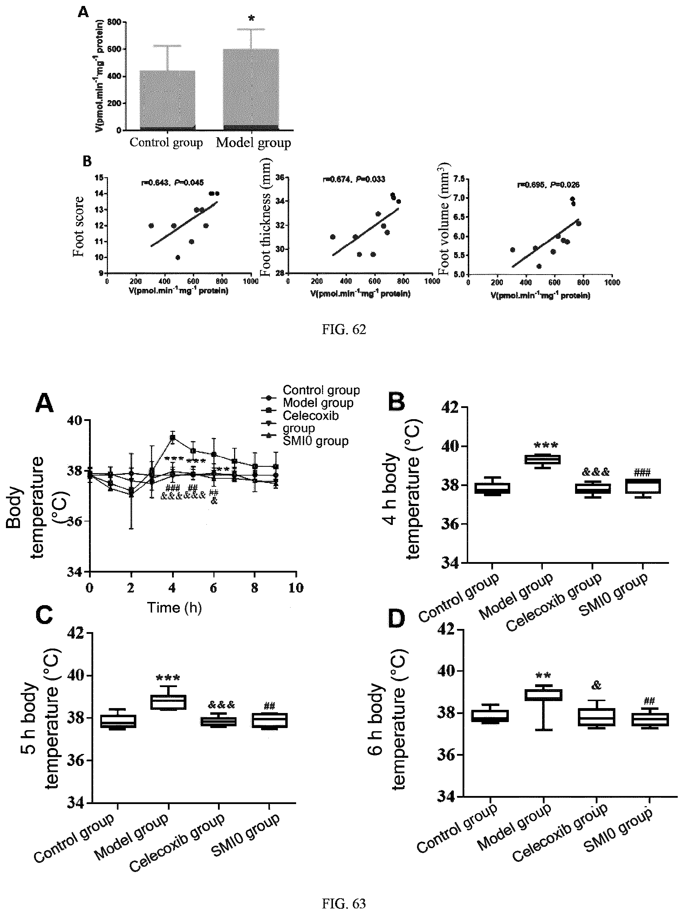

Experimental results: Compared with the control group, in the model group, the CYP2E1 activity was significantly increased (*P* < 0.05, A of FIG. 62), and the metabolic activity of CYP2E1 was significantly correlated with the arthritis score and the foot swelling thickness and volume (*P* < 0.05, as shown in B of FIG. 62), indicating that the enzymatic activity of CYP2E1 is positively correlated with the rheumatoid arthritis severity in rats, and the anti-rheumatoid arthritis effect of SMIO may be related to the inhibition on enzymatic activity of CYP2E1.

It can be seen from the above results that SMIO has a significant prevention and treatment effect on collagen and CFA-induced rheumatoid arthritis in rats, indicating that the compound SMIO can be used for the prevention and treatment of clinical rheumatic and rheumatoid arthritis.

### Example 20 Inhibition of SMIO on LPS-induced sepsis in rats and mice

### Experimental method:

Rats: An SD rat sepsis model was constructed through single intraperitoneal injection of LPS (5 mg/kg). 30 min before LPS administration, rats in the model group were administered with NS (0.5 mL/kg, i.g.), rats in the positive control group were administered with celecoxib (5 mg/kg, i.g.), and rats in the CYP2E1 inhibition group were administered with the compound SMIO (150 mg/kg, i.g.). After LPS administration, a body temperature of each rat was measured and recorded every hour until 9 h (control group, n = 8 rats; model group, n = 10 rats; celecoxib group, n = 10 rats; and SMIO compound group, n = 10 rats).

Mice: A mouse sepsis model was constructed through single intraperitoneal injection of LPS (15 mg/kg). 30 min before LPS administration, mice in the model group were administered with NS (0.5 mL/kg, i.g.), mice in the positive control group were administered with celecoxib (5 mg/kg, i.g.), and mice in the CYP2E1 inhibition group were administered with the compound SMIO (90 mg/kg, i.g.). A body temperature of each mouse was measured and recorded at 6 h, 12 h, and 24 h after LPS administration, and the body temperature was measured 10 times in total throughout the experimental process. The animals were sacrificed at 24 h, blood samples were collected, and renal and cardiac function-associated indexes were determined (control group, n = 8 mice; model group, n = 8 mice; celecoxib group, n = 8 mice; and SMIO compound group, n = 10 mice).

### Experimental results:

Rats: Compared with the control group, the body temperature was increased significantly at 4 h, 5 h, and 6 h in the model group; and compared with the model group, the increase in body temperature in the LPS-induced sepsis rat model was avoided in the SMIO compound group and the positive drug group (A to D of FIG. 63, ***P* < 0.01 and ***P < 0.001 vs the control group; and ^{#}*P* < 0.05, *^{##}P* < 0.01, and *^{###}P* < 0.001 vs the model group), indicating that the SMIO intervention can significantly inhibit the LPS-induced increase in body temperature of a rat and can maintain a body temperature of a rat normal.

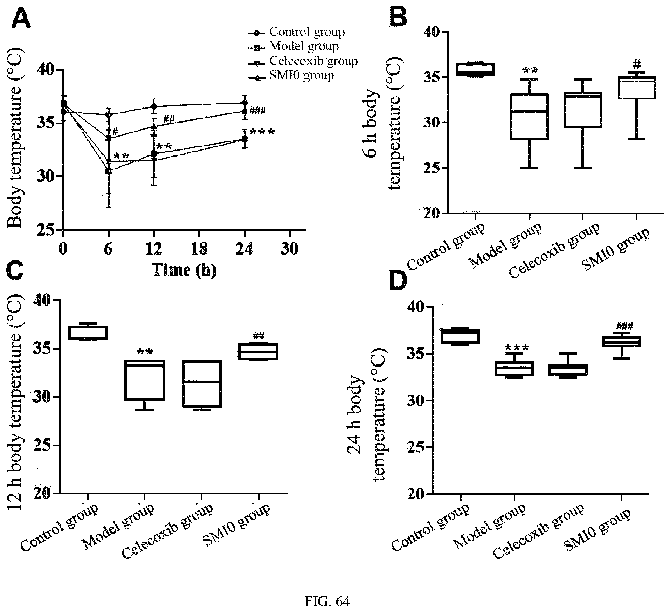

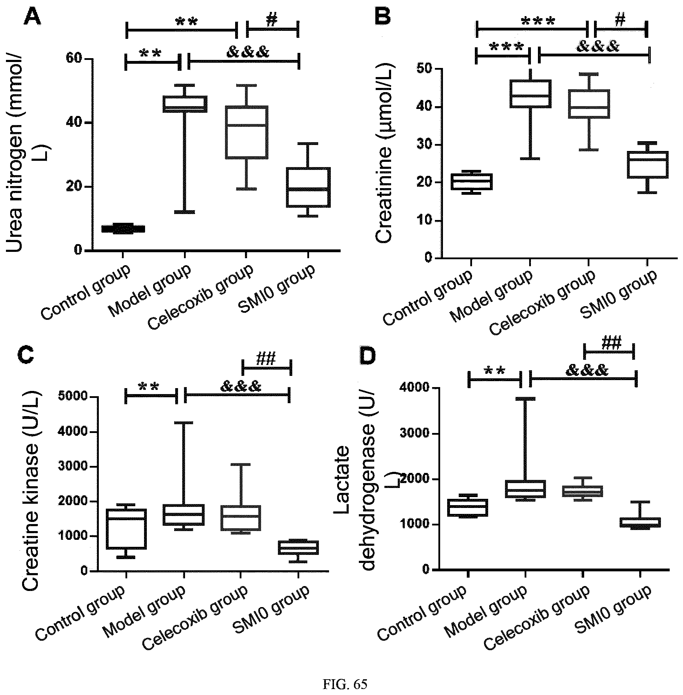

Mice: Compared with the control group, the body temperature was significantly reduced at 6 h, 12 h, and 24 h in the model group; compared with the model group, the decrease in body temperature in the LPS-induced sepsis mouse model was avoided in the SMIO compound group; compared with the control group, the body temperature at 6 h, 12 h, and 24 h was significantly lower than that before LPS administration in the model group (A to D of FIG. 64, ***P* < 0.01 and ***P < 0.001 vs the control group; *^{&}P* < 0.05 and *^{&&&}P* < 0.001 vs the model group; and *^{##}P* < 0.01 and *^{###}P* < 0.001 vs the celecoxib group); compared with the control group, the levels of renal function indexes urea nitrogen and creatinine and cardiac function indexes CK and LDH were significantly increased in the model group; and compared with the model group, the levels of renal and cardiac function-associated indexes were significantly reduced in the SMIO compound group (A to D of FIG. 65, ***P* < 0.01 and ***P < 0.001 vs the control group; *^{&&&}P <* 0.001 vs the model group; and ^{#}*P* < 0.01 and *^{##}P* < 0.001 vs the celecoxib group), indicating that the SMIO intervention can significantly inhibit the LPS-induced decrease in body temperature of a mouse, maintain a body temperature of a mouse normal, and improve the renal and cardiac function impairment in the LPS-induced sepsis mouse model.

It can be seen from the above results that SMIO can avoid a body temperature change in LPS-induced sepsis rat and mouse models. It is speculated that the compound SMIO can be used for the prevention and treatment of clinical sepsis.

### Example 21 Improvement of SMIO for cognitive dysfunction in an STZ-induced AD rat model

Experimental method: A male SD rat AD model was constructed through bilateral intracerebroventricular injection of STZ, where STZ was injected at 3 mg/kg on day 1 and STZ was injected at 1.5 mg/kg on day 3. STZ was injected into bilateral cerebral ventricles (0.9 mm posterior to the anterior fontanelle, 1.5 mm at the left or right of the sagittal suture, and below the brain surface and 3.8 mm to the skull) by a brain stereotaxic instrument. The sham-operated group was injected with a same volume of a control solvent according to same operations. The low-dose and high-dose prevention and treatment groups were intragastrically administered with the compound SMIO respectively at 10 mg/kg and 30 mg/kg starting from day 11 after modeling, and the administration was conducted consecutively for 21 d. On day 14 of the continuous administration (that is, on day 25 of modeling), the Morris water maze (MWM) was used to conduct spatial positioning navigation training consecutively for 6 d, and a spatial exploration experiment was conducted 24 h after the spatial positioning navigation training was completed (15 rats in each group).

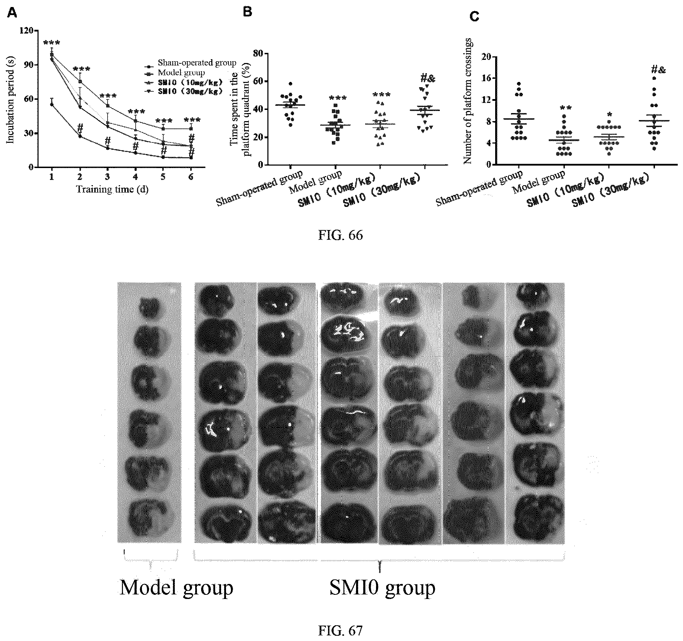

Experimental results: On day 1 to day 6 of the MWM positioning navigation experiment, the incubation period of rats in the model group was significantly extended compared with the sham-operated group; on day 2 to day 6, the incubation period of rats in the SMIO high-dose group was significantly shortened every day compared with the model group (Table 3 shows the quantification results of parameters related to improvement of the compound SMIO on cognitive dysfunction in the STZ-induced AD rat model; and as shown in Table 3 and A of FIG. 66, **P* < 0.05, ***P* < 0.01, and ****P* < 0.001 vs the sham-operated group, and ^{#}*P* < 0.05 vs the model group); on day 7 of the spatial exploration experiment, compared with the sham-operated group, the residence time in a quadrant of the original platform and the number of platform crossings were significantly shortened in the model group (***P* < 0.01 and ****P <* 0.001 vs the sham-operated group); compared with the model group, the residence time in a quadrant of the original platform and the number of platform crossings were significantly increased in the SMIO high-dose group (30 mg/kg) (as shown in Table 3 and B to C of FIG. 66, ^{#}*P* < 0.05 vs the model group); and compared with the SMIO low-dose group, the number of platform crossings and the residence time in a quadrant of the original platform were significantly increased in the SMIO high-dose group (^{&}*P* < 0.05 vs the low-dose group); and in terms of the overall acquisition of a learning and memory function, compared with the model group, the ICV-STZ-induced cognitive dysfunction was significantly improved in the SMIO high-dose group, with an improvement rate of 46.7%; and there was an improvement trend in the SMIO low-dose group, without a significant statistical difference and with an improvement rate of 32.6%, indicating that SMIO can significantly improve the cognitive dysfunction induced by lateral intracerebroventricular injection of STZ.

**Table 3 Quantification results of parameters related to improvement of the compound SMIO on cognitive dysfunction in the STZ-induced AD rat model**

| Group | n | Incubation period (s) | | | | | | Time spent in the platform quadrant (%) | Number of platform crossing |
|---|---|---|---|---|---|---|---|---|---|
| | | Day 1 | Day 2 | Day 3 | Day 4 | Day 5 | Day 6 | | |
| Sham-operated group | 15 | 55.2 ± 5.4 | 27.4 ± 3.3 | 16.6 ± 2.2 | 12.7 ± 2.9 | 8.9 ± 1.4 | 8.4 ± 1.4 | 43.1 ± 2.0 | 8.5 ± 0.9 |
| Model group | 15 | 99.1 ± 5.6*** | 75.3 ± 7.1 *** | 54.0 ± 5.6*** | 40.9 ± 5.0^{.}** | 34.0 ± 3.7*** | 34.0 ± 4.3 | 28.8 ± 1.9*** | 4.5 ± 0.6** |
| SMIO (10 mg/kg) | 15 | 95.7 ± 5.8*** | 61.1 ± 8.7** | 39.5 ± 8.0** | 33.2 ± 7.3** | 23.1 ± 5.8* | 18.5 ± 3.9*^{#} | 29.4 ± 2.4*** | 5.1 ±0.5* |
| SMIO (30 mg/kg) | 15 | 94.6 ± 4.3*** | 52.7 ± 7.2*^{#} | 35.9 ± 4.0*^{#} | 25.2 ± 3.4^{#} | 20.0 ± 3.2^{#} | 18.6 ± 3.2*^{#} | 39.3 ± 2.8^{#&} | 8.2 ± 1.0^{#&} |

It can be seen from the above results that SMIO at 30 mg/kg can significantly improve the cognitive dysfunction in the STZ-induced AD rat model. It is speculated that the compound SMIO can be used for the prevention and treatment of clinical AD.

### Example 22 Inhibition of SMIO on focal CIRI in rats

Experimental method: A focal CIRI rat model with middle cerebral artery occlusion (MCAO) was constructed by a modified Zea-longa suture-occluded method. A rat was intraperitoneally injected with 10% chloral hydrate for anaesthetization, and fixed on a thermostatic plate in a supine position such that a body temperature of the rat was maintained at 36.5°C to 37.5°C; the skin in the middle of the neck was disinfected with iodophor and then cut to expose the left common carotid artery (CCA), the CCA, external carotid artery (ECA), and internal carotid artery (ICA) were separated, and the pterygopalatine artery was ligated; a small incision was provided at a side of the ECA, and a nylon suture was inserted through the incision; with a fork route junction as a mark, the nylon suture was carefully and slowly pushed forwards when inserted by about 17 mm, and a resistance was felt at about 18 ± 0.5 mm, indicating that an end of the nylon suture had reached the anterior cerebral artery (ACA); the incision of the ECA was tightened, and the skin was sutured; 2 h after ischemia, the rat was anesthetized with inhalation sevoflurane, and the nylon suture was gently and slowly pulled out until a ball end of the nylon suture reached a bifurcation of the CCA, thereby achieving reperfusion; 2 h after ischemia, the reperfusion was conducted for 24 h, where 10 min before reperfusion, the SMIO intervention group was intragastrically administered with the compound SMIO (150 mg/kg), and the sham-operated group and the model group each were injected with an equal volume of a control solvent; neurobehavioral scoring was conducted at 2.5 h and 6 h after surgery; and 24 h after the reperfusion, the rat was decapitated, a brain tissue was collected and stained with triphenyltetrazolium chloride (TTC), and the changes of the cerebral infarction volume and cerebral edema were observed (10 rats in each group).

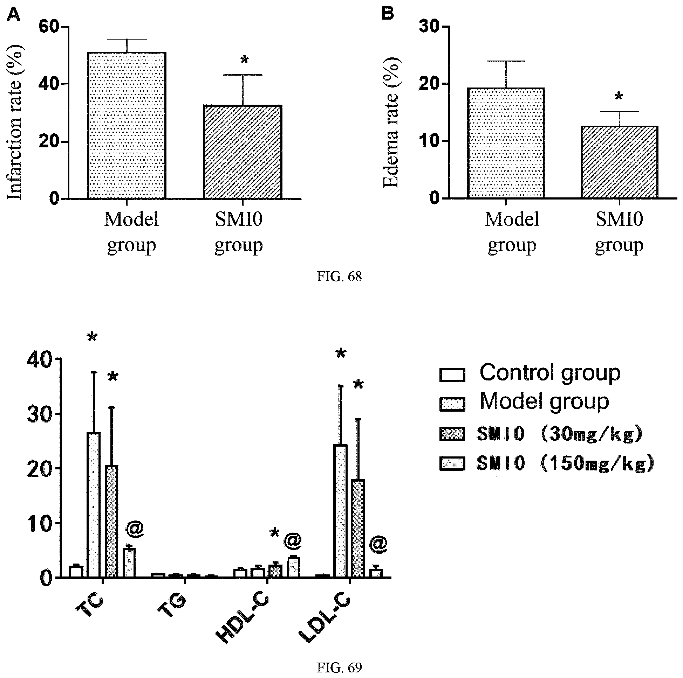

Experimental results: Compared with the model group, the cerebral infarction volume and cerebral edema volume were significantly reduced in the SMIO intervention group (as shown in FIG. 67 and A to B of FIG. 68, **P <* 0.05 vs the model group), where the cerebral infarction volume was reduced from 51.03% in the model group to 32.62%, the cerebral edema volume was reduced from 19.25% in the model group to 12.63%, and inhibition rates of SMIO (150 mg/kg) on the cerebral infarction volume and cerebral edema volume were 36.06% and 34.39% respectively, indicating that SMIO can significantly reduce the cerebral infarction volume and cerebral edema volume in the focal CIRI rat model with MCAO.

It can be seen from the above results that SMIO can significantly improve the cerebral infarction and cerebral edema in the focal CIRI rat model. It is speculated that the compound SMIO can be used for the prevention and treatment of clinical ischemic stroke.

Example 23 Reduction of SMIO in a blood lipid level of a hyperlipidemia ApoE^{-/-} mouse model induced by a high-fat diet

Experimental method: ApoE^{-/-} mice were fed with a high-fat and high-cholesterol feed to construct an ApoE^{-/-}mouse hyperlipidemia model. An SMIO intervention group was divided into a low-dose intervention group and a high-dose intervention group, which were administered with the compound SMIO at 30 mg/kg and 150 mg/kg every day from the beginning of modeling to the end of the 16-week modeling. At the end of the experiment, blood was collected from the orbit, and the levels of blood lipid-associated indexes in the serum of each mouse were determined by an automatic biochemical analyzer (8 mice in each group).

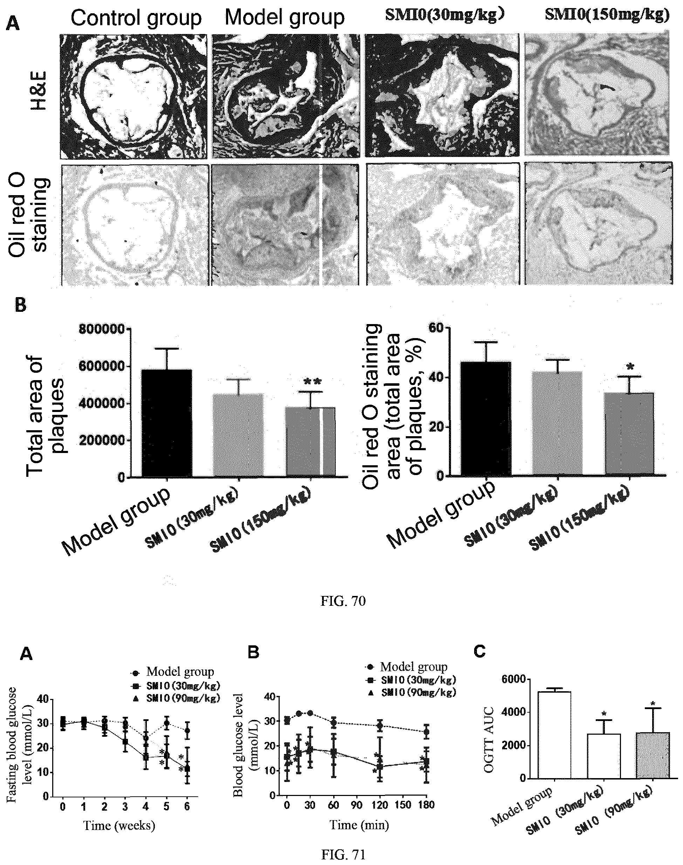

Experimental results: Compared with the control group, the levels of total cholesterol (TC) and low-density lipoprotein (LDL) in mice of the model group were significantly increased (as shown in Table 4 and FIG. 69, **P <* 0.05 vs the control group); compared with the model group, in the SMIO high-dose intervention group, the levels of TC and LDL were significantly reduced, and the level of high-density lipoprotein (HDL) was significantly increased (*^{@}P* < 0.05 vs the model group); and compared with the model group, the high blood lipid level induced by the high-fat diet was improved to some extent in the SMIO low-dose intervention group, without statistical significance. In addition, the SMIO administration significantly inhibited the formation of aortic atherosclerotic plaques in ApoE^{-/-}mice. HE staining results showed that, compared with the model group, the total area of aortic root lesions in mice was significantly reduced in the SMIO high-dose group (150 mg/kg) (as shown in FIG. 70, ***P* < 0.01 vs the model group); and oil red O staining results also showed that the total area of aortic root lesions was significantly reduced in the SMIO high-dose group (150 mg/kg) (as shown in FIG. 70, **P* < 0.05 vs the model group), indicating that the administration of SMIO at 150 mg/kg can significantly improve the high blood lipid level induced by the high-fat diet (decreasing the TC and LDL levels and increasing the HDL level) while inhibiting the formation of aortic atherosclerotic plaques in ApoE-/- mice.

**Table 4 Quantification results of parameters related to reduction of the compound SMIO in a blood lipid level in the hyperlipidemia mouse model induced by the high-fat diet**

| Group | n | Total cholesterol (mmol/L) | Triglyceride (mmol/L) | High-density lipoprotein (mmol/L) | Low-density lipoprotein(mmol/L) |
|---|---|---|---|---|---|
| Control group | 8 | 2.03 ± 0.45 | 0.64 ± 0.15 | 1.43 ± 0.42 | 0.45 ± 0.10 |
| Model group | 8 | 26.43 ± 10.23* | 0.57 ± 0.14 | 1.77 ± 0.46 | 24.26 ± 9.93* |
| SMI0 (30 mg/kg) | 8 | 20.45 ± 10.77* | 0.56 ± 0.04 | 2.37 ± 0.49* | 17.94 ± 11.16* |
| SMI0 (150 mg/kg) | 8 | 5.35 ± 0.55*^{@}* | 0.40 ± 0.10 | 3.68 ± 0.33*^{@} | 1.50 ± 0.77^{@} |

It can be seen from the above results that SMIO can significantly reduce the increase in blood lipid level and the formation of atherosclerotic plaques in mice caused by the high-fat diet. It is speculated that the compound SMIO can be used for the prevention and treatment of clinical hyperlipidemia, AS, and CHD.

### Example 24 Reduction of SMIO in a blood glucose level in a diabetes rat model induced by a high-fat diet and STZ

Experimental method: A diabetes rat model was constructed by a high-fat diet + single intraperitoneal injection of STZ (40 mg/kg). A FBG level of higher than or equal to 16.7 mmol/L after two weeks of molding was a criterion for successful molding. Modeled rats were divided into various groups according to the blood glucose level. Rats in a model group were administered with NS, and rats in SMIO low-dose and high-dose intervention groups were administered with the compound SMIO at 30 mg/kg and 150 mg/kg every day, respectively. The FBG level in rats was determined and recorded regularly every week until the sixth week after administration. 10 rats were provided in each group.

Experimental results: Compared with the model group, FBG levels in rats in the SMIO low-dose and high-dose intervention groups at weeks 5 and 6 were significantly reduced (**P* < 0.05 vs the model group) (A of FIG. 71); and glucose tolerance test results showed that the glucose tolerance of rats in the SMIO low-dose and high-dose intervention groups was significantly improved (B to C of FIG. 71), indicating that the SMIO intervention can significantly improve the high blood glucose level in rats induced by a high-fat diet and STZ.

It can be seen from the above results that SMIO can significantly reduce a blood glucose level in diabetes rats induced by a high-fat diet and STZ. It is speculated that the compound SMIO can be used for the prevention and treatment of clinical diabetes.

### Example 25 Increase in expression of CYP2E1 in a paracancerous tissue of bladder cancer

Experimental method: The changes of CYP2E1 expression in paracancerous tissues of 30 clinical bladder cancer patients were investigated with bladder tissues of 30 healthy individuals as a control, and the changes of CYP2E1 contents in the paracancerous tissues of the bladder cancer patients were determined.

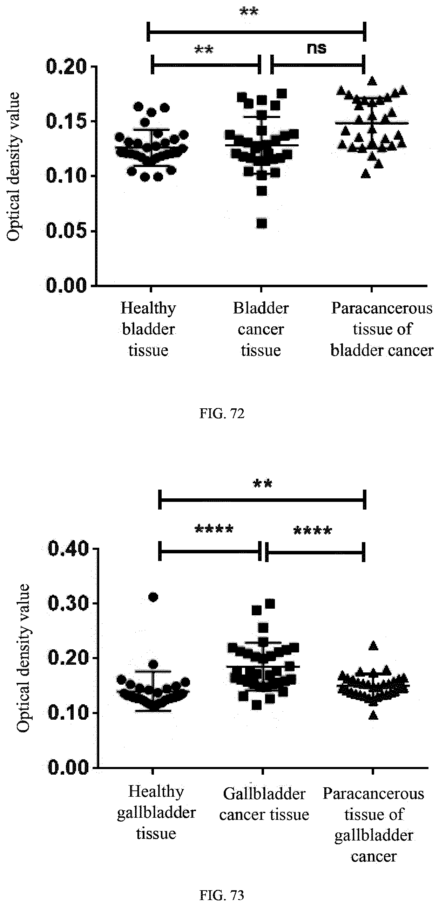

Experimental results: Immunohistochemical results showed that the expression of CYP2E1 in the paracancerous tissue of bladder cancer was significantly higher than that in the healthy bladder tissue (*P* < 0.05), as shown in FIG. 72. Since the expression of CYP2E1 in lung cancer, liver cancer, and glioma can be significantly increased, CYP2E1 can play a role in inflammation-associated tumors, and the compound SMIO can significantly inhibit the occurrence and development of lung cancer, liver cancer, and glioma, it is speculated that the increased expression of CYP2E1 in the paracancerous tissue of bladder cancer is related to the occurrence of bladder cancer, the compound SMIO can have a prevention and treatment effect on the occurrence and development of bladder cancer, and the compound SMIO can be used for the prevention and treatment of clinical bladder cancer.

### Example 26 Increase in expression of CYP2E1 in a paracancerous tissue of gallbladder cancer

Experimental method: The changes of CYP2E1 expression in paracancerous tissues of 33 clinical gallbladder cancer patients were investigated with gallbladder tissues of 31 healthy individuals as a control.

Experimental results: Immunohistochemical results showed that the expression of CYP2E1 in the paracancerous tissue of gallbladder cancer was significantly higher than that in the healthy gallbladder tissue (*P* < 0.05), as shown in FIG. 73. Since the expression of CYP2E1 in lung cancer, liver cancer, and glioma can be significantly increased, CYP2E1 can play a role in inflammation-associated tumors, and the compound SMIO can significantly inhibit the occurrence and development of lung cancer, liver cancer, and glioma, it is speculated that the increased expression of CYP2E1 in the paracancerous tissue of gallbladder cancer is related to the occurrence of gallbladder cancer, the compound SMIO can have a prevention and treatment effect on the occurrence and development of gallbladder cancer, and the compound SMIO can be used for the prevention and treatment of clinical gallbladder cancer.

## Claims

1. A compound or a salt thereof selected from 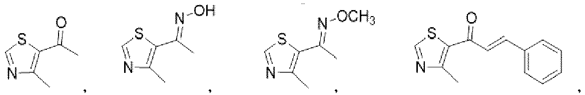 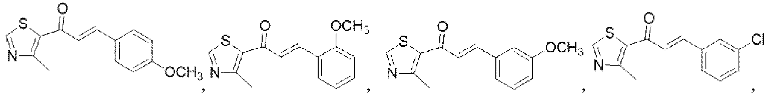 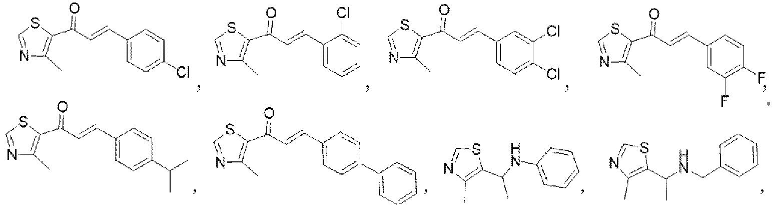 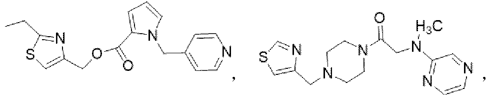 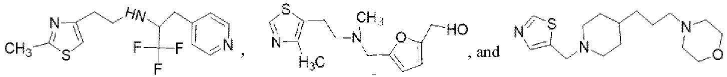 for use in the treatment or prevention of an inflammation-mediated disease selected from liver cancer, glioma, ovarian cancer, lung cancer, bladder cancer, gallbladder cancer, liver damage, fatty liver, hepatitis, liver fibrosis, pulmonary fibrosis, rheumatic and rheumatoid arthritis, sepsis, Alzheimer's disease, ischemic stroke, Parkinson's disease, hyperlipidemia, atherosclerosis, coronary heart disease, and diabetes.

2. The compound for use according to claim 1, wherein the compound is selected from the group consisting of:

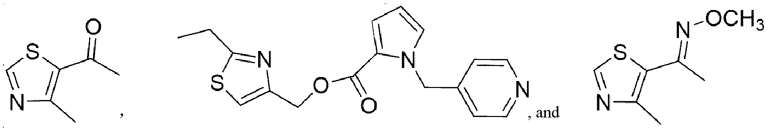

3. The salt of the compound for use according to claim 1, wherein the salt is an acid salt of the compound of an inorganic acid or an organic acid;
wherein the inorganic acid is at least one selected from the group consisting of hydrochloric acid, hydrobromic acid, hydroiodic acid, sulfuric acid, nitric acid, and phosphoric acid; and
the organic acid is at least one selected from the group consisting of acetic acid, oxalic acid, succinic acid, tartaric acid, malic acid, lactic acid, methanesulfonic acid, p-toluenesulfonic acid, citric acid, maleic acid, fumaric acid, salicylic acid, and acetylsalicylic acid.

4. The compound for use according to claim 1, wherein the compound has a structural formula of 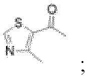 an X-ray powder diffraction pattern of a crystal form A of a hydrochloride of the compound comprises 3 or more 2*θ* values selected from the group consisting of 8.4 ± 0.2°, 13.1 ± 0.2°, 14.8 ± 0.2°, 16.6 ± 0.2°, 24.1 ± 0.2°, 27.2 ± 0.2°, 30.5 ± 0.2°, 31.8 ± 0.2°, 33.5 ± 0.2°, 35.4 ± 0.2°, and 35.7 ± 0.2°; and
a differential scanning calorimetry-thermogravimetric analyzer pattern of the crystal form A of the hydrochloride of the compound comprises a significant endothermic peak at 70°C to 220°C and shows thermal decomposition at 80°C to 170°C.

5. The compound for use according to claim 1, wherein the compound has a structural formula of 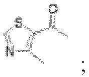 an X-ray powder diffraction pattern of a crystal form B of a sulfate of the compound comprises 5 or more 2*θ* values selected from the group consisting of 10.1 ± 0.2°, 15.1 ± 0.2°, 16.0 ± 0.2°, 16.7 ± 0.2°, 19.2 ± 0.2°, 19.9 ± 0.2°, 23.4 ± 0.2°, 24.0 ± 0.2°, 25.8 ± 0.2°, 26.5 ± 0.2°, 28.9 ± 0.2°, 30.3 ± 0.2°, and 32.2 ± 0.2°; and
a differential scanning calorimetry-thermogravimetric analyzer pattern of the crystal form B of the sulfate of the compound comprises at least one endothermic peak at 30°C to 85°C, 90°C to 160°C, or 215°C to 330°C and shows thermal decomposition at 150°C to 350°C.

6. A method for preparing the compound according to claim 1, wherein the method is at least one selected from the group consisting of the following methods:
method 1: subjecting a raw material comprising a compound A, an aprotic solvent, and a Grignard reagent to a reaction at -20°C to 25°C for 0.5 h to 3 h to obtain a CYP2E1 inhibitor A,
wherein the compound A is at least one selected from the group consisting of compounds with a structural formula shown in formula II: 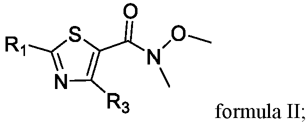 and
the CYP2E1 inhibitor A is at least one selected from the group consisting of compounds with a structural formula shown in formula III: 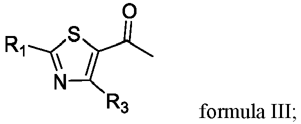
method 2: subjecting a compound with a structural formula shown in formula III and a hydroxylamine hydrochloride to a reaction in the presence of an alkali source I to obtain a CYP2E1 inhibitor B,
wherein the CYP2E1 inhibitor B is at least one selected from the group consisting of compounds with a structural formula shown in formula III-1: 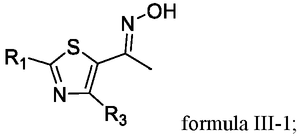
method 3: subjecting a compound with a structural formula shown in formula III and an aromatic aldehyde compound to a reaction at 25°C to 100°C for 2 h to 8 h in the presence of an alkali source II to obtain a CYP2E1 inhibitor C,
wherein the CYP2E1 inhibitor C is at least one selected from the group consisting of compounds with a structural formula shown in formula III-2: 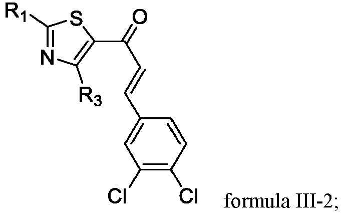 and
method 4: subjecting a compound with a structural formula shown in formula III, an amine compound, and a reducing agent to a reaction in the presence of an acid source to obtain a CYP2E1 inhibitor D,
wherein the amine compound is at least one selected from the group consisting of phenylamine and benzylamine; and
the CYP2E1 inhibitor D is at least one selected from the group consisting of compounds with a structural formula shown in formula III-3: 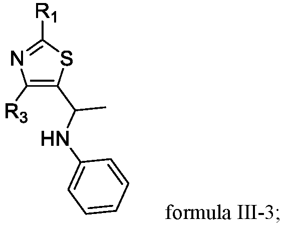
wherein in the formula II, formula III, formula III-I, formula III-2, and formula III-3, R₁ is any one selected from the group consisting of hydrogen, C₁ alkyl, and C₂ alkyl; and R₃ is at least one selected from the group consisting of hydrogen and C₁ alkyl.

7. The method according to claim 6, wherein in the method 1, the aprotic solvent is at least one selected from the group consisting of tetrahydrofuran and diethyl ether and the Grignard reagent is at least one selected from the group consisting of methylmagnesium bromide and methylmagnesium chloride;
in the method 2, the alkali source I is at least one selected from the group consisting of potassium hydroxide, sodium hydroxide, sodium carbonate, pyridine, triethylamine, and N,N-diisopropylethylamine;
in the method 3, the alkali source II is at least one selected from the group consisting of sodium hydroxide, potassium hydroxide, potassium tert-butoxide, sodium methoxide, and potassium fluoride; and
the aromatic aldehyde compound is at least one selected from the group consisting of p-methoxybenzaldehyde, o-methoxybenzaldehyde, m-methoxybenzaldehyde, p-chlorobenzaldehyde, o-chlorobenzaldehyde, m-chlorobenzaldehyde, p-phenylbenzaldehyde, p-isopropylbenzaldehyde, and 3,4-difluorobenzaldehyde; and
in the method 4, the acid source is at least one selected from the group consisting of formic acid, acetic acid, and hydrochloric acid; and
the reducing agent is at least one selected from the group consisting of sodium cyanoborohydride, sodium borohydride, and lithium aluminum hydride.

8. The method according to claim 6, wherein in the method 1, a molar ratio of the compound selected from the group consisting of compounds with the structural formula shown in formula II to the Grignard reagent is 1:1 to 1:3;
in the method 2, a molar ratio of the compound with the structural formula shown in formula III to the hydroxylamine hydrochloride is 1:1 to 1:6;
in the method 3, a molar ratio of the compound with the structural formula shown in formula III to the aromatic aldehyde compound is 1:1 to 1:5; and
in the method 4, a molar ratio of the compound with the structural formula shown in formula III to the reducing agent is 1:1 to 1:5.

9. The method according to claim 6, wherein the compound A is prepared through the following process:
subjecting a raw material comprising a compound A-1, a condensing agent, N,O-dimethylhydroxylamine hydrochloride, and an aprotic solvent to a reaction at 20°C to 60°C for 10 h to 20 h in the presence of an alkali source III to obtain the compound A,
wherein the compound A-1 is at least one selected from the group consisting of compounds with a structural formula shown in formula II-1:

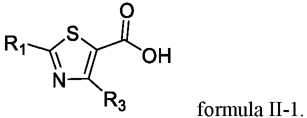

10. The method according to claim 9, wherein the condensing agent is at least one selected from the group consisting of 1-ethyl-(3-dimethylaminopropyl)carbodiimide hydrochloride, didodecyl carbonate, N,N-carbonyldiimidazole, dicyclohexylcarbodiimide, and N-(4-carboxyphenyl) maleimide; and
the alkali source III is at least one selected from the group consisting of sodium hydroxide, potassium hydroxide, calcium hydroxide, sodium carbonate, potassium carbonate, pyridine, triethylamine, and N,N-diisopropylethylamine.

11. The method according to claim 9, wherein the compound selected from the group consisting of compounds with a structural formula shown in formula II-1, the condensing agent, and the N,O-dimethylhydroxylamine hydrochloride are in a molar ratio of 1:1:1 to 1:5:5.

12. The method according to claim 9, wherein the preparation of the compound selected from the group consisting of compounds with a structural formula shown in formula II-1 at least comprises the following steps:
subjecting a raw material comprising a compound selected from the group consisting of compounds with a structural formula shown in formula II-2 to hydrolysis in the presence of an alkali source IV to obtain a mixture, and adjusting a pH of the mixture with an acid source to 2 to 3 to obtain the compound selected from the group consisting of compounds with a structural formula shown in formula II-1,

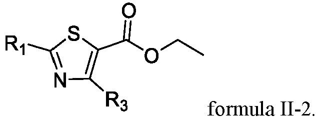

13. The method according to claim 12, wherein the alkali source IV is at least one selected from the group consisting of sodium hydroxide, potassium hydroxide, calcium hydroxide, sodium carbonate, and potassium carbonate.

14. The method according to claim 12, wherein the acid source is concentrated hydrochloric acid; and
the adjusting a pH of the mixture with an acid source to 2 to 3 is conducted at 10°C to 50°C.

15. The salt of the compound for use according to claim 1, wherein the salt is obtained by:
subjecting a material comprising the compound and a solvent A to a reaction at - 20°C to 80°C for 0.5 h to 10 h to obtain the salt of the compound;
wherein the solvent A is at least one selected from the group consisting of an ether compound, an alcohol compound, an ester compound, a nitrile compound, a ketone compound, a haloalkane, an alkane, and an aromatic hydrocarbon.

## Patentansprüche

1. Verbindung oder Salz davon, ausgewählt aus 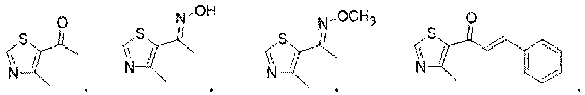 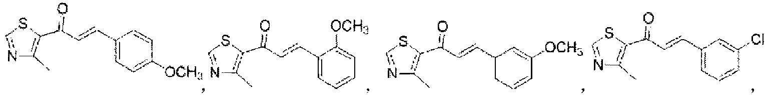 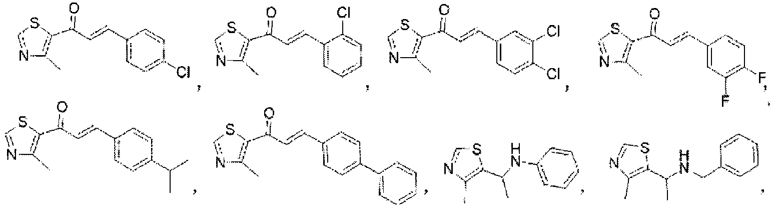 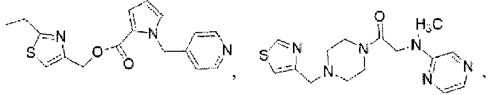 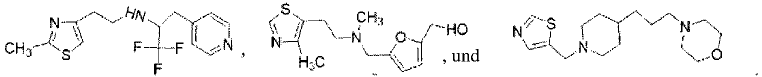 zur Verwendung bei der Behandlung oder Vorbeugung einer entzündungsbedingten Krankheit, ausgewählt aus Leberkrebs, Gliom, Eierstockkrebs, Lungenkrebs, Blasenkrebs, Gallenblasenkrebs, Leberschäden, Fettleber, Hepatitis, Leberfibrose, Lungenfibrose, rheumatischer und rheumatoider Arthritis, Sepsis, Alzheimer-Krankheit, ischämischem Schlaganfall, Parkinson-Krankheit, Hyperlipidämie, Arteriosklerose, koronarer Herzkrankheit und Diabetes.

2. Verbindung zur Verwendung nach Anspruch 1, wobei die Verbindung ausgewählt ist aus der Gruppe bestehend aus:

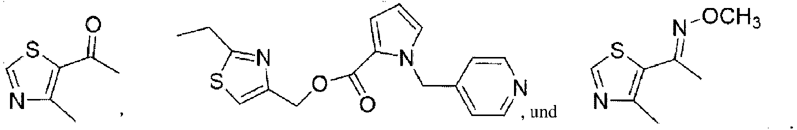

3. Salz der Verbindung zur Verwendung nach Anspruch 1, wobei das Salz der Verbindung ein Säuresalz einer anorganischen Säure oder einer organischen Säure ist;
wobei die anorganische Säure mindestens eine ausgewählt aus der Gruppe bestehend aus Chlorwasserstoffsäure, Bromwasserstoffsäure, Iodwasserstoffsäure, Schwefelsäure, Salpetersäure und Phosphorsäure ist; und
die organische Säure mindestens eine ausgewählt aus der Gruppe bestehend aus Essigsäure, Oxalsäure, Bernsteinsäure, Weinsäure, Äpfelsäure, Milchsäure, Methansulfonsäure, p-Toluolsulfonsäure, Zitronensäure, Maleinsäure, Fumarsäure, Salicylsäure und Acetylsalicylsäure ist.

4. Verbindung zur Verwendung nach Anspruch 1, wobei die Verbindung eine Strukturformel 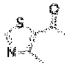 aufweist; wobei ein Röntgenpulverbeugungsmuster einer Kristallform A eines Hydrochlorids der Verbindung 3 oder mehr 2*θ*-Werte umfasst, ausgewählt aus der Gruppe bestehend aus 8,4 ± 0,2°, 13,1 ± 0,2°, 14,8 ± 0,2°, 16,6 ± 0,2°, 24,1 ± 0,2°, 27,2 ± 0,2°, 30,5 ± 0,2°, 31,8 ± 0,2°, 33,5 ± 0,2°, 35,4 ± 0,2° und 35,7 ± 0,2°; und
ein Muster eines Differentialscanningkalorimetrie-Thermogravimetrie-Analysators der Kristallform A des Hydrochlorids der Verbindung einen signifikanten endothermen Peak bei 70 °C bis 220 °C umfasst und bei 80 °C bis 170 °C thermische Zersetzung zeigt.

5. Verbindung zur Verwendung nach Anspruch 1, wobei die Verbindung eine Strukturformel 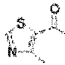 aufweist; wobei ein Röntgenpulverbeugungsmuster einer Kristallform B eines Sulfats der Verbindung 5 oder mehr 2*θ*-Werte umfasst, ausgewählt aus der Gruppe bestehend aus 10,1 ± 0,2°, 15,1 ± 0,2°, 16,0 ± 0,2°, 16,7 ± 0,2°, 19,2 ± 0,2°, 19,9 ± 0,2°, 23,4 ± 0,2°, 24,0 ± 0,2°, 25,8 ± 0,2°, 26,5 ± 0,2°, 28,9 ± 0,2°, 30,3 ± 0,2° und 32,2 ± 0,2°; und
ein Muster eines Differentialscanningkalorimetrie-Thermogravimetrie-Analysators der Kristallform B des Sulfats der Verbindung mindestens einen endothermen Peak bei 30 °C bis 85 °C, 90 °C bis 160 °C oder 215 °C bis 330 °C umfasst und bei 150 °C bis 350 °C thermische Zersetzung zeigt.

6. Verfahren zum Herstellen der Verbindung nach Anspruch 1, wobei das Verfahren mindestens eines ausgewählt aus der Gruppe bestehend aus den folgenden Verfahren ist:
Verfahren 1: Unterziehen eines Ausgangsmaterials, das eine Verbindung A, ein aprotisches Lösungsmittel und ein Grignard-Reagenz umfasst, einer Reaktion bei -20 °C bis 25 °C für 0,5 h bis 3 h, um einen CYP2E1-Inhibitor A zu erhalten,
wobei die Verbindung A mindestens eine ausgewählt aus der Gruppe bestehend aus Verbindungen mit einer in Formel II gezeigten Strukturformel ist: 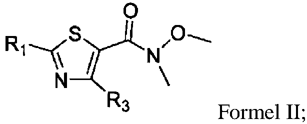 und
der CYP2E1-Inhibitor A mindestens einer ausgewählt aus der Gruppe bestehend aus Verbindungen mit einer in Formel III gezeigten Strukturformel ist: 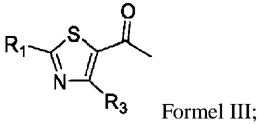
Verfahren 2: Unterziehen einer Verbindung mit einer in Formel III gezeigten Strukturformel und eines Hydroxylaminhydrochlorids einer Reaktion in Gegenwart einer Alkaliquelle I, um einen CYP2E1-Inhibitor B zu erhalten,
wobei der CYP2E1-Inhibitor B mindestens einer ausgewählt aus der Gruppe bestehend aus Verbindungen mit einer in Formel III-1 gezeigten Strukturformel ist: 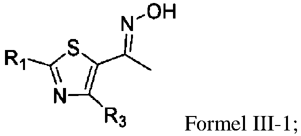
Verfahren 3: Unterziehen einer Verbindung mit einer in Formel III gezeigten Strukturformel und einer aromatischen Aldehydverbindung einer Reaktion bei 25 °C bis 100 °C für 2 h bis 8 h in Gegenwart einer Alkaliquelle II, um einen CYP2E1-Inhibitor C zu erhalten,
wobei der CYP2E1-Inhibitor C mindestens einer ausgewählt aus der Gruppe bestehend aus Verbindungen mit einer in Formel III-2 gezeigten Strukturformel ist: 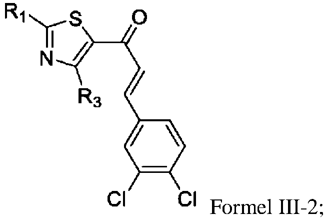 und
Verfahren 4: Unterziehen einer Verbindung mit einer in Formel III gezeigten Strukturformel, einer Aminverbindung und eines Reduktionsmittels einer Reaktion in Gegenwart einer Säurequelle, um einen CYP2E1-Inhibitor D zu erhalten,
wobei die Aminverbindung mindestens eine ausgewählt aus der Gruppe bestehend aus Phenylamin und Benzylamin ist; und
der CYP2E1-Inhibitor D ist mindestens einer ausgewählt aus der Gruppe bestehend aus Verbindungen mit einer in Formel III-3 gezeigten Strukturformel ist: 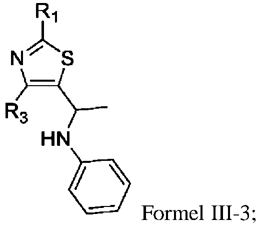
wobei in der Formel II, Formel III, Formel III-1, Formel III-2 und Formel III-3 R₁ ein beliebiges ausgewählt aus der Gruppe bestehend aus Wasserstoff, C₁-Alkyl und C₂-Alkyl ist; und R₃ mindestens eines ausgewählt aus der Gruppe bestehend aus Wasserstoff und C₁-Alkyl ist.

7. Verfahren nach Anspruch 6, wobei in Verfahren 1 das aprotische Lösungsmittel mindestens eines ausgewählt aus der Gruppe bestehend aus Tetrahydrofuran und Diethylether ist, und das Grignard-Reagenz mindestens eines ausgewählt aus der Gruppe bestehend aus Methylmagnesiumbromid und Methylmagnesiumchlorid ist;
wobei in Verfahren 2 die Alkaliquelle I mindestens eine ausgewählt aus der Gruppe bestehend aus Kaliumhydroxid, Natriumhydroxid, Natriumcarbonat, Pyridin, Triethylamin und N,N-Diisopropylethylamin ist;
wobei in Verfahren 3 die Alkaliquelle II mindestens eine ausgewählt aus der Gruppe bestehend aus Natriumhydroxid, Kaliumhydroxid, Kalium-tert-butoxid, Natriummethoxid und Kaliumfluorid ist; und
die aromatische Aldehydverbindung mindestens eine ausgewählt aus der Gruppe bestehend aus p-Methoxybenzaldehyd, o-Methoxybenzaldehyd, m-Methoxybenzaldehyd, p-Chlorbenzaldehyd, o-Chlorbenzaldehyd, m-Chlorbenzaldehyd, p-Phenylbenzaldehyd, p-Isopropylbenzaldehyd und 3,4-Difluorbenzaldehyd ist; und
bei Verfahren 4 die Säurequelle mindestens eine ausgewählt aus der Gruppe bestehend aus Ameisensäure, Essigsäure und Chlorwasserstoffsäure ist; und
das Reduktionsmittel mindestens eines ausgewählt aus der Gruppe bestehend aus Natriumcyanoborhydrid, Natriumborhydrid und Lithiumaluminiumhydrid ist.

8. Verfahren nach Anspruch 6, wobei bei Verfahren 1 ein Molverhältnis der Verbindung, ausgewählt aus der Gruppe bestehend aus Verbindungen mit der in Formel II gezeigten Strukturformel, zu dem Grignard-Reagenz 1:1 bis 1:3 beträgt;
bei Verfahren 2 das Molverhältnis der Verbindung mit der in Formel III gezeigten Strukturformel zu dem Hydroxylaminhydrochlorid 1:1 bis 1:6 beträgt;
bei Verfahren 3 das Molverhältnis der Verbindung mit der in Formel III gezeigten Strukturformel zu der aromatischen Aldehydverbindung 1:1 bis 1:5 beträgt; und
bei Verfahren 4 das Molverhältnis der Verbindung mit der in Formel III gezeigten Strukturformel zu dem Reduktionsmittel 1:1 bis 1:5 beträgt.

9. Verfahren nach Anspruch 6, wobei die Verbindung A durch den folgenden Prozess hergestellt wird:
Unterziehen eines Ausgangsmaterials, das eine Verbindung A-1, ein Kondensationsmittel, N,O-Dimethylhydroxylaminhydrochlorid und ein aprotisches Lösungsmittel umfasst, einer Reaktion bei 20 °C bis 60 °C für 10 h bis 20 h in Gegenwart einer Alkaliquelle III, um die Verbindung A zu erhalten,
wobei die Verbindung A-1 mindestens eine ausgewählt aus der Gruppe bestehend aus Verbindungen mit einer in Formel II-1 gezeigten Strukturformel ist:

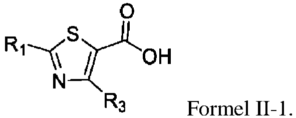

10. Verfahren nach Anspruch 9, wobei das Kondensationsmittel mindestens eines ausgewählt aus der Gruppe bestehend aus 1-Ethyl-(3-dimethylaminopropyl)carbodiimidhydrochlorid, Didodecylcarbonat, N,N-Carbonyldiimidazol, Dicyclohexylcarbodiimid und N-(4-Carboxyphenyl)maleimid ist; und
die Alkaliquelle III mindestens eine ausgewählt aus der Gruppe bestehend aus Natriumhydroxid, Kaliumhydroxid, Calciumhydroxid, Natriumcarbonat, Kaliumcarbonat, Pyridin, Triethylamin und N,N-Diisopropylethylamin ist.

11. Verfahren nach Anspruch 9, wobei die Verbindung ausgewählt aus der Gruppe bestehend aus Verbindungen mit einer in Formel II-1 gezeigten Strukturformel, das Kondensationsmittel und das N,O-Dimethylhydroxylaminhydrochlorid in einem Molverhältnis von 1:1:1 bis 1:5:5 vorliegen.

12. Verfahren nach Anspruch 9, wobei die Herstellung der Verbindung, ausgewählt aus der Gruppe bestehend aus Verbindungen mit einer in Formel II-1 gezeigten Strukturformel, mindestens die folgenden Schritte umfasst:
Unterziehen eines Ausgangsmaterials, das eine Verbindung umfasst, ausgewählt aus der Gruppe bestehend aus Verbindungen mit einer in Formel II-2 gezeigten Strukturformel, einer Hydrolyse in Gegenwart einer Alkaliquelle IV, um eine Mischung zu erhalten, und Einstellen des pH-Werts der Mischung mit einer Säurequelle auf 2 bis 3, um die Verbindung zu erhalten, ausgewählt aus der Gruppe bestehend aus Verbindungen mit einer in Formel II-1 gezeigten Strukturformel,

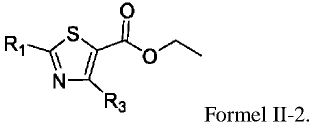

13. Verfahren nach Anspruch 12, wobei die Alkaliquelle IV mindestens eine ausgewählt aus der Gruppe bestehend aus Natriumhydroxid, Kaliumhydroxid, Calciumhydroxid, Natriumcarbonat und Kaliumcarbonat ist.

14. Verfahren nach Anspruch 12, wobei die Säurequelle konzentrierte Chlorwasserstoffsäure ist; und
das Einstellen des pH-Werts der Mischung mit einer Säurequelle auf 2 bis 3 bei 10 °C bis 50 °C ausgeführt wird.

15. Salz der Verbindung zur Verwendung nach Anspruch 1, wobei das Salz erhalten wird durch:
Unterziehen eines Materials, das die Verbindung und ein Lösungsmittel A umfasst, einer Reaktion bei -20 °C bis 80 °C für 0,5 h bis 10 h, um das Salz der Verbindung zu erhalten;
wobei das Lösungsmittel A mindestens eines ausgewählt aus der Gruppe bestehend aus einer Etherverbindung, einer Alkoholverbindung, einer Esterverbindung, einer Nitrilverbindung, einer Ketonverbindung, einem Halogenalkan, einem Alkan und einem aromatischen Kohlenwasserstoff ist.

## Revendications

1. Composé ou un sel de celui-ci choisi parmi 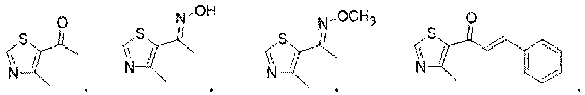 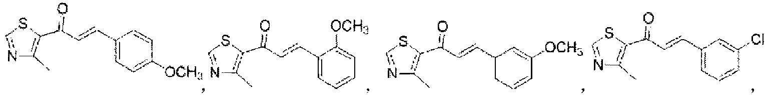 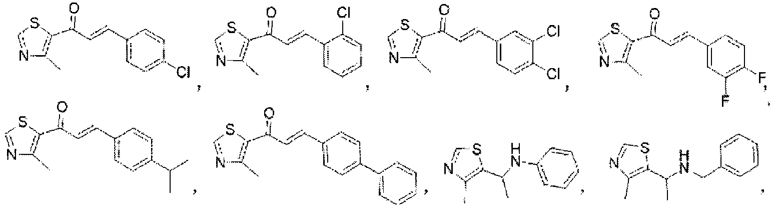 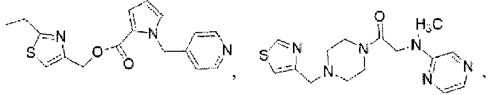 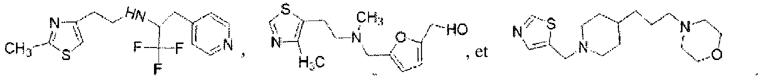 pour une utilisation dans le traitement ou la prévention d'une maladie induite par une inflammatoire choisie parmi le cancer du foie, le gliome, le cancer de l'ovaire, le cancer du poumon, le cancer de la vessie, le cancer de la vésicule biliaire, les lésions hépatiques, la stéatose hépatique, l'hépatite, la fibrose hépatique, la fibrose pulmonaire, la polyarthrite rhumatoïde et rhumatismale, la septicémie, la maladie d'Alzheimer, l'accident vasculaire cérébral ischémique, la maladie de Parkinson, l'hyperlipidémie, l'athérosclérose, la maladie coronarienne et le diabète.

2. Composé pour une utilisation selon la revendication 1, dans lequel le composé est choisi dans le groupe constitué par :

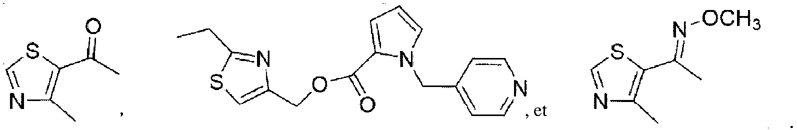

3. Sel du composé pour une utilisation selon la revendication 1, dans lequel le sel est un sel acide du composé d'un acide inorganique ou d'un acide organique ;
dans lequel l'acide inorganique est au moins l'un choisi dans le groupe constitué par l'acide chlorhydrique, l'acide bromhydrique, l'acide iodhydrique, l'acide sulfurique, l'acide nitrique et l'acide phosphorique ; et
l'acide organique est au moins l'un choisi dans le groupe constitué par l'acide acétique, l'acide oxalique, l'acide succinique, l'acide tartrique, l'acide malique, l'acide lactique, l'acide méthanesulfonique, l'acide p-toluènesulfonique, l'acide citrique, l'acide maléique, l'acide fumarique, l'acide salicylique et l'acide acétylsalicylique.

4. Composé pour une utilisation selon la revendication 1, dans lequel le composé a une formule développée de 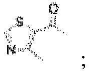 un diagramme de diffraction des rayons X sur poudre d'une forme cristalline A d'un chlorhydrate du composé comprend 3 ou plus valeurs 2*θ* choisies dans le groupe constitué par 8,4 ± 0,2° ; 13,1 ± 0,2° ; 14,8 ± 0,2° ; 16,6 ± 0,2° ; 24,1 ± 0,2° ; 27,2 ± 0,2° ; 30,5 ± 0,2° ; 31,8 ± 0,2° ; 33,5 ± 0,2° ; 35,4 ± 0,2° et 35,7 ± 0,2° ; et
un diagramme d'analyseur de calorimétrie différentielle à balayage-thermogravimétrie de la forme cristalline A du chlorhydrate du composé comprend un pic endothermique significatif de 70 °C à 220 °C et montre une décomposition thermique de 80 °C à 170 °C.

5. Composé pour une utilisation selon la revendication 1, dans lequel le composé a une formule développée de 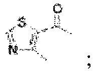 un diagramme de diffraction des rayons X sur poudre d'une forme cristalline B d'un sulfate du composé comprend 5 ou plus valeurs 2*θ* choisies dans le groupe constitué par 10,1 ± 0,2° ; 15,1 ± 0,2° ; 16,0 ± 0,2° ; 16,7 ± 0,2° ; 19,2 ± 0,2° ; 19,9 ± 0,2° ; 23,4 ± 0,2° ; 24,0 ± 0,2° ; 25,8 ± 0,2° ; 26,5 ± 0,2° ; 28,9 ± 0,2° ; 30,3 ± 0,2° et 32,2 ± 0,2° ; et
un diagramme d'analyseur de calorimétrie différentielle à balayage-thermogravimétrie de la forme cristalline B du sulfate du composé comprend au moins un pic endothermique de 30 °C à 85 °C, 90 °C à 160 °C ou 215 °C à 330 °C et montre une décomposition thermique de 150 °C à 350 °C.

6. Procédé de préparation du composé selon la revendication 1, dans lequel le procédé est au moins l'un choisi dans le groupe constitué des procédés suivants :
procédé 1 : soumettre une matière première comprenant un composé A, un solvant aprotique et un réactif de Grignard à une réaction à -20 °C à 25 °C pendant 0,5 h à 3 h pour obtenir un inhibiteur A du CYP2E1,
dans lequel le composé A est au moins l'un choisi dans le groupe constitué de composés ayant une formule développée représentée par la formule II : 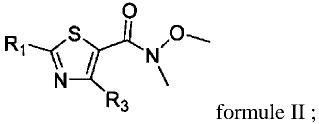 et
l'inhibiteur A du CYP2E1 est au moins l'un choisi dans le groupe constitué de composés ayant une formule développée représentée par la formule III : 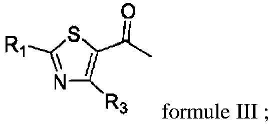
procédé 2 : soumettre un composé ayant une formule développée représentée par la formule III et un chlorhydrate d'hydroxylamine à une réaction en présence d'une source alcaline I pour obtenir un inhibiteur B du CYP2E1,
dans lequel l'inhibiteur B du CYP2E1 est au moins l'un choisi dans le groupe constitué de composés ayant une formule développée représentée par la formule III-1 : 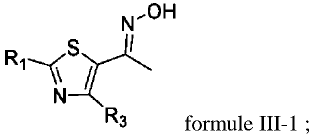
procédé 3 : soumettre un composé ayant une formule développée représentée par la formule III et un composé aldéhyde aromatique à une réaction à 25 °C à 100 °C pendant 2 h à 8 h en présence d'une source alcaline II pour obtenir un inhibiteur C du CYP2E1,
dans lequel l'inhibiteur C du CYP2E1 est au moins l'un choisi dans le groupe constitué de composés ayant une formule développée représentée par la formule III-2 : 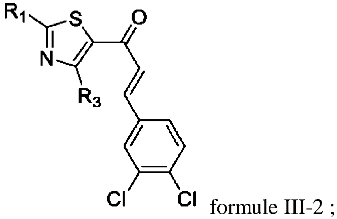 et
procédé 4 : soumettre un composé ayant une formule développée représentée par la formule III, un composé aminé et un agent réducteur à une réaction en présence d'une source d'acide pour obtenir un inhibiteur D du CYP2E1,
dans lequel le composé amine est au moins l'un choisi dans le groupe constitué par la phénylamine et la benzylamine ; et
l'inhibiteur D du CYP2E1 est au moins l'un choisi dans le groupe constitué de composés ayant une formule développée représentée par la formule III-3 : 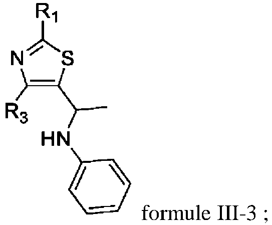
dans lequel dans la formule II, la formule III, la formule III-1, la formule III-2 et la formule III-3, R₁ est l'un quelconque choisi dans le groupe constitué par hydrogène, alkyle en C₁ et alkyle en C₂ ; et R₃ est au moins l'un choisi dans le groupe constitué par hydrogène et alkyle en C₁.

7. Procédé selon la revendication 6, dans lequel, dans le procédé 1, le solvant aprotique est au moins l'un choisi dans le groupe constitué par le tétrahydrofurane et l'éther diéthylique et le réactif de Grignard est au moins l'un choisi dans le groupe constitué par le bromure de méthylmagnésium et le chlorure de méthylmagnésium ;
dans le procédé 2, la source alcaline I est au moins l'une choisie dans le groupe constitué par l'hydroxyde de potassium, l'hydroxyde de sodium, le carbonate de sodium, la pyridine, la triéthylamine et la N,N-diisopropyléthylamine ;
dans le procédé 3, la source alcaline II est au moins l'une choisie dans le groupe constitué par l'hydroxyde de sodium, l'hydroxyde de potassium, le tert-butylate de potassium, le méthylate de sodium et le fluorure de potassium ; et
le composé aldéhyde aromatique est au moins l'un choisi dans le groupe constitué par p-méthoxybenzaldéhyde, o-méthoxybenzaldéhyde, m-méthoxybenzaldéhyde, p-chlorobenzaldéhyde, o-chlorobenzaldéhyde, m-chlorobenzaldéhyde, p-phénylbenzaldéhyde, p-isopropylbenzaldéhyde et 3,4-difluorobenzaldéhyde ; et
dans le procédé 4, la source d'acide est au moins l'une choisie dans le groupe constitué par l'acide formique, l'acide acétique et l'acide chlorhydrique ; et
l'agent réducteur est au moins l'un choisi dans le groupe constitué par le cyanoborohydrure de sodium, le borohydrure de sodium et l'hydrure de lithium et d'aluminium.

8. Procédé selon la revendication 6, dans lequel, dans le procédé 1, un rapport molaire, entre le composé choisi dans le groupe constitué par les composés ayant la formule développée représentée dans la formule II et le réactif de Grignard, est de 1:1 à 1:3 ;
dans le procédé 2, le rapport molaire, entre le composé ayant la formule développée représentée dans la formule III et le chlorhydrate d'hydroxylamine, est de 1:1 à 1:6 ;
dans le procédé 3, un rapport molaire, entre le composé ayant la formule développée représentée dans la formule III et le composé aldéhyde aromatique, est de 1:1 à 1:5 ; et
dans le procédé 4, le rapport molaire, entre le composé ayant la formule développée représentée dans la formule III et l'agent réducteur, est de 1:1 à 1:5.

9. Procédé selon la revendication 6, dans lequel le composé A est préparé par le processus suivant :
soumettre une matière première comprenant un composé A-1, un agent de condensation, du chlorhydrate de N,O-diméthylhydroxylamine et un solvant aprotique à une réaction à 20 °C à 60 °C pendant 10 h à 20 h en présence d'une source alcaline III pour obtenir le composé A,
dans lequel le composé A-1 est au moins l'un choisi dans le groupe constitué de composés ayant une formule développée représentée par la formule II-1 :

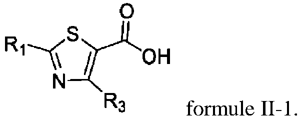

10. Procédé selon la revendication 9, dans lequel l'agent de condensation est au moins l'un choisi dans le groupe constitué par le chlorhydrate de 1-éthyl-(3-diméthylaminopropyl)carbodiimide, le carbonate de didodécyle, le N,N-carbonyldiimidazole, le dicyclohexylcarbodiimide et le N-(4-carboxyphényl)maléimide ; et
la source alcaline III est au moins l'une choisie dans le groupe constitué par l'hydroxyde de sodium, l'hydroxyde de potassium, l'hydroxyde de calcium, le carbonate de sodium, le carbonate de potassium, la pyridine, la triéthylamine et la N,N-diisopropyléthylamine.

11. Procédé selon la revendication 9, dans lequel le composé choisi dans le groupe constitué par les composés ayant une formule développée représentée par la formule II-1, l'agent de condensation et le chlorhydrate de N,O-diméthylhydroxylamine sont dans un rapport molaire de 1:1:1 à 1:5:5.

12. Procédé selon la revendication 9, dans lequel la préparation du composé choisi dans le groupe constitué par les composés ayant une formule développée représentée par la formule II-1 comprend au moins les étapes suivantes :
soumettre une matière première comprenant un composé choisi dans le groupe constitué par les composés ayant une formule développée représentée par la formule II-2 à une hydrolyse en présence d'une source alcaline IV pour obtenir un mélange, et ajuster un pH du mélange avec une source d'acide jusqu'à 2 à 3 pour obtenir le composé choisi dans le groupe constitué par les composés ayant une formule développée représentée par la formule II-1,

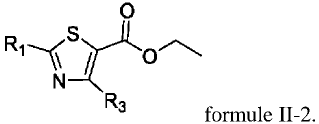

13. Procédé selon la revendication 12, dans lequel la source alcaline IV est au moins l'une choisie dans le groupe constitué par l'hydroxyde de sodium, l'hydroxyde de potassium, l'hydroxyde de calcium, le carbonate de sodium et le carbonate de potassium.

14. Procédé selon la revendication 12, dans lequel la source d'acide est de l'acide chlorhydrique concentré ; et
l'ajustement d'un pH du mélange avec une source d'acide jusqu'à 2 à 3 est effectué à 10 °C à 50 °C.

15. Sel du composé pour une utilisation selon la revendication 1, dans lequel le sel est obtenu par :
soumettre une matière comprenant le composé et un solvant A à une réaction à -20 °C à 80 °C pendant 0,5 h à 10 h pour obtenir le sel du composé ;
dans lequel le solvant A est au moins l'un choisi dans le groupe constitué par un composé éther, un composé alcool, un composé ester, un composé nitrile, un composé cétone, un haloalcane, un alcane et un hydrocarbure aromatique.
